(19) Europäisches Patentamt
European Patent Office
Office européen des brevets

(11) EP 2 958 951 B1

(12) **FASCICULE DE BREVET EUROPEEN**

(45) Date de publication et mention de la délivrance du brevet:
**04.01.2017 Bulletin 2017/01**

(21) Numéro de dépôt: **14711547.1**

(22) Date de dépôt: **21.02.2014**

(51) Int Cl.:
***C08F 220/00*** (2006.01) ***A61Q 17/00*** (2006.01)
***A61K 8/81*** (2006.01) ***A61K 31/80*** (2006.01)
***C08F 8/42*** (2006.01)

(86) Numéro de dépôt international:
**PCT/FR2014/000043**

(87) Numéro de publication internationale:
**WO 2014/128370 (28.08.2014 Gazette 2014/35)**

(54) **NANOPARTICULES POLYMERISEES EN RESEAU ACTIF OU BIOACTIF, TOPIQUES PROTECTEURS, LEURS PROCEDES DE PREPARATION ET LEURS UTILISATIONS**

SCHÜTZENDE TOPISCHE POLYMERISIERTE NANOPARTIKEL IN EINER AKTIVEN ODER BIOAKTIVEN ANORDNUNG, VERFAHREN ZUR HERSTELLUNG DAVON UND VERWENDUNGEN DAVON

TOPICAL PROTECTIVE POLYMERISED NANOPARTICLES IN AN ACTIVE OR BIOACTIVE ARRAY, METHODS FOR PREPARING SAME AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.02.2013 FR 1300396**

(43) Date de publication de la demande:
**30.12.2015 Bulletin 2015/53**

(73) Titulaires:
- **Centre National de la Recherche Scientifique (C.N.R.S.)
75016 Paris (FR)**
- **Université Nice - Sophia Antipolis
06103 Nice (FR)**

(72) Inventeurs:
- **GUITTARD, Frédéric
F-06000 Nice (FR)**
- **AMIGONI, Sonia
F-06200 Nice (FR)**
- **TAFFIN DE GIVENCHY, Elisabeth
F-06000 Nice (FR)**
- **ZENERINO, Arnaud
F-06440 Blausasc (FR)**
- **JOSSE, Denis
38410 Vaulnaveys Le Haut (FR)**

(74) Mandataire: **Macquet, Christophe
Macquet & Associés
Arche des Dolines
7, rue Soutrane
06560 Sophia Antipolis (FR)**

(56) Documents cités:
**WO-A1-2006/117476**

- **AL-SAGHEER F A ET AL: "Visco-elastic properties of chitosantitania nano-composites", CARBOHYDRATE POLYMERS, APPLIED SCIENCE PUBLISHERS, LTD. BARKING, GB, vol. 85, no. 2, 21 février 2011 (2011-02-21), pages 356-362, XP028193115, ISSN: 0144-8617, DOI: 10.1016/J.CARBPOL.2011.02.032 [extrait le 2011-02-25]**
- **Christille LE CHATELIER-BRUNET: "Synthèse et caractérisation de polymères amphiphiles très hydrophobes anioniques et application au contrôle de la rhéologie de fluides complexes", Université Paris VI , 30 mai 2005 (2005-05-30), XP002712704, Extrait de l'Internet: URL:http://pastel.archives-ouvertes.fr/docs/00/50/01/80/PDF/these.pdf [extrait le 2013-09-12]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- JAE KWAN HWANG, HAE HUN SHIN: "Rheological properties of chitosan solutions", KOREA-AUSTRALIA RHEOLOGY JOURNAL, vol. 2, no. 3/4, décembre 2000 (2000-12), pages 175-179, XP002712705,

## Description

La présente invention concerne des composés hybrides organiques/inorganiques formés par des micro- ou nanoparticules fonctionnalisées d'oxyde d'aluminium ($Al_2O_3$), d'oxyde de cuivre (CuO), d'oxyde de fer ($Fe_3O_4$ ou $\gamma$-$Fe_2O_3$), d'oxyde de magnésium (MgO), de silice ($SiO_2$), de dioxyde de titane ($TiO_2$) ou d'oxyde de zinc (ZnO), greffées de manière covalente sur des polymères modificateurs de rhéologie. Plus particulièrement, l'invention concerne des micro- ou nanoparticules de silice ou de dioxyde de titane fonctionnalisées amines, greffées à des polymères pour former un réseau actif ou bioactif. L'invention concerne encore des topiques protecteurs les comprenant, leurs procédés de synthèses ainsi que leurs utilisations notamment pour la protection ou la décontamination cutanée.

Les contaminations par des agents du risque biologique ou chimique peuvent être multiples. Les agents du risque biologique sont généralement des bactéries, des virus ou des toxines. Les agents du risque chimique sont généralement des composés neurotoxiques organophosphorés, ou des vésicants.

Depuis que le terrorisme chimique et le bioterrorisme existent, de nombreuses réflexions pour protéger les êtres humains ont été engagées. Lors d'interventions, par exemple après un acte de terrorisme ou lors de l'utilisation de toxiques par des soldats, le port d'une tenue de protection est obligatoire. Ce type de tenue doit également être employé en milieu agricole et dans certaines industries. En effet, l'emploi par exemple de pesticides dangereux peut contaminer l'organisme et provoquer des lésions importantes voire la mort de l'utilisateur. Le problème majeur des tenues de protection est qu'elles entrainent une modification des capacités opérationnelles des intervenants et une réduction de leurs capacités sensorielles (vision, audition, dextérité manuelle...). En outres, elles présentent rapidement des limites d'utilisation puisqu'elles peuvent être mal ajustées ou être déchirées et ainsi exposer la peau aux toxiques.

Pour toutes les raisons qui précèdent, il est nécessaire de trouver de nouveaux moyens de protection rendant toutes ou une grande partie de leurs facultés à leurs utilisateurs. Avant de définir quels sont ces moyens de protection, il est important d'identifier les agents du risque, ainsi que leurs propriétés et leur mode de pénétration dans la peau.

Les produits chimiques toxiques peuvent être classés selon plusieurs critères comme leur volatilité, leur utilisation militaire ou leurs effets hémotoxiques, vésicants, suffocants, neurotoxiques, incapacitants, neutralisants.

Il existe aujourd'hui principalement un intérêt dans la protection contre les agents vésicants et les composés organophosphorés qui représentent la principale menace chimique. Les agents vésicants sont principalement représentés par les ypérites au soufre ou à l'azote et plus modestement par la lewisite et l'oxime de phosgène. Les composés organophosphorés (COP) regroupent les pesticides (POP) et les neurotoxiques organophosphorés (NOP). Les POP sont le plus souvent des phosphates ou des phosphorothioates, contenant des substituants O,O-diméthyle ou O,O-diéthyle sur l'atome de phosphore. Ce sont généralement des inhibiteurs indirects des cholinestérases c'est-à-dire qu'ils ne deviennent actifs qu'après transformation métabolique : S-oxydation de la liaison P=S. Ceci se traduit par une plus grande latence entre l'exposition et l'apparition des signes d'intoxication. Les pesticides organophosphorés ont remplacé les composés organochlorés qui étaient hautement rémanents malgré la toxicité plus importante des POP. Les premiers composés comme le parathion (phosphorothioate de O,O-diéthyle et de O-p-nitrophényle), le malathion (di-thiophosphate de S-(1,2-dicarbéthoxyéthyle) et de O,O-diméthyle) et le paraoxon (phosphate de diéthyle p-nitrophényle) sont de puissants inhibiteurs des cholinestérases. Les pesticides organophosphorés sont extrêmement toxiques et provoquent de graves intoxications surtout dans le milieu agricole. L'Organisation Mondiale de la Santé (OMS) a estimé qu'il y a chaque année dans le monde 1 million de graves empoisonnements par les pesticides, avec quelque 220 000 décès. Le risque d'intoxication par les pesticides est important du fait du contact fréquent lors des pulvérisations de pesticides au sol ou aérienne et de manipulations.

Les premiers neurotoxiques organophosphorés (NOP) synthétisés ont été les agents G. Ils incluent notamment l'agent GA ou Tabun, l'agent GB ou Sarin et l'agent GD ou Soman. Ce sont des esters de dérivés des acides fluorophosphoniques ou phosphoramidiques. D'autres neurotoxiques organophosphorés (NOP) sont les agents V.

L'exposition à des concentrations excessives de tels agents peut provoquer une intense sécrétion des bronches, salivaire, oculaire et intestinale, suivie de sudation, bradycardie, contractions musculaires, tremblements, paralysie, perte de conscience, convulsions, dysfonctionnement du système respiratoire et de la mort.

Ainsi, la meilleure façon pour prévenir la toxicité percutanée d'agents chimiques est de ne jamais les laisser arriver au contact de la peau.

Comme indiqué précédemment, la prévention de la contamination cutanée est principalement réalisée par le port de protections telles que des combinaisons, masques ou des gants. Toutefois, il peut rester des zones d'exposition à ces agents, lors de mouvements de l'être humain ou par défaut de protection, par exemple si l'équipement de protection est mal ajusté ou inadapté. Par ailleurs, un transfert de contamination sur la peau est également possible lors de la phase de déshabillage.

Il existe donc un besoin de développer d'autres moyens de protection qui soient à la fois bien tolérés par leur utilisateur, qui soient faciles d'utilisation, résistants à l'environnement extérieur et qui ne gênent pas le porteur.

Pour cela, l'utilisation de topiques protecteurs permet de fournir une protection alternative ou complémentaire contre les irritants et les agressifs environnementaux, comme par exemple les microorganismes, les produits chimiques,

les agents vésicants et les composés organophosphorés.

La majorité des produits vendus sous le terme « topiques protecteurs » (TP) regroupe à la fois des produits cosmétiques simplement émollients et des formulations pouvant potentiellement exercer une fonction de barrière face à des produits chimiques agressifs. Ces topiques sont destinés à être utilisés dans des environnements à risque. Les topiques protecteurs sont utilisés dans de nombreux domaines tels que l'industrie ou l'utilisation personnelle. Ils peuvent ainsi :

- prévenir de l'allergie par contact de la peau de nombreux métaux dont le nickel, le cobalt, le chrome, le palladium et l'or ;
- être employés comme filtres solaires pour la filtration des UVA et UVB à base de nanoparticules de dioxyde de titane ou d'émulsion de silicones ;
- être employés comme insectifuges ;
- être employés pour la protection chimique contre les acides, les bases, les tensioactifs et les solvants organiques.

Les topiques protecteurs à base de composés perfluorés sont de bons moyens de protection de l'organisme et permettent une certaine facilité d'emploi. De tels topiques sont notamment décrits dans le document Zhang J.; Smith E.W.; Surber C.; Galenical principles in skin protection, Curr. Probl. Dermatol., 2007, 34, 11-18 *;* ainsi que dans les documents US 5,607,979 et GB 2,314,020.

Cependant, de tels topiques ne dégradent pas les agents contaminants et leur effet protecteur est limité notamment en cas d'exposition aux vapeurs d'agents chimiques.

Une seconde génération de topiques protecteurs consistant en l'incorporation d'actifs, organiques ou inorganiques, pouvant neutraliser les contaminants chimiques a également été développée. De tels topiques sont notamment décrits dans le document Koper O.; Lucas E.; Klabunde K.J.; Development of reactive topical skin protectants against sulfur mustard and nerve agents, J. Appl. Toxicol., 1999, 19, 59-70*;* ou encore dans le document Saxena A.; Srivastava A.K.; Singh B.; Goyal A.; Removal of sulphur mustard, sarin and simulants on impregnated silica nanoparticles, J. Hazard. Mater., 2012, 211-212, 226-232*).*

Leur efficacité protectrice est partiellement établie et non optimisée en raison notamment de l'agglomération des actifs micro- et/ou nano-particulaires.

Il existe donc à ce jour un besoin de développer de nouveaux topiques protecteurs qui soient plus efficaces, peu ou pas toxiques, faciles à mettre en place et qui aient un spectre large d'action contre les agents du risque biologique et/ou chimique. De préférence, de tels topiques protecteurs permettent de détruire les agents du risque biologique et/ou chimique. Ainsi, ces agents ne pénètrent pas la peau de l'individu.

En outre, de tels topiques devraient idéalement pouvoir être mis en oeuvre par des méthodes simples, rapides et à faible coût et devraient avoir une formulation homogène.

Dans ce contexte, le Demandeur a développé un nouveau concept consistant à greffer des micro- ou nanoparticules, connues pour leurs effets neutralisant vis-à-vis d'agents chimiques toxiques et/ou biologiques, à des polymères notamment modificateurs de rhéologie, facilitant l'intégration et la dispersion homogène des micro- ou nanoparticules dans une formulation topique mais également en évitant le relargage des micro- ou nanoparticules.

La solution au problème posé a ainsi pour objet un composé formé par des micro- ou nanoparticules fonctionnalisées, associées de façon covalente à des polymères modificateurs de rhéologie, caractérisé en ce que :

- les micro- ou nanoparticules fonctionnalisées sont des micro- ou nanoparticules fonctionnalisées d'oxyde inorganique ou métallique notamment d'oxyde d'aluminium ($Al_2O_3$), d'oxyde de cuivre (CuO), d'oxyde de fer ($Fe_3O_4$ ou $\gamma$-$Fe_2O_3$), d'oxyde de magnésium (MgO), de silice ($SiO_2$), de dioxyde de titane ($TiO_2$) ou d'oxyde de zinc (ZnO), présentant un diamètre nominal compris entre 1 et 1500 nm ;

les polymères modificateurs ou adaptateurs de rhéologie sont choisis parmi des polymères non-associatifs ou des polymères associatifs.

De façon surprenante, le Demandeur a notamment pu mettre en évidence, comme cela est illustré à l'exemple 11, que les composés selon l'invention permettent d'obtenir d'excellents topiques protecteurs qui limitent la pénétration transmembranaire d'agents toxiques, tels que le paraoxon. Le Demandeur a également pu mettre en évidence par des tests d'écotoxicité repris à l'exemple 12, que les composés selon l'invention sont faiblement toxiques pour l'environnement.

La présente invention a également pour deuxième objet un topique protecteur comprenant un composé selon l'invention, dans un milieu pharmaceutiquement et/ou cosmétiquement acceptable.

Elle a également pour troisième objet un composé ou un topique protecteur selon l'invention à titre de médicament.

Elle a pour quatrième objet un composé ou un topique protecteur selon l'invention, pour son utilisation dans

la prévention des irritations cutanées ou des allergies.

Elle a pour cinquième objet l'utilisation d'un composé ou d'un topique protecteur selon l'invention, pour la protection ou la décontamination cutanée, notamment due aux agents du risque biologique et/ou chimique.

Elle a pour sixième objet un procédé de synthèse d'un composé selon l'invention comprenant les étapes de :

- mélange d'un agent de couplage avec un catalyseur ;
- ajout du mélange obtenu à une solution de polymères modificateurs ou adaptateurs de rhéologie choisis parmi les polymères non-associatifs ou les polymères associatifs, dans de l'eau ;
- agitation du mélange réactionnel ;
- ajout des micro- ou nanoparticules fonctionnalisées d'oxyde d'aluminium ($Al_2O_3$), d'oxyde de magnésium (MgO), de silice ($SiO_2$), de dioxyde de titane ($TiO_2$), d'oxyde de zinc (ZnO) ou d'oxyde de cuivre (CuO) présentant un diamètre nominal compris entre 5 et 1 500 nm, préalablement dispersées en phase aqueuse au mélange réactionnel ;
- purification du milieu réactionnel par dialyse ; et
- récupération du composé formé par une ou plusieurs micro- ou nanoparticules fonctionnalisées amines associées de façon covalente à un ou plusieurs polymères modificateurs de rhéologie.

Elle a pour septième objet des procédés de synthèse des composés selon l'invention.

Enfin, elle a pour dernier objet un polymère modificateur ou adaptateur de rhéologie choisi parmi les polymères non-associatifs ou les polymères associatifs utilisables dans les composés selon l'invention.

Les différents composés précédemment décrits peuvent notamment être synthétisés selon les différents procédés dont les principales étapes sont décrites aux exemples 1 à 7.

L'invention sera mieux comprise à la lecture de la description non limitative qui suit, rédigée au regard des dessins annexés, dans lesquels :

- la figure 1 est une représentation schématique de composés de type téléchélique et de type peigne ;
- la figure 2a représente une synthèse de micro- ou nanoparticules particules de silice par le procédé de Stöber ;
- la figure 2b illustre une fonctionnalisation de micro- ou nanoparticules particules de silice par de l'AMS (3-aminopropyl)triméthoxysilane) ;
- la figure 3 représente une fonctionnalisation de nanoparticules commerciales par de l'AMS ;
- la figure 4 illustre un schéma de synthèse de polymères non-associatifs ASE-H et ASE-F ;
- la figure 5 illustre un schéma de synthèse de polymères associatifs HASE ;
- la figure 6 représente un schéma de synthèse de macromères destinés à être intégrés aux polymères associatifs HASE ;
- la figure 7 représente des études des angles de contact à l'huile d'olive sur différents polymères préalablement déposés sur une plaque de verre ;
- la figure 8 représente des études des angles de contact à l'huile d'olive pour différents polymères HASE-F-RF8 à 3,3 ; 13,5 et 45,9% molaire de macromères ;
- la figure 9 représente une analyse d'écoulement pour les polymères HASE-F-RF8 (3,3% molaire de macromère), (13,5% molaire de macromère) et (45,9% molaire de macromère) ;
- la figure 10 représente des clichés des topographies des films en milieu neutre des composés polymère/silice selon l'invention (à 1 équivalent) ;
- la figure 11 représente une étude d'évaluation de topiques (HASE-F-RF8 (13,5% molaire de macromère)/Si et HASE-F-RF8 (13,5% molaire de macromère)) vis-à-vis de la pénétration transmembranaire du paraoxon ;
- la figure 12 représente des mesures de la $EC_{50}$ pour *Daphnia magna* de suspensions d'eau des nanoparticules de silice pures (Si02-14) et fonctionnalisées (Si02-22; -300 et -400) ;
- la figure 13 représente des mesures de la $EC_{5o}$ de *Phaeodactylum tricornutum* et *Chlorella vulgaris* (*SAPS*) de suspensions d'eau des nanoparticules de silice pures (Si02-14) et fonctionnalisées (Si02-22; -300 et -400) ;
- la figure 14 représente des mesures de la $EC_{50}$ de *Daphnia magna* et *Phaeodactylum tricornutum* de suspensions d'eau du polymère HASE-H-RH8 (1,3% molaire de macromères) (polymère HASE ou « HASE Polymer ») et du composé HASE-H-RH8 (1,3% molaire de macromère)/Si (0,3 eq) (polymère greffé ou « Grafted Polymer »).
- la figure 15 représente une structure générale possible de composés selon l'invention permettant d'obtenir une protection nouvelle technologie ;
- la figure 16 représente des clichés des topographies des films en milieu neutre des composés HASE-F-RF8 (3,3%)/Si-22 à 1 ; 0,3 ; 0,1 ; 0,05 et 0,01 équivalent ; et
- la figure 17 illustre un histogramme contenant les valeurs d'angles de contact pour l'eau, l'eau après 1 minute (eau à t1) et l'huile d'olive en fonction du nombre d'équivalent de silice contenu dans les différents dépôts polymère/silice ;
- la figure 18 illustre un schéma de synthèse d'un réseau polymère organique/inorganique selon l'invention ;
- la figure 19 représente le spectre FT-IR (spectroscopie infrarouge à transformée de Fourier) de HASE-F-RF8/Si/RF8

en milieu neutre ;

- la figure 20 représente l'agrégation des nanoparticules de silice caractérisée par le potentiel zêta, en fonction du pH pour HASE-F-RF8 (3,3%) (carrés), HASE-F-RF8/Si (3,3%) (ronds) et HASE-F-RF8(13,5%)/Si/RF8 (triangles) ;
- la figure 21 est un graphique illustrant la mouillabilité à l'huile d'olive de HASE-F-RF8/Si (ronds) et de HASE-F-RF8/Si/RF8 (carrés) en fonction de la quantité (mg) étalée sur une plaque de verre ;
- les figures 22a et 22b représentent des images de l'angle de contact des composés HASE-F-RF8/Si (fig. 22a) et HASE-F-RF8/Si/RF8 (fig. 22b) par la méthode d'étalement avec, respectivement, 52 mg et 47 mg sur des plaques de verre ;
- les figures 23a à 23f représentent des images MEB (Microscopie Electronique à Balayage) de HASE-F-RF8/Si par étalement (figures 23a et 23b, avec 52mg déposés) et de HASE-F-RF8/Si/RF8 par étalement (figures 23c et 23d avec 47 mg déposés) et spray (figures 23e et 23f avec 15 mg déposés) ;
- les figures 24a et 24b représentent des images de l'angle de contact pour HASE-F-RF8/Si/RF8 par les méthodes d'étalement (fig. 24a) et spray (fig. 24b) avec 15 mg déposés sur une plaque de verre ;
- les figures 25a et 25b illustrent la dispersion de HASE-F-RF8/Si/RF8 par les méthodes d'étalement (fig. 25a) et par spray (fig. 25b) ;
- les figures 26a et 26b représentent des images AFM (Microscopie à Force Atomique) des dépôts par étalement des composés HASE-F-RF8/Si/RF4 pour 49 mg (fig. 26a) et HASE-F-RF8/Si/RF6 pour 60 mg (fig. 26b).

Le composé selon l'invention est un composé formé par des micro- ou nanoparticules fonctionnalisées associées de façon covalente à des polymères modificateurs de rhéologie, caractérisé en ce que :

- les micro- ou nanoparticules fonctionnalisées sont des micro- ou nanoparticules fonctionnalisées amines d'oxyde d'aluminium ($Al_2O_3$), d'oxyde de cuivre (CuO), d'oxyde de fer ($Fe_3O_4$ ou $\gamma$-$Fe_2O_3$), d'oxyde de magnésium (MgO), de silice ($SiO_2$), de dioxyde de titane ($TiO_2$) ou d'oxyde de zinc (ZnO), présentant un diamètre nominal compris entre 1 et 1500 nm ;
- les polymères modificateurs ou adaptateurs de rhéologie sont choisis parmi des polymères non-associatifs ou des polymères associatifs.

Selon l'invention, une nanoparticule est définie comme étant un nano-objet dont les trois dimensions sont à l'échelle nanométrique, c'est-à-dire une particule dont le diamètre nominal est inférieur à 100 nm.

De préférence, la nanoparticule selon l'invention présente un diamètre compris entre 1 et 50 nm. De préférence encore, le diamètre nominal de la nanoparticule est compris entre 5 nm et 25 nm.

Une microparticule selon l'invention est quant à elle un micro-objet dont les trois dimensions sont à l'échelle micrométrique, c'est-à-dire une particule dont le diamètre nominal est compris entre 100 nm et 100000 nm. De préférence, la microparticule selon l'invention présente un diamètre nominal compris entre 100 nm et 5000 nm. De préférence encore, le diamètre nominal de la microparticule est compris entre 100 nm et 1500 nm.

Les micro- ou nanoparticules peuvent être produites par des méthodes diverses, notamment par synthèse chimique en phase vapeur, en phase liquide, en milieu solide, en milieu mixte ou encore par des méthodes physico-chimiques telles que l'évaporation/condensation.

Selon un mode préféré de réalisation de l'invention, les micro- ou nanoparticules d'oxyde d'aluminium ($Al_2O_3$), d'oxyde de magnésium (MgO), de silice ($SiO_2$), de dioxyde de titane ($TiO_2$) et d'oxyde de cuivre (CuO) peuvent être synthétisées en milieu mixte par la méthode sol-gel. Le principe du procédé sol-gel repose sur l'utilisation d'une succession de réactions d'hydrolyse-condensation, à température modérée proche de l'ambiante, pour préparer des réseaux d'oxydes qui peuvent être à leur tour traités thermiquement. L'espèce métallique soluble peut aussi contenir des constituants organiques qui peuvent être ajustés selon les applications. La première étape dans la synthèse sol-gel est l'hydroxylation de l'alcoxy métallique, qui se produit lors de l'hydrolyse du groupe alcoxy :

Etape 1 : hydrolyse :

$$M'\text{-}OR' + H_2O \rightarrow M'\text{-}OH + R'OH$$

où M' représente l'Aluminium, le Cuivre, le Magnésium, le Silicium ou le Titane ; et R' représente un groupement organique alkyle comprenant de 1 à 5 atomes de carbone, préférentiellement de 2 à 3 atomes de carbone.
Les groupes réactifs hydroxy sont alors générés. La solution obtenue est appelée sol. Ils sont ensuite modifiés par des réactions de polycondensation *via* deux mécanismes compétitifs : la formation d'un pont oxygène (oxolation) ou d'un pont hydroxo (olation). Cela correspond à la formation du réseau macromoléculaire minéral avec élimination d'eau ou d'alcool :
Etape 2 : condensation :

- Oxolation : M'-OH + M'-OR' → M'-O-M' + R'OH
  où M' et R' sont tels que définis ci-dessus, c'est-à-dire que M' représente l'Aluminium, le Cuivre, le Magnésium, le Silicium ou le Titane ; et R' représente un groupement organique alkyle comprenant de 1 à 5 atomes de carbone, préférentiellement de 2 à 3 atomes de carbone.
- Olation : M'-OH + HO-M' → M' (OH) 2M' où M' représente l'Aluminium, le Cuivre, le Magnésium, le Silicium ou le Titane.

Le gel correspond à la formation d'un réseau tridimensionnel de liaisons de Van der Waals.

Les micro- ou nanoparticules utilisables selon l'invention ont préférentiellement un diamètre nominal moyen compris entre 1 et 1500 nm.

De façon avantageuse, les micro- ou nanoparticules d'oxyde d'aluminium ($Al_2O_3$) ont un diamètre nominal moyen compris entre 5 et 400 nm. De préférence, leur diamètre nominal moyen est compris entre 10 et 200 nm. De préférence encore, leur diamètre nominal moyen est de 13, 20 ou 50 nm.

De façon avantageuse, les micro- ou nanoparticules d'oxyde de cuivre (CuO) ont un diamètre nominal moyen compris entre 20 et 100 nm. De préférence, leur diamètre nominal moyen est compris entre 30 et 80 nm. De préférence encore, leur diamètre nominal moyen est compris entre 40 et 70 nm.

De façon avantageuse, les micro- ou nanoparticules d'oxyde de fer ($Fe_3O_4$ ou $\gamma$-$Fe_2O_3$) ont un diamètre nominal moyen compris entre 1 et 50 nm. De préférence, leur diamètre nominal moyen est compris entre 5 et 30 nm. De préférence encore, leur diamètre nominal moyen est compris entre 10 et 30 nm.

De façon avantageuse, les micro- ou nanoparticules d'oxyde de magnésium (MgO) ont un diamètre nominal moyen compris entre 20 et 100 nm. De préférence, leur diamètre nominal moyen est compris entre 40 et 70 nm. De préférence encore, leur diamètre nominal moyen est compris entre 50 et 60 nm.

De façon avantageuse, les micro- ou nanoparticules de silice ($SiO_2$) ont un diamètre nominal moyen compris entre 5 et 500 nm. De préférence, leur diamètre nominal moyen est compris entre 10 et 450 nm. De préférence encore, les micro- ou nanoparticules de silice ont un diamètre nominal moyen de 14, 22, 292 ou 448 nm.

De façon avantageuse, les micro- ou nanoparticules de dioxyde de titane ($TiO_2$) ont un diamètre nominal moyen compris entre 1 et 300 nm. De préférence, leur diamètre nominal moyen est compris entre 5 et 150 nm. De préférence encore, leur diamètre nominal moyen est compris entre 10 et 25 nm.

De façon avantageuse, les micro- ou nanoparticules d'oxyde de zinc (ZnO) ont un diamètre nominal moyen compris entre 5 et 500 nm. De préférence, leur diamètre nominal moyen est compris entre 20 et 200 nm. De préférence encore, leur diamètre nominal moyen est compris entre 50 et 60 nm. Selon l'invention, les micro- ou nanoparticules sont fonctionnalisées.

Les micro- ou nanoparticules peuvent être fonctionnalisées par exemple par des fonctions amines primaire ou secondaire, des fonctions époxy, des fonctions alcools et des fonctions thiol.

De préférence, les micro- ou nanoparticules d'oxyde d'aluminium ($Al_2O_3$), d'oxyde de cuivre (CuO), d'oxyde de fer ($Fe_3O_4$ ou $\gamma$-$Fe_2O_3$), d'oxyde de magnésium (MgO), de silice ($SiO_2$), de dioxyde de titane ($TiO_2$) ou d'oxyde de zinc (ZnO) sont fonctionnalisées amine.

De préférence, les micro- ou nanoparticules sont des micro- ou nanoparticules fonctionnalisées amines d'oxyde inorganique ou métallique notamment d'oxyde d'aluminium ($Al_2O_3$), d'oxyde de cuivre (CuO), d'oxyde de fer ($Fe_3O_4$ ou $\gamma$-$Fe_2O_3$), d'oxyde de magnésium (MgO), de silice ($SiO_2$), de dioxyde de titane ($TiO_2$) ou d'oxyde de zinc (ZnO).

Le schéma général de la fonctionnalisation amine des micro- ou nanoparticules est illustré aux figures 2b et 3.

Le détail d'une voie de synthèse des microparticules de silice de 292 nm et 448 nm fonctionnalisées amine est décrit à l'exemple 1. L'exemple 2 décrit quant à lui une voie de synthèse des nanoparticules de silice de 14 nm fonctionnalisées amine. L'exemple 3 décrit quant à lui la synthèse de micro-ou nanoparticules de dioxyde de titane fonctionnalisées amine.

De préférence, le taux de fonctions amines sur les micro- ou nanoparticules fonctionnalisées amine est compris entre 0,1 et 10 meq/g de micro- ou nanoparticules.

Le nombre de fonctions NH2 obtenu est en meq/g de micro- ou nanoparticules et se calcule selon l'équation suivante :

$$\text{Nombre de fonctions } NH_2\left(\frac{meq}{g}\right) = \frac{\%N \text{ massique (déterminé par analyse élémentaire)}}{\text{Masse molaire de l'azote}} \times 100$$

De préférence, le taux de fonctions amines sur les micro- ou nanoparticules fonctionnalisées amine est compris entre 0,5 et 5 meq/g de micro- ou nanoparticules. De préférence encore, le taux de fonctions amines sur les micro- ou nanoparticules est d'environ 1 ou 2,5 meq/g de micro- ou nanoparticules.

Comme cela a été décrit précédemment, les micro- ou nanoparticules selon l'invention sont fonctionnalisées

amine, ceci afin de réagir sur les fonctions acides des polymères.

Les micro- ou nanoparticules fonctionnalisées amine selon l'invention sont préférentiellement des micro- ou nanoparticules fonctionnalisées amine de silice ($SiO_2$) et de dioxyde de titane ($TiO_2$).

Les polymères modificateurs ou adaptateurs de rhéologie selon l'invention sont déjà utilisés, seuls, pour leurs propriétés rhéologiques dans des domaines comme les cosmétiques ou la peinture.

Il s'agit de polymères hydrocarbonés ou fluorocarbonés synthétisés par exemple par polymérisation en émulsion.

Ils sont ensuite caractérisés par infrarouge (IR) par la méthode de la pastille KBr, par goniométrie lors de dépôts secs sur surface de verre, mais aussi en solution par résonance magnétique nucléaire (RMN) et rhéologie. Les polymères selon l'invention contribuent également à la stabilité des formulations ou topiques protecteurs.

Les polymères utilisables appartiennent à deux classes différentes : les polymères non-associatifs et les polymères associatifs.

Les polymères non-associatifs ou émulsions pouvant gonfler en milieu alcalin pour « Alkali-Swellable Emulsions » (ASE) utilisables selon l'invention sont déjà largement utilisés, seuls, comme épaississants dans les revêtements en latex, les peintures et les adhésifs.

Ils sont constitués principalement de monomères d'acide acrylique ou méthacrylique et d'acrylate d'alkyle en C1-C4, préférentiellement l'acrylate d'éthyle.

Ils sont généralement synthétisés par polymérisation en émulsion en milieu aqueux acide (pH moins de 4) et sont obtenus sous forme de suspension colloïdale de polymères (ou latex synthétiques). Les fonctions acides du copolymère sont ionisées en milieu basique ce qui provoque la solubilisation et le gonflement du polymère (augmentation du volume hydrodynamique). Les groupements d'acrylate d'éthyle sont assez bloqués pour induire des associations hydrophobes entre chaînes de polymère et augmenter la viscosité.

En outre, les polymères non-associatifs ASE peuvent être optimisés par réticulation. Le phénomène de réticulation permet de densifier physiquement le réseau polymère ce qui diminue la possibilité de mouvement des molécules et donc augmente la viscosité.

Les polymères non-associatifs selon l'invention peuvent comprendre une chaine hydrocarbonée (ASE-H) et/ou une chaine fluorocarbonée (ASE-F).

De préférence, les polymères non-associatifs ASE-H répondent à la formule générale (I) suivante :

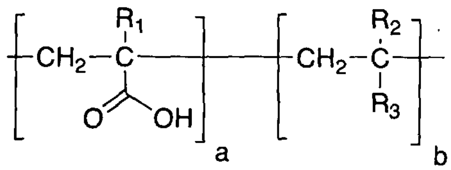

(I)

dans laquelle :

- R1 et R2 représentent un atome d'hydrogène ou un groupement méthyle $-CH_3$ ;
- R3 représente $[Q]_{d1}-(CH_2)_n-H$ dans laquelle n est compris entre 1 et 30, d1 correspond à 0 ou à 1, et Q correspond à -C(O)-O ou -C(O)-NH- ;
  ou
  R3 représente $[Q]_{d2}-\alpha$, dans laquelle :
    - d2 correspond à 0 ou 1 ;
    - Q correspond à -C(O)-O ou -C(O)-NH- ; et
    - $\alpha$ correspond à $-C(CH_3)3$ ; $-CH(CH_3)2$ ; $-C(CH_3)_2-CH_2-C(CH_3)_3$; % -CN ; $-CH_2CH_2-N+(CH_3)_2(CH_2CO_2^-)$ ; $-CH_2CH_2-NH-C(CH_3)3$ ; $-CH_2CH_2-N(CH_3)_2$ ; pyrrolidinone ; caprolactame ;

  et dans laquelle les indices a et b sont des nombres entiers, identiques ou différents, supérieurs à 1. Préférentiellement, a est compris entre 1 et 10 000 et b est compris entre 1 et 20 000.

Dans l'ensemble de la description, les polymères sont composés de différents monomères ou macromères, à des concentrations molaires données, qui varient en fonction des valeurs de a, b et/ou c. Bien entendu, l'enchainement des différents monomères ou macromères dans les polymères obtenus est variable et n'est pas figé dans les formules (I), (II), (III), (V), (VI), (VII) et (VIII).

De préférence encore, les polymères ASE à chaine hydrocarbonée (ASE-H) comprennent des monomères d'acide acrylique et/ou méthacrylique et d'acrylates d'alkyle en C1-C4.

De façon avantageuse, les polymères ASE à chaine hydrocarbonée (ASE-H) comprennent :

- de 5 à 50% molaire d'acide acrylique et/ou méthacrylique ;
- de 50 à 95% molaire d'acrylates d'alkyle en C1-C4.

Plus préférentiellement, les polymères ASE-H comprennent les monomères suivants :

- de 10 à 20% molaire d'acide méthacrylique (AM) ;
- de 80 à 90% molaire d'acrylate d'éthyle (AE).

Les polymères ASE à chaine hydrocarbonée (ASE-H) présentent des propriétés épaississantes particulièrement avantageuses lorsque le ratio acide méthacrylique/ acrylate d'éthyle est compris entre 0,1 et 0,5. Le ratio plus préféré encore est de 0,21.

Comme indiqué précédemment, les polymères non-associatifs ASE selon l'invention peuvent également comprendre une chaine fluorocarbonée (ASE-F).

De préférence, les polymères non-associatifs ASE-F répondent à la formule générale (II) suivante :

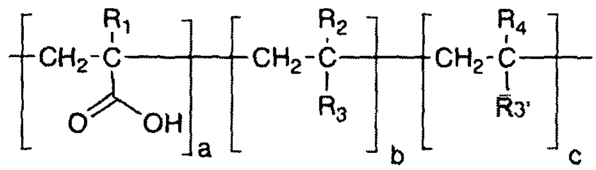

(II)

dans laquelle :

- R1, R2 et R4 représentent un atome d'hydrogène ou un groupement méthyle $-CH_3$ ;
- R3 représente $[Q]_{d1}-(CH_2)_n-H$ dans laquelle n est compris entre 1 et 30, d1 correspond à 0 ou à 1, et Q correspond à -C(O)-O ou -C(O)-NH- ;
  ou
  R3 représente $[Q]_{d2}-\alpha$, dans laquelle :
  - d2 correspond à 0 ou 1 ;
  - Q correspond à -C(O)-O ou -C(O)-NH- ; et
  - $\alpha$ correspond à $-C(CH_3)_3$ ; $-CH(CH_3)2$ ; $-C(CH3)2-CH2-C(CH_3)_3$ ; -CN ; $-CH_2CH_2-N+(CH_3)_2(CH_2CO_2^-)$ ; $-CH_2CH_2-NH-C(CH_3)_3$ ; $-CH_2CH_2-N(CH_3)_2$ ; pyrrolidinone ; caprolactame ;
- R3' représente $[Q]_{d1}-(CH_2)_n-(CX_2)_pX$ dans laquelle n est compris entre 1 et 30, d1 correspond à 0 ou à 1, Q correspond à -C(O)-O ou -C(O)-NH- , X est un atome de fluor F et p est compris entre 1 et 12 ;
  et dans laquelle les indices a et c sont des nombres entiers, identiques ou différents, supérieurs à 1 et b est supérieur ou égal à 0 ; préférentiellement, a est compris entre 1 et 10 000, b est compris entre 0 et 5 000 et c est compris entre 1 et 8 000.

Ces polymères ASE-F sont préférentiellement constitués de monomères :

- d'acide méthacrylique (AM),
- de méthacrylate de 2,2,2-trifluoroéthyle (MTFE) ou d'acrylate de trifluoroéthyle ou d''acrylate de 2-perfluorobutyléthyle ou d'acrylate de 2-perfluorohexyléthyle ou d'acrylate de 2-perfluorooctyléthyle ; et
- d'acrylate d'alkyle en C1-C4, préférentiellement l'acrylate d'éthyle (AE) .

L'introduction d'une chaîne fluorocarbonée permet, pour une utilisation cosmétique ou pharmaceutique, de diminuer l'adsorption des toxiques en surface.

De préférence, les polymères ASE à chaine fluorocarbonée (ASE-F) sont composés des monomères suivants :

- de 20 à 75% molaire d'acide acrylique et/ou méthacrylique ;
- de 0 à 30% molaire d'acrylates d'alkyle en C1-C4 ; et
- de 10 à 55% molaire de méthacrylate de 2,2,2-trifluoroéthyle (MTFE) ou d'acrylate de trifluoroéthyle ou d''acrylate de 2-perfluorobutyléthyle ou d'acrylate de 2-perfluorohexyléthyle ou d'acrylate de 2-perfluorooctyléthyle.

De préférence encore, les polymères ASE à chaine fluorocarbonée (ASE-F) comprennent des monomères d'acide méthacrylique (AM), d'acrylate d'éthyle (AE) et de méthacrylate de 2,2,2-trifluoroéthyle (MTFE).
De façon avantageuse, les polymères ASE à chaine hydrocarbonée (ASE-F) comprennent :

- de 50 à 60% molaire d'acide méthacrylique (AM) ;
- de 5 à 15% molaire d'acrylate d'éthyle (AE) ; et
- de 30 à 40% molaire de méthacrylate de 2,2,2-trifluoroéthyle (MTFE) .

Plus préférentiellement encore, les polymères ASE à chaine fluorocarbonée (ASE-F) présentent des propriétés épaississantes particulièrement avantageuses lorsque le ratio des monomères acide méthacrylique (AM)/ acrylate d'éthyle (AE) est compris entre 7 et 0,1.Une formule générale de polymères ASE-H et ASE-F utilisables selon l'invention est illustrée à la figure 4. Une voie de synthèse des polymères ASE-H et ASE-F est détaillée à l'exemple 4, et est résumée à la figure 4, dans laquelle les indices a, b et c sont des nombres entiers, identiques ou différents, supérieurs à 1. Préférentiellement, a est compris entre 1 et 10 000 ; b est compris entre 1 et 5 000 et c est compris entre 1 et 8 000.
Les polymères associatifs utilisables selon l'invention sont constitués d'une structure macromoléculaire hydrophile sur laquelle est présent des groupements hydrophobes. Ces groupements hydrophobes, sont souvent des chaînons alkyles à courtes (ayant entre 1 et 6 atomes de carbone) ou longues chaînes (ayant plus de 6 atomes de carbone) capables de former des agrégats, des clusters, de type micellaire à partir d'une concentration dite d'agrégation critique. Ces agrégats sont appelés jonctions hydrophobes.
A ce jour, trois types de polymères associatifs différents, utilisables selon l'invention, sont commercialisés, il s'agit :

- des uréthanes à base d'oxyde d'éthylène rendus hydrophobes ou « hydrophobically modified ethylene-oxide urethane » (HEUR) ;
- des dérivés de cellulose ; et
- des émulsions pouvant gonfler en milieu alcalin modifiées pour les rendre hydrophobes ou « hydrophobically modified alkaliswellable emulsions » (HASE).

Ces trois types de polymères sont classés en deux catégories de polymères associatifs suivant leur architecture moléculaire :

1. les polymères dit téléchélique ; ou
2. les polymères de type peigne.

La représentation schématique de composés de type téléchélique et de type peigne est illustrée à la figure 1. Les polymères téléchéliques (HEUR) sont des chaînes linéaires de polymères contenant des groupements hydrophobes en fin de chaîne. Les polymères de type peigne (les dérivés de cellulose et les polymères HASE) sont des polymères contenant des chaînes hydrophobes le long du squelette.
Les polymères HASE sont généralement des copolymères d'acide méthacrylique (AM), d'acrylate d'éthyle (AE) et d'une quantité de groupes hydrophobes, qui sont des macromonomères ou des macromères.
Malgré la présence de groupements hydrophobes, qui sont des longues chaînes hydrocàrbonées, les polymères HASE sont solubles en milieu aqueux ce qui les rend particulièrement intéressants.
Tout comme les polymères ASE, les polymères HASE sont généralement préparés par polymérisation en émulsion à faible pH, ce qui permet d'obtenir des polymères de masses molaires comprises entre 300 000 et 1 800 000 g/mol.
Les propriétés rhéologiques des polymères HASE ont montré que la viscosité était fortement dépendante du pH. Dans le domaine de pH compris entre 2,4 et 4,5, le squelette du polymère se plie pour former une bobine compacte à cause de la faible qualité du solvant. La solution de polymère est laiteuse et consiste en des particules colloïdales insolubles. A pH 6, la viscosité augmente brusquement puis reste constante jusqu'à pH 11, les groupes carboxyles sur le squelette du polymère se dissolvent et la solution devient transparente. La chaîne de polymère ressemble alors à un polyélectrolyte ce qui provoque l'extension du squelette du polymère dû à la répulsion mutuelle des carboxylates et l'augmentation du volume hydrodynamique. En même temps, un grand nombre d'associations inter- et intramoléculaires entre les groupements hydrophobes sont formées ce qui entraine la construction d'un réseau au sein du milieu aqueux.

A pH basique, les polymères HASE combinent les propriétés des polyélectrolytes et les propriétés des polymères associatifs non-chargés. Au-delà de pH 11, la viscosité diminue lentement dû à l'effet de protection de la charge. D'autres facteurs peuvent faire varier la nature dynamique du polymère et la structure des polymères HASE, tels que notamment la concentration en sels dans le milieu. Lorsque celle-ci est importante, la viscosité diminue significativement. L'effet négatif de l'ajout de sels peut être compensé par l'addition d'un tensioactif. La concentration en tensioactif peut faire varier la viscosité du milieu. Une croissance de la concentration par un tensioactif non-ionique fait augmenter la viscosité du milieu, tandis qu'un tensioactif anionique fait croître la viscosité jusqu'à une concentration critique où celle-ci chute.

La viscosité du milieu peut également être diminuée lorsque la température augmente.

L'utilisation d'une base forte (NaOH) pour la neutralisation d'un polymère HASE engendre la dégradation de celui-ci après une période de quatre semaines (pH = 9,5). L'utilisation d'une base organique faible (1-amino-1-méthylpropanol) induit une stabilité des propriétés rhéologiques de la solution durant six semaines (pH = 9,5).

Les neutralisants monovalents peuvent également être recommandés. En effet lors de l'utilisation de molécule de base di- ou trivalente ayant ainsi le pouvoir de neutraliser plus d'une fonction acide carboxylique, il peut y avoir une réduction de la capacité du polymère à se dérouler et se déployer complètement.

Enfin, l'addition d'un solvant organique peut diminuer la viscosité du milieu en cassant les associations hydrophobes au sein du milieu aqueux et en solubilisant les groupements hydrophobes.

De préférence, les polymères selon l'invention sont des polymères HASE à macromères à chaine hydrocarbonée (HASE-H-RH ou.HASE-F-RH) ou une chaine fluorocarbonée (HASE-F-RF).

Les polymères associatifs HASE à macromères présentant une chaine hydrocarbonée (HASE-H-RH ou HASE-F-RH) ou une chaine fluorocarbonée (HASE-F-RF) selon l'invention répondent à la formule générale (III) suivante :

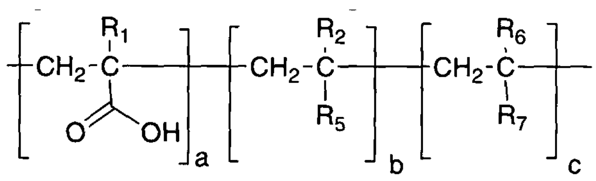

(III)

dans laquelle :

- R1, R2 et R6 représentent un atome d'hydrogène ou un groupement méthyle ;
- R5 représente $[Q]_{d1}$-$(CH_2)_n$-$(CX_2)_pX$ dans laquelle n est compris entre 1 et 30, d1 correspond à 0 ou à 1, et Q correspond à -C(O)-O ou - C(O)-NH- ; et :

  - lorsque X est un atome d'hydrogène, alors p est égal à 0 ;
  - lorsque X est un atome de fluor, alors p est compris entre 1 et 12 ;

  ou

  R5 représente $[Q]_{d2}$-$\alpha$, dans laquelle :

  - d2 correspond à 0 ou 1 ;
  - Q correspond à -C(O)-O ou -C(O)-NH- ; et
  - $\alpha$ correspond à -$C(CH_3)_3$ ; -$CH(CH_3)_2$ ; -$C(CH3)_2$-$CH_2$-$C(CH_3)_3$ ; -CN ; -$CH_2CH_2$-N+$(CH_3)_2(CH_2CO_2)^-$ ; -$CH_2CH_2$-NH-$C(CH_3)_3$ ; -$CH_2CH_2$-$N(CH_3)_2$ ; pyrrolidinone ; caprolactame ;

- R7 représente -$[Q']_{d3}$-$(OCH_2CH_2)_q$-$[Q'']_{d4}$-$(CH_2)_n(CX_2)_pX$ dans laquelle Q' correspond à -$CH_2$, C(O), O-C(O) ou -NH-C(O), n est compris entre 1 et 30, q est compris entre 1 et 150, d3 et d4 correspondent à 0 et/ou à 1, Q" correspond à -O-C(O) ou -NH-C(O) ; et :

  - lorsque X est un atome d'hydrogène, alors p est égal à 0 ;
  - lorsque X est un atome de fluor, alors p est compris entre 1 et 12 ;
  et dans laquelle les indices a et c sont des nombres entiers, identiques ou différents, supérieurs ou égaux à 1, et b est supérieur ou égal à 0.

Préférentiellement, a est compris entre 1 et 10 000 ; b est compris entre 0 et 10 000 et c est compris entre 1

et 5 000.

De préférence, les polymères HASE-H-RH à chaine hydrocarbonée sont composés de monomères de l'acide méthacrylique (AM), d'acrylates d'alkyle en C1-C4 et d'un macromère qui est un ester de formule générale (IV) :

$$CH_2{=}CH(CH_3)\text{-}C(O)(OCH_2CH_2)_qOC(O)(CH_2)_n\text{-}H \qquad (IV)$$

- dans laquelle q désigne un nombre compris entre 5 et 10 et n est compris entre 6 et 30 atomes de carbone.

De préférence encore, les polymères HASE-H-RH comprennent des monomères d'acide méthacrylique (AM), d'acrylate d'éthyle (AE), et d'un macromère qui est un ester de formule générale (IV) telle que définie ci-dessus, et répondent donc à la formule générale (V) suivante :

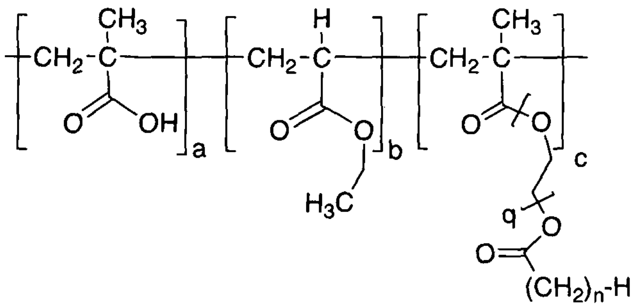

(V)

dans laquelle :

- q désigne un nombre compris entre 5 et 10 ;
- n est compris entre 1 et 30 ;
- a et c sont des nombres entiers, identiques ou différents, supérieurs ou égaux à 1, et b est supérieur ou égal à 0 ; préférentiellement, a est compris entre 1 et 10 000, b est compris entre 0 et 10 000 et c est compris entre 1 et 5 000.

De préférence encore, les polymères HASE-H-RH répondent à la formule générale (V) et comprennent :

- de 5 à 85% molaire d'acide méthacrylique (AM) ;
- de 5 à 60% molaire d'acrylate d'éthyle (AE) ; et
- de 1 à 90% molaire d'un macromère qui est un ester de formule générale (IV) définie ci-dessus.

Les polymères HASE-H-RH particulièrement préférés répondent à la formule (V) ci-dessus, et sont tels que :

- q équivaut à 7 et n est égal à 6 atomes de carbone (appelé par la suite polymère HASE-H-RH4) ;
- q équivaut à 7 et n est égal à 8 atomes de carbone (appelé par la suite polymère HASE-H-RH6) ;
- q équivaut à 9 et n est égal à 10 atomes de carbone (appelé par la suite polymère HASE-H-RH8).

De façon alternative, selon l'invention, le monomère d'acrylate d'éthyle (AE) du polymère HASE-H-RH de la formule (V) ci-dessus peut être remplacé par un monomère de méthacrylate de 2,2,2-trifluoroéthyle (MTFE) et correspond donc à un polymère HASE-F-RH qui répond à la formule générale (VI) suivante :

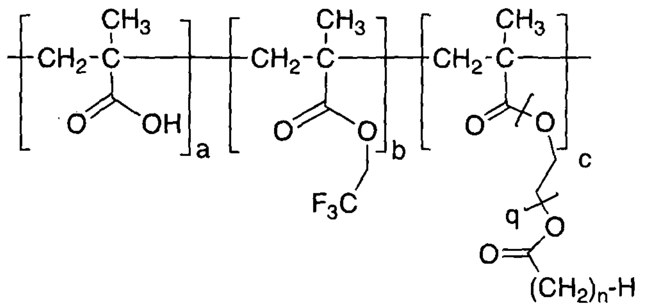

(VI)

dans laquelle :

- q désigne un nombre compris entre 5 et 10 ;
- n est compris entre 6 et 30 atomes de carbone ;
- a et c sont des nombres entiers, identiques ou différents, supérieurs ou égaux à 1, et b est supérieur ou égal à 0 ; préférentiellement, a est compris entre 1 et 10 000, b est compris entre 0 et 10 000 et c est compris entre 1 et 5 000.

De préférence encore, les polymères HASE-F-RH répondent à la formule générale (VI) ci-dessus et comprennent :

- de 30 à 85% molaire d'acide méthacrylique (AM) ;
- de 0 à 50% molaire de méthacrylate de 2,2,2-trifluoroéthyle (MTFE); et
- de 1 à 90% molaire d'un macromère qui est un ester de formule générale (IV).

Les polymères HASE-F-RH particulièrement préférés répondent à la formule (VI) ci-dessus, et sont tels que :

- q équivaut à 7 et n est égal à 6 atomes de carbone (appelé par la suite polymère HASE-F-RH4) ;
- q équivaut à 7 et n est égal à 8 atomes de carbone (appelé par la suite polymère HASE-F-RH6) ;
- q équivaut à 9 et n est égal à 10 atomes de carbone (appelé par la suite polymère HASE-F-RH8).

De façon avantageuse, le Demandeur a, par ailleurs, pu mettre en évidence que la substitution de chaînons hydrocarbonés par des fluorocarbonés sur le macromère était possible dans un squelette HASE.

En modifiant ainsi son squelette avec des macromères fluorocarbonés, le polymère modificateur de rhéologie selon l'invention peut disperser les micro- ou nanoparticules tout en apportant l'hydrophobie et l'oléophobie nécessaire à la protection contre les agents chimiques.

La structure des tels polymères HASE à chaine fluorocarbonée (HASE-F), également utilisables selon l'invention, répond à la formule générale (VII) suivante :

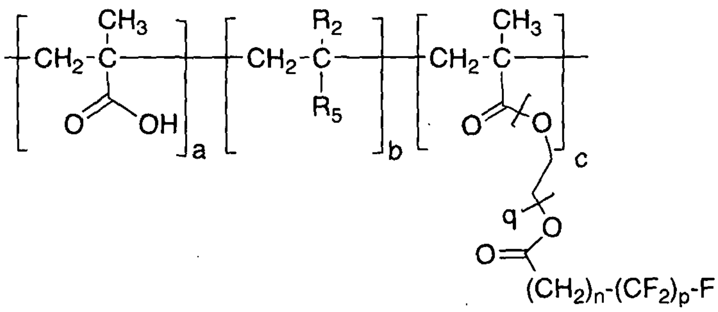

(VII)

dans laquelle :

- R2 représentent un atome d'hydrogène ou un groupement méthyle ;
- R5 représente $[Q]_{d1}$-$(CH_2)_n$-$(CX_2)_p$X dans laquelle n est compris entre 1 et 30, d1 correspond à 0 ou à 1, et Q

correspond à -C(O)-O ou -C(O)-NH- ; et :

- lorsque X est un atome d'hydrogène, alors p est égal à 0 ;
- lorsque X est un atome de fluor, alors p est compris entre 1 et 12 ;

ou R5 représente $[Q]_{d2}$-$\alpha$ dans laquelle :

- d2 correspond à 0 ou 1 ;
- Q correspond à -C(O)-O ou -C(O)-NH- ; et
- $\alpha$ correspond à $-C(CH_3)_3$ ; $-CH(CH_3)_2$ ; $-C(CH_3)_2-CH_2-C(CH_3)_3$ ; -CN ; $-CH_2CH_2-N+(CH_3)_2(CH_2CO_2^-)$ ; $-CH_2CH_2-NH-C(CH_3)_3$ ; $-CH_2CH_2-N(CH_3)_2$ ; pyrrolidinone ; caprolactame ;

- q désigne un nombre compris entre 1 et 150 ;
- n est un nombre entier compris entre 1 et 30 ;
- p est un nombre entier compris entre 1 et 12 ;

et dans laquelle les indices a et c sont des nombres entiers, identiques ou différents, supérieurs ou égaux à 1, et b est supérieur ou égal à 0 ; préférentiellement, a est compris entre 1 et 10 000, b est compris entre 0 et 10 000 et c est compris entre 1 et 5 000.

De préférence encore, les polymères HASE-F répondent à la formule générale (VIII) suivante :

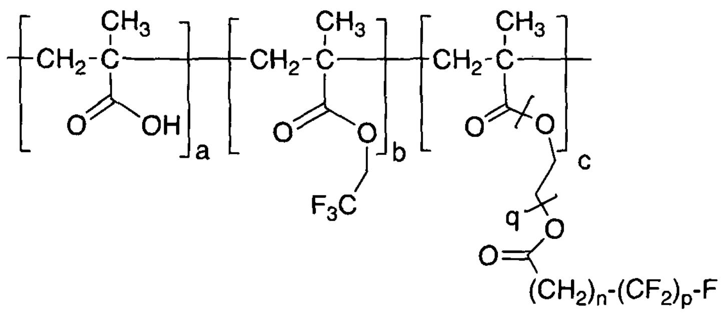

(VIII)

dans laquelle :

- q désigne un nombre compris entre 5 et 10 ;
- n est un nombre entier compris entre 1 et 30 ;
- p est un nombre entier compris entre 1 et 12 ;
- a et c sont des nombres entiers, identiques ou différents, supérieurs ou égaux à 1, et b est supérieur ou égal à 0 ; préférentiellement, a est compris entre 1 et 10 000, b est compris entre 0 et 10 000 et c est compris entre 1 et 5 000.

Plus préférentiellement encore, les polymères HASE-F répondent à la formule générale (VIII) ci-dessus dans laquelle :

- q équivaut à 5, 7 ou 9 ;
- n équivaut à 2 ;
- p équivaut à 4, 6 ou 8 ; et
- a et c sont des nombres entiers, identiques ou différents, supérieurs ou égaux à 1, et b est supérieur ou égal à 0 ; préférentiellement, a est compris entre 1 et 10 000, b est compris entre 0 et 10 000 et c est compris entre 1 et 5. 000. De préférence encore, les polymères HASE-F répondant à la formule générale (VIII) comprennent les monomères suivants :
- de 5 à 85% molaire d'acide méthacrylique (AM) ;
- de 1 à 70% molaire de méthacrylate de 2,2,2-trifluoroéthyle (MTFE) ; et
- de 1 à 50% molaire d'un macromère qui est un ester de formule générale (IX) :

$$- CH_2=CH(CH_3)-C(O)(OCH_2CH_2)_qOC(O)(CH_2)_2-(CF_2)_pF \qquad (IX)$$

dans laquelle :

- q équivaut à 5, 7 ou 9 ; et
- p équivaut à 4, 6 ou 8.

Les polymères HASE-F particulièrement préférés répondent à la formule (VIII) ci-dessus, et sont tels que :

- q équivaut à 5 et p équivaut à 4 (appelé par la suite polymère HASE-F-RF4) ;
- q équivaut à 7 et p équivaut à 6 (appelé par la suite polymère HASE-F-RF6) ;
- q équivaut à 7 et p équivaut à 6. (appelé par la suite polymère HASE-F-RF8) ;

Les polymères HASE décrits ci-dessus peuvent être synthétisés par le même procédé que pour les polymères ASE. Le détail d'une voie de synthèse des polymères HASE-H et HASE-F est détaillée à l'exemple 5.

De façon avantageuse, les polymères modificateurs ou adaptateurs de rhéologie utilisables dans les composés selon l'invention sont choisis :

i) parmi des polymères non-associatifs ASE-H de formule générale (I) suivante :

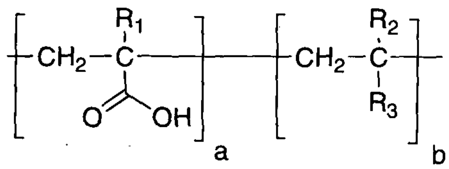

(I)

dans laquelle :

- R1 et R2 représentent un atome d'hydrogène ou un groupement méthyle $-CH_3$ ;
- R3 représente $[Q]_{d1}-(CH_2)_n-H$ dans laquelle n est compris entre 1 et 30, d1 correspond à 0 ou à 1, et Q correspond à -C(O)-O ou -C(O)-NH- ;
  ou
  R3 représente $[Q]_{d2}-\alpha$, dans laquelle :
  - d2 correspond à 0 ou 1 ;
  - Q correspond à -C(O)-O ou -C(O)-NH- ; et
  - $\alpha$ correspond à $-C(CH_3)3$; $-CH(CH_3)_2$; $-C(CH_3)_2-CH2-C(CH_3)_3$ ; -CN ; $-CH_2CH_2-N+(CH_3)_2(CH_2CO_2^-)$ ; $-CH_2CH_2-NH-C(CH_3)_3$ ; $-CH_2CH_2-N(CH_3)_2$ ; pyrrolidinone ; caprolactame ;
    et dans laquelle les indices a et b sont des nombres entiers, identiques ou différents, supérieurs à 1 ;
    ou

ii) parmi des polymères non-associatifs ASE-F de formule générale (II) suivante :

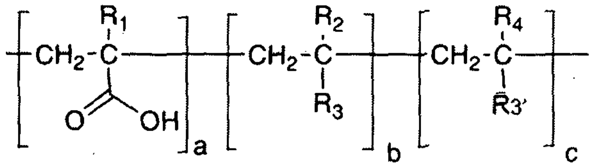

(II)

dans laquelle :

- R1, R2 et R4 représentent un atome d'hydrogène ou un groupement méthyle $-CH_3$ ;
- R3 représente $[Q]_{d1}-(CH_2)_n-H$ dans laquelle n est compris entre 1 et 30, d1 correspond à 0 ou à 1, et Q correspond à -C(O)-O ou -C(O)-NH- ;

ou

R3 représente $[Q]_{d2}$-α, dans laquelle :

- d2 correspond à 0 ou 1 ;
- Q correspond à -C(O)-O ou -C(O)-NH- ; et
- α correspond à $-C(CH_3)_3$ ; $-CH(CH_3)2$ ; $-C(CH_3)_2-CH_2-C(CH_3)_3$ ; -CN ; $-CH_2CH_2-N+(CH_3)_2(CH_2CO_2^-)$ ; $-CH_2CH_2-NH-C(CH_3)_3$ ; $-CH_2CH_2-N(CH_3)_2$ ; pyrrolidinone ; caprolactame ;

- R3' représente $[Q]_{d1}-(CH_2)_n-(CX_2)_pX$ dans laquelle n est compris entre 1 et 30, d1 correspond à 0 ou à 1, Q correspond à -C(O)-O ou -C(O)-NH- , X est un atome de fluor F et p est compris entre 1 et 12 ;
  et dans laquelle les indices a et c sont des nombres entiers, identiques ou différents, supérieurs à 1 et b est supérieur ou égal à 0 ;
  ou

iii) parmi les polymères associatifs HASE à macromères à chaine hydrocarbonée (HASE-H-RH ou HASE-F-RH) ou à chaine fluorocarbonée (HASE-F-RF) répondant à la formule générale (III) suivante :

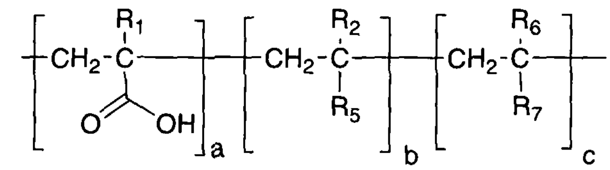

(III)

dans laquelle :

- R1, R2 et R6 représentent un atome d'hydrogène ou un groupement méthyle ;
- R5 représente $[Q]_{d1}-(CH_2)_n-(CX_2)_pX$ dans laquelle n est compris entre 1 et 30, d1 correspond à 0 ou à 1, et Q correspond à -C(O)-O ou - C(O)-NH- ; et :

  - lorsque X est un atome d'hydrogène, alors p est égal à 0 ;
  - lorsque X est un atome de fluor, alors p est compris entre 1 et 12 ;
    ou
    R5 représente $[Q]_{d2}$-α, dans laquelle :

    - d2 correspond à 0 ou 1 ;
    - Q correspond à -C(O)-O ou -C(O)-NH- ; et
    - α correspond à $-C(CH_3)_3$ ; $-CH(CH_3)_2$ ; $-C(CH_3)_2-CH_2-C(CH_3)_3$ ; -CN ; $-CH_2CH_2-N+(CH_3)_2(CH_2CO_2^-)$ ; $-CH_2CH_2-NH-C(CH_3)_3$ ; $-CH_2CH_2-N(CH_3)2$ ; pyrrolidinone ; caprolactame ;

- R7 représente $-[Q']_{d3}-(OCH_2CH_2)_q-[Q'']_{d4}-(CH_2)_n(CX_2)_pX$ dans laquelle Q' correspond à $-CH_2$, C(O), O-C(O) ou -NH-C(O), n est compris entre 1 et 30, q est compris entre 1 et 150, d3 et d4 correspondent à 0 et/ou à 1, Q" correspond à -O-C(O) ou -NH-C(O) ; et :

  - lorsque X est un atome d'hydrogène, alors p est égal à 0 ;
  - lorsque X est un atome de fluor, alors p est compris entre 1 et 12 ;
    et dans laquelle les indices a et c sont des nombres entiers, identiques ou différents, supérieurs ou égaux à 1, et b est supérieur ou égal à 0.

De façon particulièrement avantageuse, les polymères modificateurs ou adaptateurs de rhéologie sont choisis parmi :

- les polymères ASE-H qui comprennent les monomères suivants :

  - de 10 à 20% molaire d'acide méthacrylique (AM) ;
  - de 80 à 90% molaire d'acrylate d'éthyle (AE) ;

ou

- les polymères ASE à chaine hydrocarbonée (ASE-F) qui comprennent les monomères suivants :

  - de 50 à 60% molaire d'acide méthacrylique (AM) ;
  - de 5 à 15% molaire d'acrylate d'éthyle (AE) ; et
  - de 30 à 40% molaire de méthacrylate de 2,2,2-trifluoroéthyle (MTFE) ;

ou

- les polymères HASE-H-RH qui répondent à la formule générale (V) et qui comprennent :

  - de 5 à 85% molaire d'acide méthacrylique (AM) ;
  - de 5 à 60% molaire d'acrylate d'éthyle (AE) ; et
  - de 1 à 90% molaire d'un macromère qui est un ester de formule générale (IV) définie ci-dessus ;

ou

- les polymères HASE-F-RH qui répondent à la formule générale (VI) et qui comprennent :

  - de 30 à 85% molaire d'acide méthacrylique (AM) ;
  - de 0 à 50% molaire de méthacrylate de 2,2,2-trifluoroéthyle (MTFE); et
  - de 1 à 90% molaire d'un macromère qui est un ester de formule générale (IV).

De façon plus avantageuse encore, les polymères modificateurs ou adaptateurs de rhéologie sont choisis parmi les polymères :

- ASE-H ;
- ASE-F ;
- HASE-H-RH4 ;
- HASE-H-RH6 ;
- HASE-H-RH8 ;
- HASE-F-RH4 ;
- HASE-F-RH6 ;
- HASE-F-RH8 ;
- HASE-F-RF4 ;
- HASE-F-RF6 ;
- HASE-F-RF8.

Par ailleurs, le Demandeur a pu mettre en évidence, comme cela est décrit à l'exemple 6 que l'augmentation du taux molaire de macromères hydrocarbonés ou fluorés dans les polymères HASE permet d'augmenter la viscosité des polymères.

Aussi, de préférence, le pourcentage molaire de macromères dans les polymères HASE est compris entre 1 et 85% molaire. Plus préférentiellement, le pourcentage molaire est compris entre 3 et 50% molaire de macromères.

Plus préférentiellement encore, le pourcentage molaire est de 13,5% molaire de macromères.

La caractérisation de l'ensemble des polymères en rhéologie et goniométrie a permis de mettre en évidence que les polymères HASE-F-RF8 à 3,3% ; 13,5% et 45,9% molaire de macromères étaient particulièrement préférés notamment pour leur oléophobie et la viscosité de leurs solutions.

De façon particulièrement avantageuse, le polymère selon l'invention est le polymère HASE-F-RF8, préférentiellement à 13,5% molaire de macromère.

Le composé selon l'invention est formé par l'association covalente d'une ou plusieurs micro- ou nanoparticules fonctionnalisées amines telles que décrites ci-dessus, avec un ou plusieurs polymères modificateurs de rhéologie tels que décrits ci-dessus.

En outre, les composés selon l'invention possédant encore des fonctions amines libres situées sur les micro- ou nanoparticules, il est possible d'y greffer d'autres molécules. A titre d'exemple non limitatif de molécule additionnelle pouvant être greffée, on peut citer l'acide 4, 4, 5, 5, 6, 6, 7, 7, 7-nonafluoroheptanoïque (RF4), l'acide 4,4,5,5,6,6,7,7,8,8,9,9,9-tridecafluorononanoïque (RF6) et l'acide 4,4,5,5,6,6,7, 7,8,8,9,9,10,10,11,11,11-heptadecafluoroundécanoïque (RF8).

Ces molécules sont préférentiellement greffées, de manière covalente, au composé HASE-F-RF8/Si selon l'invention.

Afin d'obtenir un effet protecteur optimal contre les agents toxiques, il est préférable d'obtenir une dispersion

homogène des micro- ou nanoparticules au sein de la matrice polymérique, en éliminant toute agrégation.

En outre, les micro- ou nanoparticules étant pulvérulentes, elles peuvent provoquer l'inflammation des poumons en s'y fixant dessus (par inhalation) ou le passage dans le sang (par pénétration cutanée). Aussi, afin d'éviter ce type de toxicité et afin de contrôler la dispersion, le Demandeur a greffé de manière covalente les micro- ou nanoparticules aux polymères. Ce greffage peut être réalisé par la méthode dite de « grafting to ».

L'association covalente de micro- ou nanoparticules avec des polymères peut être réalisée en faisant réagir des micro- ou nanoparticules fonctionnalisées amine sur des polymères possédants des fonctions acides carboxyliques (réaction d'amidation) en phase aqueuse. Le greffage des micro- ou nanoparticules sur les polymères peut être fait *via* l'estérification ou *via* l'amidation.

De préférence, le greffage est réalisé par l'amidation qui est une réaction dont le rendement est supérieur à l'estérification du fait de la nucléophilie plus importante de l'azote comparé à l'alcool.

Des exemples de greffage de micro- ou nanoparticules fonctionnalisées amine avec des polymères associatifs ou non-associatifs sont détaillés à l'exemple 7.

Comme cela a été décrit ci-dessus, avant l'étape de greffage, les micro- ou nanoparticules d'oxyde d'aluminium ($Al_2O_3$), d'oxyde de cuivre (CuO), d'oxyde de fer ($Fe_3O_4$ ou $\gamma$-$Fe_2O_3$), d'oxyde de magnésium (MgO), de silice ($SiO_2$), de dioxyde de titane ($TiO_2$) ou d'oxyde de zinc (ZnO), ont été synthétisées puis fonctionnalisées amine pour réagir sur les fonctions acides des polymères modificateurs ou adaptateurs de rhéologie.

La liaison covalente présente de nombreux avantages. Elle va permettre d'une part d'empêcher la pénétration des micro- ou nanoparticules par voie respiratoire mais aussi par voie cutanée au sein du corps humain ou animal, et d'autre part de contrôler la dispersion des micro- ou nanoparticules au sein de la matrice dans laquelle les micro- ou nanoparticules sont liées. Pour permettre ceci, le polymère, ou la matrice polymérique, contient des composés dont les fonctions permettent de réagir avec les micro- ou nanoparticules mais aussi qui peuvent être utilisés dans un topique.

Selon l'invention, les micro- ou nanoparticules sont liées de façon covalente à un polymère modificateur de rhéologie contenant des monomères fluorés pour d'une part une propriété filmogène augmentée et d'autre part une augmentation de l'hydrophobie et de l'oléophobie afin de « repousser » les toxiques. L'objectif est également d'apporter un caractère filmogène optimum pour une protection de surface grâce aux différents polymères, et à une interaction plus ou moins forte entre les micro- ou nanoparticules.

Les micro- ou nanoparticules, en fonction de leur nature vont permettre une adsorption (par exemple pour les nanoparticules de silice) ou une destruction (par exemple pour les nanoparticules de dioxyde de titane) par photodégradation des toxiques arrivant au contact du film avant leur pénétration dans la peau ou dans le support.

De façon avantageuse, selon l'invention, les nanoparticules sont préférées aux microparticules. En effet, le choix des nanoparticules est orienté par leur très grande surface spécifique par rapport à des microparticules afin d'augmenter l'efficacité d'adsorption. Ces nano-objets sont, de plus, transparents permettant à la protection de rester invisible. Ce réseau micro- ou nanoparticulaire est dispersible dans un milieu aqueux basique grâce à la présence d'acide carboxylique dans le copolymère et peut donc facilement être intégré dans un topique.

Le Demandeur s'est également intéressé à faire varier le nombre d'équivalent d'oxyde d'aluminium ($Al_2O_3$), d'oxyde de cuivre (CuO), d'oxyde de fer ($Fe_3O_4$ ou $\gamma$-$Fe_2O_3$), d'oxyde de magnésium (MgO), de silice ($SiO_2$), de dioxyde de titane ($TiO_2$) ou d'oxyde de zinc (ZnO) dans les composés selon l'invention, c'est-à-dire le nombre d'équivalent de fonctions amines portées par les micro- ou nanoparticules d'oxyde d'aluminium ($Al_2O_3$), d'oxyde de cuivre (CuO), d'oxyde de fer ($Fe_3O_4$ ou $\gamma$-$Fe_2O_3$), d'oxyde de magnésium (MgO), de silice ($SiO_2$), de dioxyde de titane ($TiO_2$) ou d'oxyde de zinc (ZnO) fonctionnalisées amine par rapport au nombre d'équivalent de fonctions acides portées par le polymère.

Le Demandeur a ainsi pu mettre en évidence, comme cela est décrit à l'exemple 13, qu'un nombre d'équivalent, par exemple de silice, inférieur ou égal à 1 permet d'obtenir des composés présentant des propriétés améliorées, par exemple de dispersion et d'oléophobie.

Le rapport polymère/micro- ou nanoparticules a été calculé par le nombre d'équivalents de fonctions acides portées par le polymère en fonction du nombre d'équivalents de fonctions amines portées par les micro- ou nanoparticules (pour 1 équivalent de fonctions acides contenus dans le polymère, 1 équivalent de fonctions amines a été introduit) (où pour 1 eq de fonctions acides, 0,3 eq de fonctions amines introduit).

De préférence, le nombre d'équivalent d'oxyde d'aluminium ($al_2O_3$), d'oxyde de cuivre (CuO), d'oxyde de fer ($Fe_3O_4$ ou $\gamma$-$Fe_2O_3$), d'oxyde de magnésium (MgO), de silice ($SiO_2$), de dioxyde de titane ($TiO_2$) ou d'oxyde de zinc (ZnO) dans les composés selon l'invention est compris entre 0,05 et 1. Plus préférentiellement, il est compris entre 0,3 et 0,8. Plus préférentiellement encore, le nombre d'équivalent est de 0,3.

L'invention a pour deuxième objet un topique protecteur comprenant un composé selon l'invention, dans un milieu pharmaceutiquement et/ou cosmétiquement acceptable.

Selon un mode particulier de réalisation de l'invention, le topique protecteur comprend en outre un ou plusieurs agents détoxifiants et/ou un ou plusieurs polymères complémentaires.

Les agents détoxifiants sont non-toxiques et dermatologiquement acceptables.

A titre d'exemple non limitatifs d'agents détoxifiants, on peut citer le peroxyde de benzoyle, le peroxyde de

zinc, le monoperoxyphtalate de magnésium, le perborate de sodium, le percarbonate de sodium, le permanganate de potassium, le peroxyde carbamide (peroxyde d'urée), le peroxyde de calcium, le dioxyde de titane, et les agents soufrés comme la N-acétyl cystéine, l'acide perpropionique, le peroxyde de magnésium ou des agents neutralisants comme l'oxyde de zinc, les agents complexants comme l'acide étidronique et son sel tétrasodique, l'acide 1-hydroxyéthylènediamine (1,1-diphosphonique), le propionate de sodium, l'hydroxy carbonate de magnésium, le nitrate de potassium ou l'acide thioglycolique.

Le pourcentage massique de ces agents détoxifiants est préférentiellement compris entre 0,001 et 60% du poids total de la composition.

En outre, le topique protecteur selon l'invention peut également comprendre un ou plusieurs polymères complémentaires choisis parmi le polyperfluorométhyl-isopropyl éther, le copolymère de diméthicone et de vinyldiméthicone, le copolymère de diéthylèneglycol, d'acide adipique et de glycérine, le Polysilicone-8 et les polyglycérides d'acides oléique/linoléique/ linolénique dont le rôle consiste à rendre les compositions plus fluides ou plus agréables à appliquer. Le polyperfluorométhylisopropyl éther est notamment commercialisé sous la marque Fomblin™ HC.

Le polymère de diméthicone et de vinyldiméthicone est notamment commercialisé sous la marque Silicone Elastomer Blend DC9041™. Le copolymère de diéthylèneglycol, d'acide adipique et de glycérine est notamment commercialisé sous la marque Lexorez 100™. Le polysilicone-8 est notamment commercialisé sous la marque Silicones Plus Polymer VS80Dry™. Ces polymères complémentaires sont introduits en pourcentage massique variant par exemple de 0,005% à 10% du poids total de la composition. La composition dermatologique et/ou cosmétique selon l'invention peut en outre renfermer des agents émollients, des agents adoucissants, des conservateurs ou encore des parfums.

Les topiques protecteurs peuvent se présenter sous forme de gel, de lotion, d'émulsion huile dans l'eau ou eau dans l'huile, de dispersion, de lait, de crème, d'onguent, de mousse, de bâton, de spray, d'aérosols ou sous toute autre forme appropriée à une application topique.

Les topiques protecteurs selon l'invention sont destinés à être appliquées sur la peau, en prévention et en prévision d'un éventuel contact avec des agents chimiques toxiques. Ils sont appliqués en couche suffisante sur le visage et sur toutes les parties du corps susceptibles d'être exposées aux agents chimiques toxiques. Les topiques protecteurs contiennent donc préférentiellement également une base protectrice barrière et un ou plusieurs agents détoxifiants, afin, d'une part de retarder la pénétration cutanée des agents chimiques toxiques et/ou d'autre part de les neutraliser avant qu'ils puissent atteindre leurs sites d'action dans un organisme vivant.

Selon un mode particulier de réalisation, les composés objet de l'invention sont introduits dans la crème BariedermTech™ commercialisée par la société Uriage™ qui contient notamment de l'eau, le complexe Poly-2p® constitué de polymère de pyrrolidone et de polymère de phosphorylcholine biomimétique (Poly-2P™), de Glycérine et d'alcool.

L'invention a pour troisième objet un composé ou un topique protecteur selon l'invention à titre de médicament.

En effet, en agissant sur la barrière cutanée, le composé ou le topique protecteur selon l'invention possède des propriétés préventives à l'égard d'affections humaines ou animales. Ainsi, le composé ou le topique protecteur selon l'invention peut également être employé comme une substance ou composition utilisable chez l'homme ou l'animal ou pouvant leur être administrée, en vue de corriger ou modifier leurs fonctions physiologiques en exerçant une action pharmacologique, immunologique ou métabolique. Le composé ou le topique protecteur selon l'invention trouvent des applications en médecine humaine, notamment en dermatologie, pour la prévention des irritations cutanées ou des allergies.

L'invention a donc pour quatrième objet un composé ou un topique protecteur selon l'invention pour son utilisation dans la prévention des irritations ou des allergies.

Les irritations peuvent être par exemple des irritations cutanées ou des allergies liées à des pratiques professionnelles à risque ou des activités de bricolage.

Les pratiques professionnelles à risque incluent l'utilisation d'agent du risque chimique ou biologique, par exemple en milieu hospitalier ou militaire.

Les activités de bricolage incluent l'utilisation de produits chimiques par exemple dans des activités de peinture, mécanique, jardinage ou encore rénovation de meubles.

L'invention a pour cinquième objet l'utilisation d'un composé ou d'un topique protecteur selon l'invention, pour la protection ou la décontamination cutanée, notamment due aux agents du risque biologique ou chimique.

En effet, le composé ou le topique protecteur peut également être utilisé pour des applications non-thérapeutiques, par exemple cosmétique, c'est-à-dire qu'il trouve une application comme barrière épidermique de protection contre des agressions extérieures.

Ainsi, l'invention concerne également l'utilisation cosmétique du composé ou du topique protecteur selon l'invention, pour la protection cutanée contre les agents toxiques de la famille des composés organophosphorés (COP) que regroupent les pesticides (POP) et les neurotoxiques organophosphorés (NOP), ou contre des agents vésicants tels que les ypérites au soufre ou à l'azote, ou la lewisite et l'oxime de phosgène.

L'invention concerne également l'utilisation cosmétique du composé ou du topique protecteur selon l'invention, pour la protection contre les rayons ultraviolets UVA et/ou UVB provenant d'une source naturelle ou artificielle.

Pour cette utilisation contre les rayons ultraviolets UVA et/ou UVB, préférentiellement, le composé associe des micro- ou nanoparticules de dioxyde de titane ($TiO_2$), sous forme anatase, fonctionnalisées amine avec des polymères modificateurs ou adaptateurs de rhéologie.

Enfin, l'invention a encore pour objet des procédés de synthèse des composés selon l'invention. De tels procédés de synthèse sont décrits dans les exemples ci-dessous.

Le procédé de préparation préféré d'un composé selon l'invention comprend les étapes de :

- mélange d'un agent de couplage avec un catalyseur ;
- ajout du mélange obtenu à une solution de polymères modificateurs ou adaptateurs de rhéologie choisis parmi les polymères non-associatifs ou les polymères associatifs, dans de l'eau ;
- agitation du mélange réactionnel ;
- ajout des micro- ou nanoparticules fonctionnalisées d'oxyde d'aluminium ($Al_2O_3$), d'oxyde de magnésium (MgO), de silice ($SiO_2$), de dioxyde de titane ($TiO_2$), d'oxyde de zinc (ZnO) ou d'oxyde de cuivre (CuO) présentant un diamètre nominal compris entre 5 et 1 500 nm, préalablement dispersées en phase aqueuse au mélange réactionnel ;
- purification du milieu réactionnel par dialyse ; et
- récupération du composé formé par une ou plusieurs micro- ou nanoparticules fonctionnalisées amines associées de façon covalente à un ou plusieurs polymères modificateurs de rhéologie.

De préférence, l'agent de couplage est l'hydrochlorure de N-(3-diméthylaminopropyl)-N'-éthylcarbodiimide (EDC) et le catalyseur est le N-hydroxysuccinimide (NHS).

Enfin, l'invention a pour dernier objet un polymère associatif ou non-associatif tel que défini ci-dessus, à titre de polymère modificateur de rhéologie.

Le polymère selon l'invention est un polymère non-associatif ou un polymère associatif répondant aux formules générales (I), (II) ou (III) telles que détaillées ci-dessus.

Ainsi, selon l'invention, le polymère répond à la formule générale (I) suivante :

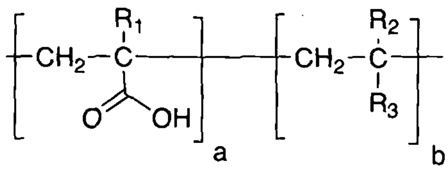

(I)

dans laquelle :

- R1 et R2 représentent un atome d'hydrogène ou un groupement méthyle $-CH_3$ ;
- R3 représente $[Q]_{d1}-(CH_2)_n-H$ dans laquelle n est compris entre 1 et 30, d1 correspond à 0 ou à 1, et Q correspond à -C(O)-O ou -C(O)-NH- ;
  ou
  R3 représente $[Q]_{d2}-\alpha$, dans laquelle :
  - d2 correspond à 0 ou 1 ;
  - Q correspond à -C(O)-O ou -C(O)-NH- ; et
  - $\alpha$ correspond à $-C(CH_3)_3$ ; $-CH(CH_3)_2$ ; $-C(CH_3)_2-CH_2-C(CH_3)_3$ ; $-C_N$ ; $-CH_2CH_2-N+(CH_3)_2(CH_2CO_2^-)$ ; $-CH_2CH_2-NH-C(CH_3)_3$ ; $-CH_2CH_2-N(CH_3)_2$ ; pyrrolidinone ; caprolactame ;
    et dans laquelle les indices a et b sont des nombres entiers, identiques ou différents, supérieurs à 1 ; préférentiellement, a est compris entre 1 et 10 000 et b est compris entre 1 et 20 000 ;
    ou
    répond à la formule (II) suivante :

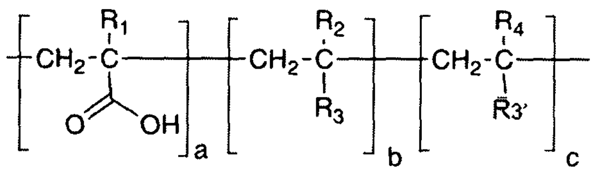

(II)

dans laquelle :

- R1, R2 et R4 représentent un atome d'hydrogène ou un groupement méthyle $-CH_3$ ;
- R3 représente $[Q]_{d1}-(CH_2)_n-H$ dans laquelle n est compris entre 1 et 30, d1 correspond à 0 ou à 1, et Q correspond à -C(O)-O ou -C(O)-NH- ;
  ou
  R3 représente $[Q]_{d2}-\alpha$, dans laquelle :
  - d2 correspond à 0 ou 1 ;
  - Q correspond à -C(O)-O ou -C(O)-NH- ; et
  - $\alpha$ correspond à $-C(CH_3)_3$; $-CH(CH_3)_2$ ; $-C(CH_3)_2-CH_2-C(CH_3)_3$ ; -CN ; $-CH_2CH_2-N+(CH_3)_2(CH_2CO_2^-)$ ; $-CH_2CH_2-NH-C(CH_3)_3$ ; $-CH_2CH_2-N(CH_3)_2$ ; pyrrolidinone ; caprolactame ;
- R3' représente $[Q]_{d1}-(CH_2)_n-(CX_2)_pX$ dans laquelle n est compris entre 1 et 30, d1 correspond à 0 ou à 1, Q correspond à -C(O)-O ou -C(O)-NH- , X est un atome de fluor F et p est compris entre 1 et 12 ;
  et dans laquelle les indices a et c sont des nombres entiers, identiques ou différents, supérieurs à 1 et b est supérieur ou égal à 0 ; préférentiellement, a est compris entre 1 et 10 000, b est compris entre 0 et 5 000 et c est compris entre 1 et 8 000 ;
  ou
  répond à la formule (III) suivante :

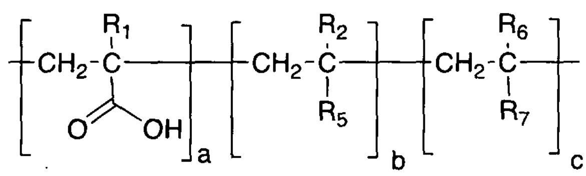

(III)

dans laquelle :

- R1, R2 et R6 représentent un atome d'hydrogène ou un groupement méthyle ;
- R5 représente $[Q]_{d1}-(CH_2)_n-(CX_2)_pX$ dans laquelle n est compris entre 1 et 30, d1 correspond à 0 ou à 1, et Q correspond à -C(O)-O ou - C(O)-NH- ; et :
  - lorsque X est un atome d'hydrogène, alors p est égal à 0 ;
  - lorsque X est un atome de fluor, alors p est compris entre 1 et 12 ;

  ou
  R5 représente $[Q]_{d2}-\alpha$, dans laquelle :
  - d2 correspond à 0 ou 1 ;
  - Q correspond à -C(O)-O ou -C(O)-NH- ; et
  - $\alpha$ correspond à $-C(CH_3)_3$ ; $-CH(CH_3)_2$ ; $-C(CH_3)_2-CH_2-C(CH_3)_3$ ; -CN ; $-CH_2CH_2-N+(CH_3)_2(CH_2CO_2^-)$ ; $-CH_2CH_2-NH-C(CH_3)_3$ ; $-CH_2CH_2-N(CH_3)_2$ ; pyrrolidinone ; caprolactame ;
- R7 représente $-[Q']_{d3}-(OCH_2CH_2)_q-[Q'']_{d4}-(CH_2)_n(CX_2)_pX$ dans laquelle Q' correspond à $-CH_2$, C(O),

O-C(O) ou -NH-C(O), n est compris entre 1 et 30, q est compris entre 1 et 150, d3 et d4 correspondent à 0 et/ou à 1, Q'' correspond à -O-C(O) ou -NH-C(O) ; et :

- lorsque X est un atome d'hydrogène, alors p est égal à 0 ;
- lorsque X est un atome de fluor, alors p est compris entre 1 et 12 ;

et dans laquelle les indices a et c sont des nombres entiers, identiques ou différents, supérieurs ou égaux à 1, et b est supérieur ou égal à 0 ; préférentiellement, a est compris entre 1 et 10 000, b est compris entre 0 et 10 000 et c est compris entre 1 et 5 000.

De préférence, les polymères ASE à chaine hydrocarbonée (ASE-H) comprennent des monomères d'acide acrylique et/ou méthacrylique et d'acrylates d'alkyle en C1-C4.

De façon avantageuse, les polymères ASE à chaine hydrocarbonée (ASE-H) comprennent :

- de 5 à 50% molaire d'acide acrylique et/ou méthacrylique ;
- de 50 à 95% molaire d'acrylates d'alkyle en C1-C4.

Plus préférentiellement, les polymères ASE-H comprennent les monomères suivants :

- de 10 à 20% molaire d'acide méthacrylique (AM) ;

de 80 à 90% molaire d'acrylate d'éthyle (AE).De préférence, les polymères non-associatifs ASE-F répondent à la formule générale (II) suivante :

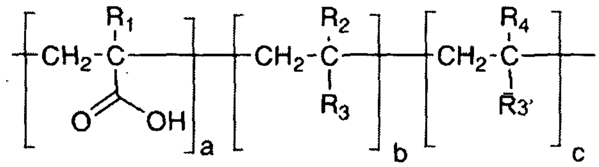

(II)

dans laquelle :

- R1, R2 et R4 représentent un atome d'hydrogène ou un groupement méthyle $-CH_3$ ;
- R3 représente $[Q]_{d1}-(CH_2)_n-H$ dans laquelle n est compris entre 1 et 30, d1 correspond à 0 ou à 1, et Q correspond à -C(O)-O ou -C(O)-NH- ;
  ou
  R3 représente $[Q)_{d2}-\alpha$, dans laquelle :
  - d2 correspond à 0 ou 1 ;
  - Q correspond à -C(O)-O ou -C(O)-NH- ; et
  - $\alpha$ correspond à $-C(CH_3)_3$ ; $-CH(CH_3)_2$ ; $-C(CH_3)_2-CH_2-C(CH_3)_3$ ; -CN ; $-CH_2CH_2-N+(CH_3)_2(CH_2CO_2^-)$ ; $-CH_2CH_2-NH-C(CH_3)_3$ ; $-CH_2CH_2-N(CH_3)_2$ ; pyrrolidinone ; caprolactame ;
- R3' représente $[Q]_{d1}-(CH_2)_n-(CX_2)_pX$ dans laquelle n est compris entre 1 et 30, d1 correspond à 0 ou à 1, Q correspond à -C(O)-O ou -C(O)-NH- , X est un atome de fluor F et p est compris entre 1 et 12 ;
  et dans laquelle les indices a et c sont des nombres entiers, identiques ou différents, supérieurs à 1 et b est supérieur ou égal à 0 ; préférentiellement, a est compris entre 1 et 10 000, b est compris entre 0 et 5 000 et c est compris entre 1 et 8 000.

Ces polymères ASE-F sont préférentiellement constitués de monomères :

- d'acide méthacrylique (AM),
- de méthacrylate de 2,2,2-trifluoroéthyle (MTFE) ou d'acrylate de trifluoroéthyle ou d''acrylate de 2-perfluorobutyléthyle ou d'acrylate de 2-perfluorohexyléthyle ou d'acrylate de 2-perfluorooctyléthyle ; et
- d'acrylate d'alkyle en C1-C4, préférentiellement l'acrylate d'éthyle (AE).

L'introduction d'une chaîne fluorocarbonée permet, pour une utilisation cosmétique ou pharmaceutique, de diminuer l'adsorption des toxiques en surface.

De préférence, les polymères ASE à chaine fluorocarbonée (ASE-F) sont composés des monomères suivants :

- de 20 à 75% molaire d'acide acrylique et/ou méthacrylique ;
- de 0 à 30% molaire d'acrylates d'alkyle en C1-C4 ; et
- de 10 à 55% molaire de méthacrylate de 2,2,2-trifluoroéthyle (MTFE) ou d'acrylate de trifluoroéthyle ou d''acrylate de 2-perfluorobutyléthyle ou d'acrylate de 2-perfluorohexyléthyle ou d'acrylate de 2-perfluorooctyléthyle.

De préférence encore, les polymères ASE à chaine fluorocarbonée (ASE-F) comprennent des monomères d'acide méthacrylique (AM), d'acrylate d'éthyle (AE) et de méthacrylate de 2,2,2-trifluoroéthyle (MTFE).

De façon avantageuse, les polymères ASE à chaine hydrocarbonée (ASE-F) comprennent :

- de 50 à 60% molaire d'acide méthacrylique (AM) ;
- de 5 à 15% molaire d'acrylate d'éthyle (AE) ; et
  de 30 à 40% molaire de méthacrylate de 2,2,2-trifluoroéthyle (MTFE).

De préférence, les polymères HASE-H-RH à chaine hydrocarbonée sont composés de monomères de l'acide méthacrylique (AM), d'acrylates d'alkyle en C1-C4 et d'un macromère qui est un ester de formule générale (IV) :

$$CH_2{=}CH(CH_3)\text{-}C(O)(OCH_2CH_2)_qOC(O)(CH_2)_n\text{-}H \qquad (IV)$$

- dans laquelle q désigne un nombre compris entre 5 et 10 et n est compris entre 6 et 30 atomes de carbone.

De préférence encore, les polymères HASE-H-RH comprennent des monomères d'acide méthacrylique (AM), d'acrylate d'éthyle (AE), et d'un macromère qui est un ester de formule générale (IV) telle que définie ci-dessus, et répondent donc à la formule générale (V) suivante :

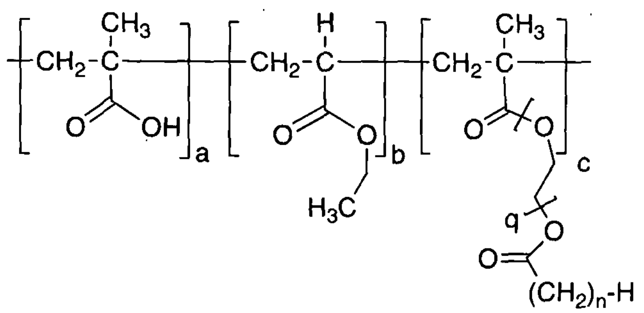

(V)

dans laquelle :

- q désigne un nombre compris entre 5 et 10 ;
- n est compris entre 1 et 30 ;
- a et c sont des nombres entiers, identiques ou différents, supérieurs ou égaux à 1, et b est supérieur ou égal à 0 ; préférentiellement, a est compris entre 1 et 10 000, b est compris entre 0 et 10 000 et c est compris entre 1 et 5 000.

De préférence encore, les polymères HASE-H-RH répondent à la formule générale (V) et comprennent :

- de 5 à 85% molaire d'acide méthacrylique (AM) ;
- de 5 à 60% molaire d'acrylate d'éthyle (AE) ; et
- de 1 à 90% molaire d'un macromère qui est un ester de formule générale (IV) définie ci-dessus.

Les polymères HASE-H-RH particulièrement préférés répondent à la formule (V) ci-dessus, et sont tels que :

- q équivaut à 7 et n est égal à 6 atomes de carbone (appelé par la suite polymère HASE-H-RH4) ;.

- q équivaut à 7 et n est égal à 8 atomes de carbone (appelé par la suite polymère HASE-H-RH6) ;
- q équivaut à 9 et n est égal à 10 atomes de carbone (appelé par la suite polymère HASE-H-RH8).

De façon alternative, selon l'invention, le monomère d'acrylate d'éthyle (AE) du polymère HASE-H-RH de la formule (V) ci-dessus peut être remplacé par un monomère de méthacrylate de 2,2,2-trifluoroéthyle (MTFE) et correspond donc à un polymère HASE-F-RH qui répond à la formule générale (VI) suivante :

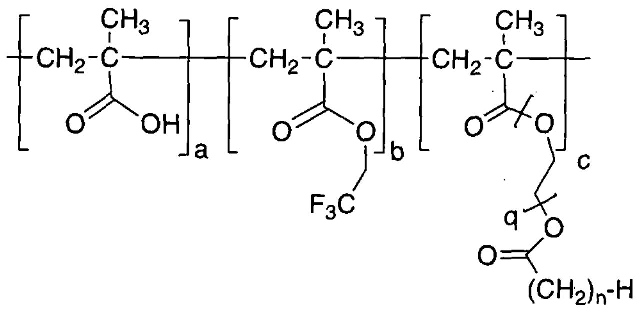

(VI)

dans laquelle :

- q désigne un nombre compris entre 5 et 10 ;
- n est compris entre 6 et 30 atomes de carbone ;
- a et c sont des nombres entiers, identiques ou différents, supérieurs ou égaux à 1, et b est supérieur ou égal à 0 ; préférentiellement, a est compris entre 1 et 10 000, b est compris entre 0 et 10 000 et c est compris entre 1 et 5 000.

De préférence encore, les polymères HASE-F-RH répondent à la formule générale (VI) ci-dessus et comprennent :

- de 30 à 85% molaire d'acide méthacrylique (AM) ;
- de 0 à 50% molaire de méthacrylate de 2,2,2-trifluoroéthyle (MTFE); et
- de 1 à 90% molaire d'un macromère qui est un ester de formule générale (IV).

Les polymères HASE-F-RH particulièrement préférés répondent à la formule (VI) ci-dessus, et sont tels que :

- q équivaut à 7 et n est égal à 6 atomes de carbone (appelé par la suite polymère HASE-F-RH4) ;
- q équivaut à 7 et n est égal à 8 atomes de carbone (appelé par la suite polymère HASE-F-RH6) ;

q équivaut à 9 et n est égal à 10 atomes de carbone (appelé par la suite polymère HASE-F-RH8).

De façon avantageuse, le Demandeur a, par ailleurs, pu mettre en évidence que la substitution de chaînons hydrocarbonés par des fluorocarbonés sur le macromère était possible dans un squelette HASE.

En modifiant ainsi son squelette avec des macromères fluorocarbonés, le polymère modificateur de rhéologie selon l'invention peut disperser les micro- ou nanoparticules tout en apportant l'hydrophobie et l'oléophobie nécessaire à la protection contre les agents chimiques.

La structure des tels polymères HASE à chaine fluorocarbonée (HASE-F), également utilisables selon l'invention, répond à la formule générale (VII) suivante :

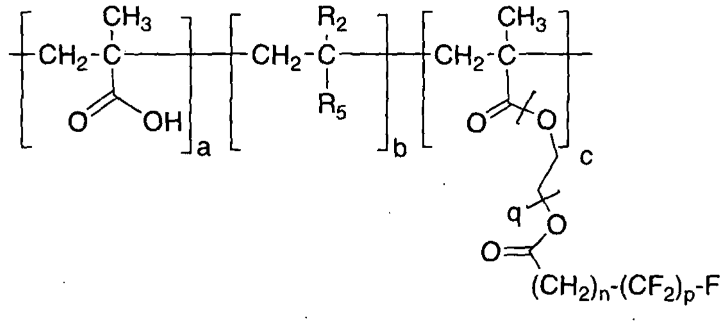

(VII)

dans laquelle :

- R2 représentent un atome d'hydrogène ou un groupement méthyle ;
- R5 représente $[Q]_{d1}$-$(CH_2)_n$-$(CX_2)_pX$ dans laquelle n est compris entre 1 et 30, d1 correspond à 0 ou à 1, et Q correspond à -C(O)-0 ou-C(O)-NH- ; et :

  - lorsque X est un atome d'hydrogène, alors p est égal à 0 ;
  - lorsque X est un atome de fluor, alors p est compris entre 1 et 12 ;

  ou R5 représente $[Q]_{d2}$-α, dans laquelle :

  - d2 correspond à 0 ou 1 ;
  - Q correspond à -C(O)-O ou -C(O)-NH- ; et
  - α correspond à -C(CH3)3 ; -$CH(CH_3)_2$ ; -$C(CH_3)_2$-$CH_2$-$C(CH_3)_3$ ; -CN ; -$CH_2CH_2$-N+$(CH_3)_2(CH_2CO_2^-)$ ; -$CH_2CH_2$-NH-$C(CH_3)_3$ ; -$CH_2CH_2$-$N(CH_3)_2$ ; pyrrolidinone ; caprolactame ;

- q désigne un nombre compris entre 1 et 150 ;
- n est un nombre entier compris entre 1 et 30 ;
- p est un nombre entier compris entre 1 et 12 ;
  et dans laquelle les indices a et c sont des nombres entiers, identiques ou différents, supérieurs ou égaux à 1, et b est supérieur ou égal à 0 ; préférentiellement, a est compris entre 1 et 10 000, b est compris entre 0 et 10 000 et c est compris entre 1 et 5 000.

De préférence encore, les polymères HASE-F répondent à la formule générale (VIII) suivante :

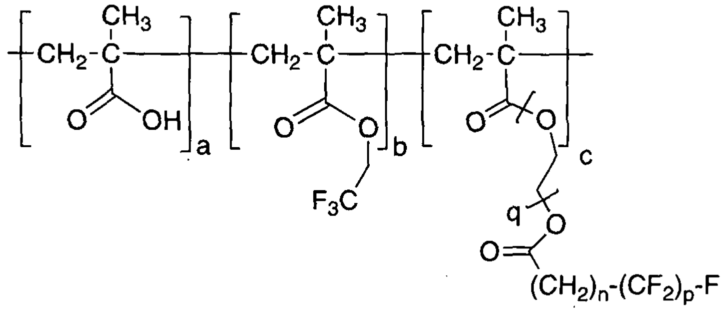

(VIII)

dans laquelle :

- q désigne un nombre compris entre 5 et 10 ;
- n est un nombre entier compris entre 1 et 30 ;
- p est un nombre entier compris entre 1 et 12 ;
- a et c sont des nombres entiers, identiques ou différents, supérieurs ou égaux à 1, et b est supérieur ou égal à 0 ;

préférentiellement, a est compris entre 1 et 10 000, b est compris entre 0 et 10 000 et c est compris entre 1 et 5 000.

Plus préférentiellement encore, les polymères HASE-F répondent à la formule générale (VIII) ci-dessus dans laquelle :

- q équivaut à 5, 7 ou 9 ;
- n équivaut à 2 ;
- p équivaut à 4, 6 ou 8 ; et
- a et c sont des nombres entiers, identiques ou différents, supérieurs ou égaux à 1, et b est supérieur ou égal à 0 ; préférentiellement, a est compris entre 1 et 10 000, b est compris entre 0 et 10 000 et c est compris entre 1 et 5 000. De préférence encore, les polymères HASE-F répondant à la formule générale (VIII) comprennent les monomères suivants :
- de 5 à 85% molaire d'acide méthacrylique (AM) ;
- de 1 à 70% molaire de méthacrylate de 2,2,2-trifluoroéthyle (MTFE) ; et
- de 1 à 50% molaire d'un macromère qui est un ester de formule générale (IX) :

$$- CH_2{=}CH(CH_3){-}C(O)(OCH_2CH_2)_qOC(O)(CH_2)_2{-}(CF_2)_pF \qquad (IX)$$

dans laquelle :

- q équivaut à 5, 7 ou 9 ; et
- p équivaut à 4, 6 ou 8.

Les polymères HASE-F particulièrement préférés répondent à la formule (VIII) ci-dessus, et sont tels que :

- q équivaut à 5 et p équivaut à 4 (appelé par la suite polymère HASE-F-RF4) ;
- q équivaut à 7 et p équivaut à 6 (appelé par la suite polymère HASE-F-RF6) ;
q équivaut à 7 et p équivaut à 6 (appelé par la suite polymère HASE-F-RF8) ;

Les polymères HASE décrits ci-dessus peuvent être synthétisés par le même procédé que pour les polymères ASE. Le détail d'une voie de synthèse des polymères HASE-H et HASE-F est détaillée à l'exemple 5.

De façon particulièrement avantageuse, les polymères modificateurs ou adaptateurs de rhéologie sont choisis parmi les polymères :

- ASE-H ;
- ASE-F ;
- HASE-H-RH4 ;
- HASE-H-RH6 ;
- HASE-H-RH8 ;
- HASE-F-RH4 ;
- HASE-F-RH6 ;
- HASE-F-RH8;
- HASE-F-RF4 ;
- HASE-F-RF6 ; ou
- HASE-F-RF8.

Plus préférentiellement encore, les polymères modificateurs ou adaptateurs de rhéologie sont choisis parmi les polymères :

- HASE-H-RH4 ;
- HASE-H-RH6 ;
- HASE-H-RH8 ;
- HASE-F-RH4 ;
- HASE-F-RH6 ;
- HASE-F-RH8 ;
- HASE-F-RF4 ;
- HASE-F-RF6 ; ou
- HASE-F-RF8.

Des expériences ont permis de démontrer que ces polymères présentent des propriétés rhéologiques (viscoélasticité, écoulement, thixotropie,...), d'oléophobie leur permettant d'être avantageusement utilisés, en tant que tels :

- dans la protection ou la décontamination cutanée (par exemple comme topique protecteur applicable en prévention d'un contact avec des agents chimiques toxiques),
- en médecine humaine, notamment en dermatologie, pour la prévention des irritations cutanées ou des allergies ;
- en cosmétique, notamment comme barrière épidermique de protection contre des agressions extérieures.

Exemple 1 : Synthèse des microparticules de silice de 292 nm et de 448 nm

La synthèse des microparticules de silice est réalisée suivant la méthode de Stöber. Cette méthode sol-gel consiste à hydrolyser et condenser le tetraéthylorthosilicate (TEOS) dans un mélange d'alcool, d'eau et d'ammoniaque. Le schéma de synthèse des microparticules de silice par le procédé Stöber est illustré à la figure 2a.
Le rapport eau/ammoniaque ($H_2O/NH_3$) va contrôler la taille des particules. L'eau va hydrolyser les particules tandis que l'ammoniaque va les stabiliser dans le milieu. Les rapports $H_2O/NH_3$ employés sont de 3,82 et de 2,57 pour la silice de 448 et 292 nm, respectivement. La première réaction consiste à hydrolyser un groupement -OEt en -OH, puis dans une deuxième réaction la condensation va avoir lieu pour former les microparticules de silice.
L'acide silicique est produit durant l'hydrolyse et quand sa concentration est au-dessus de sa solubilité dans l'éthanol, il croît (nucléation) de façon homogène et forme des particules de silice de taille submicronique. Cette méthode est utilisée pour la préparation de particules de silice pures.
L'incorporation de composés est possible par co-condensation du TEOS avec un silane (ex : 3-aminopropyltriethoxysilane (APTES) qui est une amine réactive contenant du silane) après l'étape d'hydrolyse. Le diamètre des particules peut être inférieur ou égal à 10 nm et peut aller jusqu'à environ 1 $\mu$m de diamètre. Elles sont généralement non uniformes dans la taille. Donc, des filtrations et séparations supplémentaires sont requises pour isoler les fractions de différentes tailles. Le contrôle du diamètre des particules peut s'effectuer par la température de réaction et le rapport eau/ammoniaque dans la réaction.
Le mode opératoire utilisé permet de réaliser des silices, de tailles contrôlées, fonctionnalisées par du GPTMS (3-glycidyloxypropyltrimethoxysilane).
Pour cet exemple, les silices ont été fonctionnalisées par un groupement amine introduit grâce au (3-aminopropyl)triméthoxysilane (AMS)), comme cela est illustré à la figure 2b.
Une fonctionnalisation grâce par exemple aux composés ((aminomethyl)triethoxysilane, (aminomethyl) triméthoxysilane), ((5-aminopentyl)tri-ethoxysilane ou (5-amino-pentyl)triméthoxysilane) était également envisageable.

Exemple 2 : fonctionnalisation de nanoparticules de silice :

Des nanoparticules commerciales de silice pyrogénées de 14 nm ont été fonctionnalisées par du (3-aminopropyl)triéthoxysilane dans du toluène anhydre avec un ratio silice/aminosilane de 10/1, comme cela est illustré à la figure 3.
L'aminosilane va réagir par réaction de condensation avec la silice fonctionnalisée par les groupements -OH.
Pour cette expérience, il est important de travailler en milieu anhydre afin que l'aminosilane ne soit pas hydrolysé, ce qui provoquerait la condensation entre les groupements Si-OH hydrolysés et des nanoparticules de tailles différentes seraient obtenues.
Après élimination du toluène par centrifugation et lavages à l'éthanol, les nanoparticules sont stockées en solution aqueuse afin d'éviter toute contamination par voie respiratoire.

Exemple 3 : synthèse de nanoparticules de titane :

Des nanoparticules de titane ont été obtenues par fonctionnalisation de nanoparticules commerciales de forme anatase (du fait de leur propriété photocatalytique). Les nanoparticules de dioxyde de titane peuvent par exemple être fonctionnalisées par des alkoxysilanes (formation de liaisons Ti-O-Si) ou par des composés phosphorés (formation de liaisons Ti-O-P).
Les composés phosphorés, comme l'acide phosphonique ou les phosphonates par exemples, sont connus pour former de fortes liaisons avec les oxydes métalliques et n'ont pas tendance à se condenser comme les alkoxysilanes. Pour l'obtention des nanoparticules de titane, le même procédé de fonctionnalisation que celui décrit précédemment à l'exemple 2 pour les nanoparticules de silice a été utilisé.

Exemple 4 : Synthèse des polymères ASE :

Comme cela est illustré à la figure 4, Les polymères ASE (ASE-H ou ASE-F) peuvent par exemple être constitués

d'acide méthacrylique (AM), d'acrylate d'éthyle (AE) et/ou de méthacrylate de 2,2,2-trifluoroéthyle (MTFE).

Ces polymères peuvent être synthétisés par polymérisation en émulsion avec du dodécyl sulfate de sodium (SDS) comme tensioactif et de l'acétone comme co-solvant. Deux polymères ont été synthétisés : ASE-H et ASE-F.

La synthèse d'un polymère avant le greffage des micro- ou nanoparticules permet de changer la chaine du polymère selon l'application ou les propriétés voulues pour le micro- ou nanocomposite.

L'introduction d'un monomère fluoré a une conséquence sur la stabilité de l'émulsion durant la polymérisation et une augmentation du phénomène de coagulation a été observée quand la longueur et la quantité de monomères fluorés augmente, induisant une diminution dans les rendements de conversion. Afin de stabiliser le système, 1% en masse d'acétone est utilisé comme co-solvant. Ce solvant est connu pour solubiliser les monomères fluorés dans les émulsions. Des rendements de 88 et 51% pour les polymères ASE-H et ASE-F ont été obtenus après purification par dialyse (4 000-6 000 Da).

D'après le tableau 1 ci-dessous, il apparait que la quantité d'acide méthacrylique (AM) augmente significativement lors de l'ajout du monomère fluoré. Ceci est probablement dû à la moins bonne intégration du MTFE durant le processus d'émulsion. Ceci peut être confirmé par la diminution du rendement du copolymère (51%).

Tableau 1 : Composition de polymères ASE-H et ASE-F déterminé par RMN$^1$H :

| Résonance | No. de proton (nominal) | Intensité | Comp (%mol.) | $\delta$H (ppm) | $\delta$F (ppm) |
|---|---|---|---|---|---|
| ASE-H | | | | | |
| AM | 1 | 1000 | 17,8 | 12,3 | - |
| AE | 2 | 4620,6 | 82,2 | 4,01 | - |
| MTFE | 0 | 0 | 0 | - | - |
| ASE-F | | | | | |
| AM | 1 | 1000 | 55,9 | 12,41 | - |
| AE | 2 | 179,8 | 10,0 | 3,96 | - |
| MTFE | 2 | 610,5. | 34,1 | - | 4,62 |

Dans le tableau 1 ci-dessus, AM concerne la résonance du proton de l'acide carboxylique, AE concerne la résonance assignée au proton méthylène de l'ester éthylique et MTFE concerne la résonance du proton méthylène de l'ester trifluoroéthylène. L'intensité de la résonance d'AM est normalisée à 1 000 dans chaque cas et les intensités sont normalisées en fonction du nombre de protons nominal apportés par la résonance.

Exemple 5 : Synthèse des polymères HASE :

Comme cela est illustré à la figure 5, les polymères HASE sont synthétisés par le même procédé que pour les polymères ASE. Ils sont constitués d'acide méthacrylique (AM), d'acrylate d'éthyle (AE) et/ou de méthacrylate de 2,2,2-trifluoroéthyle (MTFE) et par un macromère hydrocarboné ou fluoré.

Selon la figure 5, HASE-X-RXn sera le nom de référence du polymère synthétisé.

X correspond à la lettre H si le monomère d'acrylate d'éthyle est employé. X correspond à la lettre F (HASE-F-RXn) si c'est le monomère fluoré qui est employé.

RXn varie en fonction du type de chaine hydrocarbonée (HASE-X-RHn) ou fluorocarbonée (HASE-X-RFn) et n varie en fonction du nombre de carbone au sein du macromère.

Les macromères (MHn ou MFn) n'étant pas des produits commerciaux, ils ont été synthétisés par réaction d'estérification d'un monométhylméthacrylate polyéthylène glycol sur un composé acide carboxylique hydrocarboné ou fluorocarboné, comme cela est illustré à la figure 6.

La réaction a été catalysée par l'utilisation d'un agent de couplage, le N,N'-dicyclohexylcarbodiimide (DCC) en présence de N,N'-diméthylamino pyridine (DMAP).

Les composés synthétisés sont obtenus avec différents rendements et sont caractérisés par infrarouge et RMN. Les composés synthétisés sont listés dans le tableau 2 ci-dessous. Le nombre de monomères PEG, noté m dans la figure 6, est déterminé par l'intégration des signaux en RMN$^1$H dans les domaines de déplacements chimiques de 4,39-4,21 ppm (multiplet) et 3,76-3,64 ppm (multiplet). Le monométhylméthacrylate polyéthylène glycol de départ utilisé étant commercial et polydisperse, la colonne chromatographique a permis de séparer les différents monomères. Les rendements des monomères qui ont été utilisés dans la synthèse des polymères sont donnés dans le tableau 2.

Tableau 2 : Récapitulatif des chaines employées, de la référence des macromères et des rendements de réactions :

| Chaine | Référence du macromère (MXn) | Nombre m dans le macromère (détermin par RMN[1]H) | Rendement (%) |
|---|---|---|---|
| C4H9 | MH4 | 7 | 11 |
| C6H13 | MH6 | 7 | 23 |
| C8H17 | MH8 | 9 | 24 |
| C4F9 | MF4 | 5 | 27 |
| C6F13 | MF6 | 7 | 63 |
| C8F17 | MF8 | 7 | 55 |

Les polymères HASE ont ensuite été synthétisés selon le même procédé de polymérisation en émulsion que les polymères ASE. Ces polymères sont donc synthétisés par polymérisation en émulsion avec du dodécyl sulfate de sodium (SDS) comme tensioactif et de l'acétone comme co-solvant. Deux polymères ont été synthétisés : HASE-H et HASE-F. La composition du milieu de la polymérisation est donnée dans le tableau 3. Le macromère a été introduit à 0,5% en masse.

Tableau 3 : Composition en masse des polymères :

| Composé (%massue) | HASE-X-RXn |
|---|---|
| AM | 8,25 |
| AE ou MTFE | 10,9 |
| MXn | 0,5 |
| SDS | 1,42 |
| $K_2S_2O_8$ | 0,035 |
| Acétone | 1 |
| Eau | 77,9 |

A la fin de la polymérisation les polymères obtenus, dans des rendements allant de 52 à 81%, sont purifiés par dialyse (4 000-6 000 Da) puis caractérisés par IR, RMN[1]H et 19F. Les polymères synthétisés ainsi que la composition molaire calculée par RMN[1]H sont répertoriés dans le tableau 4 ci-dessous :

Tableau 4 : Récapitulatif des principaux polymères synthétisés et de leur composition molaire déterminée par RMN[1]H :

| Résonance | No. de protons (nominal) | Intensité | Comp (% mol.) |
|---|---|---|---|
| HASE-H-RH4 | | | |
| AM | 1 | 1 000 | 8,5 |
| AE | 2 | 1 071,9 | 9,1 |
| MH4 | 24 | 9 694,9 | 82,4 |
| Résonance | No. de protons | Intensité | Comp (% mol.) |
| HASE-H-RH6 | | | |
| AM | 1 | 1 000 | 64,7 |
| AE | 2 | 492 | 31,9 |
| MH6 | 24 | 52,9 | 3,4 |
| Résonance | No. de protons | Intensité | Comp (% mol.) |
| HASE-H-RH8 | | | |

(suite)

| Résonance | No. de protons (nominal) | Intensité | Comp (% mol.) |
|---|---|---|---|
| AM | 1 | 1 000 | 51,4 |
| AE | 2 | 921,4 | 47,3 |
| MH8 | 32 | 24,6 | 1,3 |
| Résonance | No. de protons | Intensité | Comp (% mol.) |
| HASE-F-RH4 | | | |
| AM | 1 | 1 000 | 78,9 |
| MTFE | 2 | 0 | 0 |
| MH4 | 24 | 266,7 | 21,1 |
| Résonance | No. de protons | Intensité | Comp (% mol.) |
| HASE-F-RH6 | | | |
| AM | 1 | 1 000 | 63,1 |
| MTFE | 2 | 482,5 | 30,5 |
| MH6 | 24 | 100,9 | 6,4 |
| Résonance | No. de protons | Intensité | Comp (% mol.) |
| HASE-F-RH8 | | | |
| AM | 1 | 1 000 | 69,0 |
| MTFE | 2 | 400,1 | 27,6 |
| MH8 | 32 | 49,4 | 3,4 |
| HASE-F-RF4 | | | |
| AM | 1 | 1 000 | 61,4 |
| MTFE | 2 | 41,6 | 2,6 |
| MF4 | 16 | 586,5 | 36,0 |
| HASE-F-RF6 | | | |
| AM | 1 | 1 000 | 16,0 |
| MTFE | 2 | 4 227,6 | 67,4 |
| MF6 | 24 | 1 041,5 | 16,6 |
| HASE-F-RF8 | | | |
| AM | 1 | 1 000 | 78,2 |
| MTFE | 2 | 236,8 | 18,5 |
| MF8 | 24 | 42,5 | 3,3 |

Dans le tableau 4 ci-dessus, AM concerne la résonance du proton de l'acide carboxylique, AE concerne la résonance assignée au proton méthylène de l'ester éthylique, MTFE concerne la résonance du proton méthylène de l'ester trifluoroéthylène et MHn concerne la résonance des protons des motifs éthoxylés excepté les quatre protons en alpha des deux esters. L'intensité de la résonance d'AM est normalisée à 1 000 dans chaque cas et les intensités sont normalisées en fonction du nombre de protons nominal apportés par la résonance.

Aussi, le tableau 4 ci-dessus suggère que le macromère MH4 s'incorpore au sein du polymère de manière plus importante que les autres.

La comparaison des différentes familles de polymères, HASE-H-RHn, HASE-F-RHn et HASE-F-RFn, permet d'observer que, lorsque la longueur de la chaine hydrocarbonée ou fluorocarbonée constituant le macromère augmente, le taux d'incorporation du macromère au sein du polymère diminue.

Pour ce qui concerne les monomères AM, AE et MTFE aucune variation linéaire du taux d'incorporation en fonction du type de macromères et de la longueur de la chaîne n'est observée. L'analyse par DSC a permis de déterminer la (les) température (s) de transition vitreuse des polymères et l'analyse par CES la masse des polymères ainsi que leur indice de polydispersité. Deux polymères HASE ont été caractérisés par CES et les résultats sont montrés dans le tableau 5 ci-dessous avec les rendements des polymérisations.

Tableau 5 :

| Polymère | Mw (g.mol-1) | Mw/Mn | Tg (°C) | Rendement (%) |
|---|---|---|---|---|
| HASE-H-RH8 | 1 162 632 | 4,48 | 54 | 64 |
| HASE-F-RF8 | 593 115 | 1,75 | 63 | 70 |

Les différences de masse molaire entre les polymères hydrocarbonés et fluorocarbonés peuvent s'expliquer par le fait que l'introduction de chaines fluorées entraine la déstabilisation du milieu lors du processus de polymérisation ce qui apporte la création de chaines polymériques plus courtes.

Exemple 6 : sélection du polymère préféré et modification dudit polymère en vue de l'optimiser :

a. *Sélection du polymère préféré :*

Les différents polymères synthétisés ont subi différentes analyses (rhéologiques, par diffusion dynamique de la lumière, ...) ainsi que des analyses par goniométrie.

Pour la réalisation des analyses par goniométrie, des solutions de polymères ont été étalées sur une surface modèle. 2,5 mg de chacun des polymères a été déposé sur une plaque de verre sur environ 2-3 $cm^2$ puis l'eau a été évaporée à l'air libre. Des gouttes d'huile d'olive ont été déposées sur les surfaces, ce qui a permis de déterminer les propriétés d'oléophilie/ oléophobie de chaque polymère.

Trois gouttes de liquide sonde de 3 $\mu$L ont été déposées afin de faire une moyenne. Les résultats de cette analyses sont repris à la figure 7 qui illustre les angles de contact à l'huile d'olive sur les polymères préalablement déposés sur une plaque de verre.

L'huile d'olive a été choisie car ce liquide possède une tension superficielle proche des agents toxiques utilisés pour l'étude ($\gamma$ = 32 mN/m à 20°C). L'eau n'a pas été utilisée car le polymère étant soluble dans l'eau, le film serait solubilisé et l'angle de contact obtenu serait peu significatif.

Comme illustré à la figure 7, excepté pour HASE-H-RH6, dès l'insertion d'un monomère ou d'une chaine fluorocarbonée, la valeur de l'angle de contact croît. Ceci se remarque surtout avec le polymère HASE-F-RF8 et son homologue hydrocarboné qui possède un angle de contact de 66° contre 25°, respectivement. Cette valeur est aussi dépendante de la longueur de la chaine fluorocarbonée comme il peut être constaté par une augmentation de 10 à 15° lorsque la chaine $C_8F_{17}$ est introduite comparé aux chaînes $C_4F_8$ et $C_6F_{13}$. Aussi, par rapport au verre seul, une très faible quantité de polymère permet d'augmenter l'angle de contact à l'huile d'olive. On peut ainsi déduire que le polymère HASE-F-RF8 est celui qui présente le meilleur angle de contact ($\theta$ = 66°) comparé aux autres polymères.

Compte tenu des différentes analyses réalisées, par regroupement des études rhéologiques et goniométrique, il a pu être mis en évidence que le polymère le plus visqueux à l'état initial est celui qui possède le meilleur angle de contact à l'huile d'olive, c'est-à-dire le polymère HASE-F-RF8. Pour une application comme topique protecteur, cette dernière particularité est très importante car le toxique n'adhérerait pas, ou très peu, à la surface du topique. Dès lors, le polymère HASE-F-RF8 a été choisi comme polymère préféré pour la suite des expériences. Le polymère HASE-H-RH8 a également été étudié afin de réaliser certaines analyses et pour une comparaison avec son homologue fluorocarboné HASE-F-RF8.

*b. Modification du polymère préféré :*

Le polymère HASE-F-RF8 avec un taux molaire de macromères de 3,3% ayant montré des propriétés de surface (en dépôt) et rhéologiques intéressantes, la quantité de macromères MF8 a été augmentée pour accroître la quantité de chaîne fluorée dans le milieu. Pour cela deux autres polymères ont été synthétisés : l'un à 13,5% et l'autre à 45,9% molaire de macromères. La voie de synthèse est la polymérisation en émulsion ; la quantité de monomères introduite est donnée dans le tableau 6 ci-dessous :

Tableau 6 : Composition en masse des monomères introduits lors de la polymérisation

| Composé (%masse) | HASE-F-RF8 (13,5%) | HASE-F-RF8 (45,9%) |
|---|---|---|
| AM | 6,75 | 8,25 |
| MTFE | 9,9 | 10,9 |
| MF8 | 3 | 0,5 |
| SDS | 1,42 | 1,42 |
| $K_2S_2O_8$ | 0,035 | 0,035 |
| Acétone | 1 | 1 |
| Eau | 77,895 | 77,895 |

La composition molaire des monomères constituants les polymères, déduite par RMN$^1$H, est donnée dans le tableau 7 ci-dessous :

Tableau 7 :

| Résonance | No. de protons (nominal) | Intensité | Comp (% mol.) |
|---|---|---|---|
| HASE-F-RF8 (13,5%) | | | |
| AM | 1 | 1 000 | 41,9 |
| MTFE | 2 | 1 061 | 44,5 |
| MF8 | 24 | 322,5 | 13,5 |
| Résonance | No. de protons | Intensité | Comp (% mol.) |
| HASE-F-RF8 (45,9%) | | | |
| AM | 1 | 1 000 | 33,8 |
| MTFE | 2 | 600,6 | 20,3 |
| MF8 | 24 | 1 361,8 | 45,9 |

Les analyses de goniométrie réalisées pour ces polymères HASE-F-RF8 (13,5% molaire de macromères) et HASE-F-RF8 (45,9% molaire de macromères), qui sont illustrées à la figure 8, montrent que l'augmentation du taux de macromères ne change pas l'angle de contact pour 2,5 mg de polymères déposé. En revanche, l'augmentation du taux de macromères de 3,3% à 13,5% molaire fait augmenter la viscosité sur toute la gamme de gradient de vitesse, comme cela est illustré à la figure 9 qui présente les courbes d'écoulements pour les polymères HASE-F-RF8 (3,3%), (13,5%) et (45,9%).

Cependant, le comportement du copolymère avec 45,9% molaire de macromères montre qu'il existe une valeur seuil à partir de laquelle la quantité de macromères n'améliore plus les propriétés rhéologiques.

Exemple 7 : Greffage des micro- ou nanoparticules de silice aux polymères :

Le greffage polymère/nanoparticules a été réalisé par une réaction d'amidation dans des conditions douces.

Le rapport EDC/NHS (en mole) utilisé a été de [1:0,07]. Le rapport polymère/micro- ou nanoparticules a été calculé par le nombre d'équivalents de fonctions acides portées par le polymère en fonction du nombre d'équivalents de fonctions amines portées par les micro- ou nanoparticules (pour 1 équivalent de fonctions acides contenus dans le polymère, 1 équivalent de fonctions amines a été introduit). A la fin de la réaction, le milieu réactionnel est purifié par dialyse puis centrifugé à 3 300 tr/min. Les couplages réalisés dans cette étude sont résumés dans le tableau 8 ci-dessous.

Tableau 8 : Récapitulatif des couplages polymères/silice (1 équivalent de silice) :

| Polymère (1 eq) | Si-448(1 eq) | Si-292(1 eq) | Si-22 (1 eq) |
|---|---|---|---|
| ASE-H | X | X | X |
| ASE-F | X | X | X |

(suite)

| Polymère (1 eq) | Si-448(1 eq) | Si-292(1 eq) | Si-22 (1 eq) |
|---|---|---|---|
| HASE-H-RH8 (1,3%) | - | - | X |
| HASE-F-RF8 (3,3%) | - | X | X |

Les polymères utilisés pour les greffages sont ASE-H, ASE-F, HASE-H-RH8 (1,3% molaire de macromères) et HASE-F-RF8 (3,3% molaire de macromères).

Les polymères ASE-H et ASE-F permettent de construire le modèle et de les comparer aux polymères HASE.

Le polymère HASE-F-RF8 (3,3% molaire de macromères) a été choisi pour ses meilleures propriétés rhéologiques et oléophobique et l'utilisation de son homologue carboné permet de comparer les propriétés de dispersion des nanoparticules, en fonction de la chaine hydrocarbonée ou fluorocarbonée, en milieu aqueux ou sur support. Les composés de greffage sont notés : ASE-H/Si-22 (1 eq) où ASE-H représente le polymère employé, Si-22 le type de nanoparticule ainsi que la taille et (1 eq) le nombre de fonctions amines par rapport au nombre de fonctions acides.

<u>Exemple 8 : Analyses des composés polymères/silice (1 équivalent) :</u>

L'analyse de la dispersion des micro- ou nanoparticules lors d'un dépôt sur un support permet de discriminer certains composés ou nanoparticules en fonction de l'homogénéité ou de l'inhomogénéité de la dispersion. Pour cela, des films des composés ont été réalisés en déposant 100 $\mu$L du surnageant en milieu neutre sur une plaque de verre puis l'eau a été évaporée dans un dessiccateur à pression atmosphérique. D'après les images de topographies, reprises à la figure 10, par rapport à la taille de la silice pour les composés ASE-H et ASE-F, plus celle-ci diminue, moins il y a d'agrégations.

Les dépôts des composés avec le polymère ASE-F montrent peu d'agrégations et les nanoparticules ou particules sont dispersées de manière homogène contrairement aux composés avec le polymère ASE-H qui présentent de nombreuses zones d'agrégations.

Le composé ASE-F/Si-22 est celui ayant le dépôt où les nanoparticules sont les mieux dispersées.

A la vue des résultats détaillés ci-dessus, il apparait que les micro- ou nanoparticules les plus préférées sont les nanoparticules de silice de 22 nm car :

- la quantité de polymère au sein du milieu doit avantageusement être élevée pour permettre une protection barrière, ce qui est moins le cas pour la silice de 448 nm où peu de polymères y est greffé ;
- afin d'obtenir une bonne efficacité protectrice de la part des nanoparticules, elles doivent être dispersées de manière homogène au sein du polymère. Ceci a été le mieux observé pour la silice de 22 nm.

Aussi, il est supposé que du fait de leur taille et de leur fonctionnalisation de surface, la quantité par $cm^2$ de nanoparticules de 22 nm sera plus importante que les particules de 448 et 292 nm. Ceci permettant par exemple une plus grande adsorption d'agent toxique par l'actif.

<u>Exemple 9 : Introduction des composés polymères/ nanoparticules dans des formulations cosmétique de type crème et gel</u>

*a. Formulation dans une crème :*

Les composés ont été introduits dans la crème BariedermTech™ commercialisée par la société Uriage™.

Les produits ont été concentrés puis introduits dans la crème à froid à 10% en masse et à pH = 9. Le pH choisi est relativement élevé par rapport au pH de la peau (pH $\approx$ 5) et il est important de ne pas l'agresser par une crème basique (risque de dommages sur la peau) mais les composés choisis ont les meilleures propriétés vers pH = 9. Les composés testés formulés dans ces crèmes ont été HASE-F-RF8 (3,3% molaire de macromères)/Si (0,3 eq) ; HASE-F-RF8 (13,5% molaire de macromères)/Si (0,3 eq) ; HASE-F-RF8 (3,3% molaire de macromères)/TiO2 (0,3 eq).

*b. Formulation dans un gel :*

Deux types de gels ont été synthétisés, un gel hydrophile et un gel hydrophobe.

Les formulations des gels sont détaillées dans le tableau 9 ci-dessous :

Tableau 9 :

| Gel hydrophile | | Gel hydrophobe | |
|---|---|---|---|
| Composé | Gel (%massique) | Composé | Gel (%massique |
| Eau | 67,80 | HASE-F-RF8 (13,5%) | 1 |
| Carbopol™ Ultrez 10 | 0,70 | Polymère/ nanoparticules | 2,4 |
| EtOH (96%) | 30,00 | Eau | 59,6 |
| TEA (99%) (50% en sol.) | 1,40 | EtOH (96%) | 25 |
| Polymère/ nanoparticules | 0,10 | TEA (99 %) (50% en sol.) | 2 |
| - | - | Fomblin™ | 10 |

Le Fomblin™ est un perfluoropolyéther (huile perfluorée) qui joue deux rôles. Le premier est d'augmenter l'hydrophobie et l'oléophobie du milieu et le second est de fluidifier le gel. Cette seconde propriété a été très utile car à fort taux de polymères, le gel présente un aspect élastique et ne peut être correctement étalé. La formule présentée dans le tableau 9 ci-dessus est celle qui permet d'obtenir un gel visqueux avec un bon étalement sur la peau et un pH compris entre 7,30 et 7,50.

Exemple 10 : Test d'efficacité du produit pur

Le but de cette expérience est de déterminer le potentiel protecteur d'un topique protecteur selon l'invention par une cinétique de pénétration sur des membranes semi-perméables, vis-à-vis de composés organophosphorés tel que le O-éthyl-O-(nitro-4-phenyl)phosphonate d'éthyle (paraoxon ou POX).

*Tests sur membrane synthétique :*

La membrane synthétique utilisée est une membrane en silicone (polydiméthylsiloxane) d'une épaisseur de 400 $\pm$ 100 $\mu$m commercialisée par la société Samco Silicone Products (Nuneaton, UK).
La membrane est découpée sous forme de disques d'environ 10 cm2. Le test de perméation a été réalisé en cellules de diffusion statiques de type Frantz en verre (cellules fabriquées par un verrier : Laboratoires VERRE LABO-MULA, Corbas, France). Le milieu récepteur est rempli de solution « Hank's Balance Salt Solution » (HBSS).

Tableau 11 : Répartition des membranes par rapport aux produits.

| Produits | Nombre de membranes |
|---|---|
| Témoin (non traitées) | 3 |
| HASE-F-RF8 (13,5%) | 4 |
| HASE-F-RF8 (13,5%)/Si | 4 |

*Traitement :*

Le topique protecteur selon l'invention est appliquée à raison de 5 mg/cm$^2$, répartie à l'aide d'une spatule en silicone souple.
La membrane est ensuite déposée sur le compartiment récepteur. Un joint en Teflon™ est ajouté sur la membrane puis la cellule est fermée par le compartiment donneur ce qui laisse une surface d'exposition de la membrane de 1,13 cm$^2$. Lorsque la cellule est fermée, elle est mise sur un porte cellule disposée dans un bain marie, puis une vis est ajoutée afin de permettre un bon contact entre la membrane et le milieu récepteur. Enfin la membrane et le gel sont équilibrés à la température durant 20 min. Le bain-marie est réglé à 38°C afin d'obtenir une température de 32°C $\pm$ 1°C à la surface des membranes.
Le toxique est déposé au centre de la membrane sous forme de goutte. La quantité de paraoxon (POX)déposée est de 5 mg/cm$^2$ soit de 4,9$\mu$L.
Des prélèvements de 400 $\mu$L sont effectués dans le coude de prélèvement toutes les 1h30 min pour le POX. Une fois les prélèvements réalisés, un volume identique d'HBSS est ajouté afin de garder constante la quantité de milieu

récepteur. Tous les prélèvements sont conservés au congélateur à - 20°C.

*Dosage de la quantité de POX dans les milieux récepteurs :*

La méthode employée est un dosage enzymatique du toxique. Cette méthode de dosage est une méthode indirecte qui permet de doser l'activité d'une enzyme en présence de paraoxon (POX). La concentration de paraoxon dans le prélèvement est déterminée d'après une gamme de concentration bien définie et est proportionnelle au degré d'inhibition de l'enzyme (la butyrylcholinestérase) qui a été ajoutée en quantité connue dans chaque prélèvement.

Les résultats sont présentés dans la figure 11 où apparaît l'évaluation du produit vis-à-vis de la pénétration transmembranaire du paraoxon. %QO représente le pourcentage de dose absorbée de paraoxon.

On constate que la pénétration transmembranaire du paraoxon est ralentie et diminuée légèrement lorsque la membrane est prétraitée avec le polymère HASE-F-RF8 (13,5% molaire de macromères) mais fortement avec le composé HASE-F-RF8 (13,5% molaire de macromères)/Si.

Pour comparer les différents produits entre eux, un test statistique de Kruskal-Wallis non-paramétrique qui compare les variances de plus de deux échantillons indépendants à t = 6h, suivi d'un test de Dunn qui permet la comparaison d'un échantillon avec un autre, ont permis de tirer les conclusions sur l'effet protecteur des composés. Le polymère n'a pas un effet protecteur significatif, par contre le composé polymère/silice a un effet protecteur significatif vis-à-vis de la membrane.

Ceci met en avant l'effet protecteur du composé polymère fluorocarboné/ micro- ou nanoparticules synthétisé sur la pénétration transmembranaire du POX.

<u>Exemple 11 : Ecotoxicité des composés selon l'invention :</u>

Le but a donc été de déterminer l'impact écotoxicologique des nanoparticules de silice fonctionnalisées (de 448, 292 et 22 nm, notées respectivement dans cette étude : 400, 300 et 22 nm) ou pure (14 nm) ainsi que du composé de greffage HASE-H-RH8 (1,3% molaire de macromères)/Si (0,3eq).

Quatre tests d'organismes ont été choisis pour leur sensibilité, leur représentativité de l'écosystème et le niveau trophique (position d'un organisme qu'il occupe au sein de la chaîne alimentaire) : *Chlorella vulgaris* (SAPS) et *Phaeodactylum tricornutum algae* (eau douce et eau de mer), *Daphnia Magna micro-crustacean* (écosystème aquatique), et des graines de *Linum usitatissimum* (écosystème terrestre).

Ces tests ont été réalisés dans des conditions *in vitro* afin d'étudier les changements des organismes testés après incubation en contact avec les produits chimiques et pour déterminer le sort des produits chimiques après le contact avec les organismes biologiques. Les résultats sont schématisés aux figures 12, 13 et 14

La figure 12 représente les mesures de la $EC_{50}$ pour *Daphnia magna* de suspensions d'eau.

La figure 13 représente les mesures de la $EC_{50}$ de *Phaeodactylum* tricornutum et *Chlorella vulgaris (SAPS)* de suspensions d'eau des nanoparticules de silice pures ($SiO_2$-14) et fonctionnalisées ($SiO_2$-22; -300 et -400).

La figure 14 représente les mesures de la $EC_{50}$ de *Daphnia magna* et *Phaeodactylum tricornutum* de suspensions d'eau du polymère HASE-H-RH8 (1,3% molaire de macromères) (polymère HASE) et du composé HASE-H-RH8 (1,3% molaire de macromères)/Si (0,3 eq) (composé greffé selon l'invention).

Ces différents tests de toxicité avec *Daphnia magna, Phaeodactylum tricornutum* et *Chlorella vulgaris (SAPS)* mettent en évidence, que la toxicité des nanoparticules diminue lorsque la taille des composés augmente. Ces tests montrent également que la fonctionnalisation des particules de silice de 14 nm par le composé amine-silane (silice de 22 nm) entraine une diminution de la toxicité.

De même, le polymère seul et le composé greffé selon l'invention ont montré une toxicité plus faible que les nanoparticules de 22 nm.

Le quatrième test, avec *Linum usitatissimum*, est un test de germination de graines qui permet par comparaison de la taille de germination (méthode de mesure) par rapport au témoin (un toxique de référence : le K2Cr207) de déduire la toxicité ou non du composé étudié. Les tests réalisés n'ont pas montré de différence de toxicité entre le polymère seul (HASE-H-RH8 (1,3% molaire de macromères) (polymère HASE)) et le composé greffé (HASE-H-RH8 (1,3% molaire de macromères)/Si (0,3 eq)) selon l'invention.

Toutefois, une différence notable entre ces deux composés (polymère seul et composé greffé) et le témoin a pu être observée. La comparaison des nanoparticules de silice de 22 nm avec le composé greffé a mis en évidence que le composé greffé selon l'invention était significativement moins toxique. Pour ces expériences le composé greffé est ajouté à différentes concentrations dans les graines (0,01 mg/L, 0,1 mg/L, 1 mg/L, 10 mg/L et 100 mg/L). Ceci permettant de déterminer la concentration à partir de laquelle le composé est toxique.

Compte tenu des expériences d'écotoxicité décrites ci-dessus, il apparait que le polymère HASE-H-RH8 (1,3% molaire de macromères) est le moins toxique des composés testés.

En outre, le greffage de nanoparticules avec ce polymère permet de diminuer d'avantage la toxicité, en comparaison avec aux nanoparticules fonctionnalisées de 22 nm et non fonctionnalisées de 14 nm.

Ces résultats démontrent que les composés selon l'invention présentent une toxicité réduite en comparaison aux autres produits testés.

Exemple 12 : Exemple de composés selon l'invention :

La figure 15 représente un schéma général de composés selon l'invention permettant d'obtenir une protection nouvelle technologie. Dans le schéma de cette figure 15 :

- les groupements $R_1$ correspondent par exemple à des atomes d'hydrogène ou à des groupement alkyles ayant de 1 à 4 atomes de carbone ;
- les groupements $R_2$ correspondent à des groupements $C_nX_{2n+1}$ où X représente un atome d'hydrogène ou de fluor et n est compris entre 1 et 9 ;
- les groupements $R_3$ correspondent à une chaine hydrocarbonée ou fluorocarbonnée $C_nX_{2n+1}$ dans laquelle X représente un atome d'hydrogène ou de fluor et n est compris entre 4 et 8 ;
- p est compris entre 5 et 10 ;
- a, a', a" , a'" b, b', b", c, c' et c", identiques ou différents, sont des nombres entiers, supérieurs à 1 ; et
- les billes ou cercles représentent des micro- ou nanoparticules d'oxyde d'aluminium ($Al_2O_3$), d'oxyde de cuivre (CuO), d'oxyde de fer ($Fe_3O_4$ ou $\gamma$-$Fe_2O_3$), d'oxyde de magnésium (MgO), de silice ($SiO_2$), de dioxyde de titane ($TiO_2$) ou d'oxyde de zinc (ZnO), présentant un diamètre nominal compris entre 1 et 1500 nm.

Exemple 13 : Etude de la dispersion des micro- ou nanoparticules au sein du milieu et optimisation des composés selon l'invention :

La dispersion des nanoparticules étant obtenue à priori par la quantité de fonctions acides carboxyliques présentes dans le milieu, le Demandeur s'est intéressé à diminuer le nombre d'équivalent de silice. La quantité de nanoparticules greffées sera moins importante et le nombre de fonctions acides carboxyliques libres sera plus élevé afin de contrôler la dispersion des nanoparticules. L'étude sur la diminution du nombre d'équivalents de silice s'est portée sur le polymère HASE-F-RF8 (3,3% molaire de macromères) couplé avec de la silice de 22 nm à 0,8 ; 0,3 ; 0,1 ; 0,05 et 0,01 équivalent de silice (nombre d'équivalent de fonctions amines portées par la silice par rapport au nombre d'équivalent de fonctions acides portées par le polymère) et la quantité d'EDC a été divisée par deux par rapport au nombre d'équivalent de silice en vue de limiter le taux de greffage de fonctions acides mais aussi afin d'avoir des fonctions acides carboxyliques libres en solution permettant la dispersion des nanoparticules en milieu basique par des interactions ioniques. Le rapport EDC/NHS utilisé est de [1:0,07].

Le demandeur a ensuite déterminé le nombre d'équivalent pour lequel la silice est le mieux dispersée et a observé si les propriétés de mouillabilité étaient différentes. Des études par Microscopie à Force Atomique (AFM) et par goniométrie ont donc été réalisées.

*a. Topographies des films en milieu neutre des composés*

*HASE-F-RF8 (3,3%)/Si-22 à 1 ; 0,3 ; 0,1 ; 0,05 et 0,01 équivalent :*

D'après les images de topographie illustrées à la figure 16, on remarque que le dépôt à 1 équivalent contient beaucoup d'agrégats à la différence des quatre autres dépôts. Concernant les dépôts des formulations à 0,1 et 0,05 équivalent, la répartition des nanoparticules au sein de ces derniers est faible. Pour les dépôts des formulations à 0,3 et 0,01 équivalent, on observe une bonne dispersion des nanoparticules en plus d'une faible agrégation. Ces formulations seraient les meilleures équivalents pour la dispersion des nanoparticules.

*b. Caractérisation par goniométrie :*

Comme cela est illustré à la figure 17, ces cinq dépôts ont aussi été caractérisés par goniométrie (en utilisant l'eau (pour hydrophilie/hydrophobie) et l'huile d'olive (pour l'oléophilie/l'oléophobie et possédant une tension superficielle proche de celle de l'agent chimique VX).

D'après la figure 17, on peut conclure que lorsque l'on diminue le nombre d'équivalent de silice de 1 à 0,05 équivalent, l'angle de contact avec l'eau augmente de 30° jusqu'à environ 70°, puis diminue à 60° pour 0,01 équivalent.

Toutefois, ces valeurs ne sont pas constantes et comme le montre l'histogramme, à 0,1 et 0,05 équivalent, après une minute l'angle de contact diminue vers 30° (valeur de l'angle de contact pour 1 équivalent). Ceci s'expliquant par la solubilisation du polymère en milieu aqueux. Par contre, pour le composé à 0,3 équivalent la valeur de l'angle

(environ 50°) reste constante même après une minute. L'analyse avec l'huile d'olive permet de montrer que la diminution du nombre d'équivalent n'a pas d'influence sur la valeur de l'angle de contact, dont celle-ci se situe vers 25° pour tous les dépôts. Les angles de contact avec l'huile d'olive restent constants après une minute.

En comparant les images de topographies obtenues par AFM et les valeurs d'angles de contact obtenues par goniométrie, le couplage avec un nombre d'équivalent de nanoparticules de 0,3 équivalent a montré les meilleurs résultats de dispersion et d'oléophobie.

Exemple 14 : Exemple de composés selon l'invention :

Le Demandeur a essayé dans cet exemple d'optimiser l'efficacité du produit HASE-F-RF8 (13,5%) greffé avec des nanoparticules de silice (HASE-F-RF8/Si) dans un topique protecteur pour une utilisation dans la protection ou la décontamination cutanée vis-à-vis des agents du risque biologique ou chimique.

De nombreux travaux ont, par le passé, réalisés des surfaces hautement oléophobes avec un angle de contact compris entre 90° et 140° avec des liquides de faibles tensions de surface ($\gamma$), comme par exemple l'hexadécane ($\gamma$ = 27,3 mN/m) ou l'huile d'olive ($\gamma$ = 32 mN/m), grâce à des composés perfluorés et une structuration de la surface. Le composé HASE-F-RF8/Si ayant aussi montré une bonne dispersion des nanoparticules de silice en solution et lors de dépôts sur des surfaces, il serait intéressant d'accroitre cette dispersion et l'efficacité de protection.

HASE-F-RF8/Si possédant encore des fonctions amines libres situées sur les nanoparticules, il est possible de greffer à nouveau une molécule. Pour cela, l'acide 4,4,5,5,6,6,7,7,7-nonafluoroheptanoïque (RF4), l'acide 4,4,5,5,6,6,7,7,8,8,9,9,9-tridecafluorononanoïque (RF6) et l'acide 4,4,5,5,6,6,7, 7,8,8,9,9,10,10,11,11,11-heptadecafluoroundécanoïque (RF8) ont été accrochés de manière covalente au composé HASE-F-RF8/Si, en milieu aqueux et en présence d'un agent de couplage, afin d'augmenter l'oléophobie des dépôts. Comme cela est illustré à la figure 18, les fonctions amines libres portées par les nanoparticules de silice réagissent avec les fonctions acides carboxyliques des composés fluorés RF4, RF6 et RF8 pour obtenir les composés HASE-F-RF8/Si/RFn avec n = 4, 6 et 8.

Ce nouveau composé hybride organique/inorganique HASE-F-RF8/Si/RF8 en suspension dans l'eau a été caractérisé par Diffusion Dynamique de la Lumière (DDL). La mouillabilité des composés HASE-F-RF8/Si et HASE-F-RF8/Si/RFn lors de dépôts par étalement ou par spray a également été déterminée et l'état de surface analysé par Microscopie Electronique à Balayage (MEB) et Microscopie à Force Atomique (AFM).

*a. Greffage des composés fluorés au composé HASE-F-RF8/Si :*

Un mélange d'EDC/NHS (en mole) de [1:2] a été ajouté à une solution d'acide 4,4,5,5,6,6,7,7,8,8,9,9,10,10,11,11,11-heptadecafluoroundécanoïque (RF8) ou d'acide 4,4,5,5,6,6,7,7,8,8,9,9,9-tridecafluorononanoïque (RF6) ou d'acide 4,4,5,5,6,6,7,7,7-nonafluoroheptanoïque (RF4) dans de l'eau à un pH compris entre 4 et 5. Le milieu réactionnel a été agité durant 2 heures à 28°C. Ensuite une solution à pH = 8,5 du composé HASE-F-RF8/Si (1 mole) a été ajoutée et la réaction s'est poursuivie durant 5 jours à 28°C pour obtenir les composés HASE-F-RF8/Si/RFn avec n = 4, 6 et 8. Les composés obtenus ont été purifiés par dialyse (4 000 - 6 000 Da) puis analysés.

*b. Analyses des composés HASE-F-RF8/Si/RFn :*

La réaction entre les fonctions amine libres portées par les nanoparticules et les fonctions acides carboxyliques des composés RFn avec n = 4, 6 et 8 permettent d'obtenir les composés HASE-F-RF8/Si/RFn. Les composés RFn sont préalablement activés avec l'EDC et le NHS dans l'eau pour permettre la réaction intramoléculaire entre les groupements acides carboxyliques et les fonctions amines du composé HASE-F-RF8/Si. Le greffage induit la création d'une fonction amide (-CO-NH-). La figure 19 représente le spectre infrarouge des composés HASE-F-RF8/Si et HASE-F-RF8/Si/RFn. Il n'y aucune différence entre ces graphiques car les fonctions dans ces composés sont les mêmes. En milieu neutre, les fonctions acides carboxyliques s'ionisent partiellement et la bande du carbonyle est déplacée de 1708 $cm^{-1}$ à 1570 $cm^{-1}$ alors que la bande du carbonyle de l'amide reste à 1637 $cm^{-1}$ et la bande ester à 1741 $cm^{-1}$. De plus, la bande C-F des composés fluorés, la bande C-o de l'ester et la bande Si-O-Si sont à 1285 $cm^{-1}$ 1245 $cm^{-1}$ et 1107 $cm^{-1}$ respectivement. L'analyse infrarouge a montré la présence de fonctions acides carboxyliques libres qui sont nécessaires au gonflement du composé dans l'eau et à la dispersion des nanoparticules en solution.

Pour les composés HASE-F-RF8/Si et HASE-F-RF8/Si/RF8, la quantité de fluor et de carbone a été estimée par analyse élémentaire.

Les résultats de cette analyse élémentaire de HASE-F-RF8/Si et de HASE-F-RF8/Si/RF8 sont repris dans le tableau 12 ci-dessous :

Tableau 12 :

| Composé | C(%) | N(%) | F(%) |
|---|---|---|---|
| HASE-F-RF8 /Si | 29,65 | 0,69 | 9,20 |
| HASE-F-RF8/Si/RF8 | 26,36 | 1,03 | 12,84 |

La valeur du pourcentage de fluor augmente pour HASE-F-RF8/Si/RF8 (F : 12,84%) contrairement à HASE-F-RF8/Si (F : 9,20%). Ceci met en évidence le greffage entre les groupements acide carboxylique du composé RF8 et les fonctions amine du composé HASE-F-RF8/Si.

*c. Dépôts des composés :*

Les composés HASE-F-RF8/Si et HASE-F-RF8/Si/RFn avec n = 4, 6 et 8 ont été déposés sur des plaques de verre de 5 $cm^{-1}$ par étalement et par spray. L'étalement a été réalisé en étalant les composés sur la plaque de verre avec une spatule. Le revêtement par spray a été réalisé avec un pistolet à air comprimé possédant une canule de 0,3/0,5 mm de diamètre et à une distance de l'échantillon de 10 cm. Des solutions de 2%, 10,7% et 7,5% ont été utilisées pour les composés HASE-F-RF8/Si/RF4, HASE-F-RF8/Si/RF6 et HASE-F-RF8/Si/RF8, respectivement.

*d. Analyse de la dispersion des nanoparticules en solution.*

La dispersion des nanoparticules dans l'eau dépend du pH de la suspension. Afin de déterminer le pH de la suspension pour lequel la meilleure stabilisation est obtenue, le potentiel zêta des composés HASE-F-RF8, HASE-F-RF8/Si et HASE-F-RF8/S1/RF8 a été mesuré dans le domaine de pH de 3 à 9. Ces mesures sont reprises à la figure 20. Pour HASE-F-RF8/Si au pH d'environ 4, le potentiel zêta est proche de -20 mV dû à la protonation des groupes amine en ammoniums contrairement à HASE-F-RF8/Si/RF8 qui atteint un potentiel zêta de -30 mV.

Cette différence met en avant la diminution des groupements amine dans le composé HASE-F-RF8/Si/RF8 et justifie le greffage du composé RF8 sur les nanoparticules de silice. Quand le pH augmente, une forte diminution du potentiel zêta est mesurée et correspond à l'ionisation des groupements acides carboxyliques portés par le copolymère. En même temps, les groupes ammoniums commencent à être neutralisés jusqu'à atteindre un potentiel zêta proche de -70 mV. Le maximum de stabilisation est obtenu à pH = 7 aussi bien pour HASE-F-RF8/Si/RF8 que pour HASE-F-RF8/Si. Une étude de stabilité à pH = 7 de HASE-F-RF8/Si/RF8 a été réalisée durant 21h. La concentration de la solution utilisée est de 2% en masse.

Les résultats montrent que :

- le milieu est stable pendant 4 h puis une décantation du milieu est observée à partir de 4h ;
- après 21h, si le composé est agité il est re-dispersé puis ;
- il est à nouveau observé une décantation après 4h.

*e. Etude de la mouillabilité à l'huile d'olive des composés HASE-F-RF8/Si et HASE-F-RF8/Si/RFn.*

Les composés HASE-F-RF8/Si et HASE-F-RF8/Si/RFn sont déposés par étalement sur des plaques en verre, à un pH proche de 7, en faisant varier la quantité de 0 à 60 mg. De plus, 15 mg de HASE-F-RF8/Si/RF8 est déposé par la méthode de spray puis comparé à la surface obtenue par la méthode d'étalement.

Dans le cas de HASE-F-RF8/Si/RF4 et HASE-F-RF8/Si/RF6 c'est 1 mg et 1,5 mg respectivement qui ont été déposés par spray. L'oléophobicité des surfaces des composés hybrides organiques/inorganiques a été évaluée en mesurant l'angle de contact statique de gouttes d'huile d'olive de 4,9 $\mu$L (yL = 32 mN/m). Dans les cas de HASE-F-RF8/Si et HASE-F-RF8/Si/RF8 la variation de l'angle de contact en fonction de la quantité de composé (mg) est donnée à la figure 21. Par la méthode d'étalement, des revêtements oléophobes sont obtenus rapidement dès 5 mg de composé déposé avec un angle de contact de 90° pour les deux composés. Lorsque la quantité déposée sur la plaque de verre est augmentée, l'angle de contact pour HASE-F-RF8/Si reste constant mais l'écart type augmente. Cet écart peut être expliqué par la fracture du film et sa non-adhésion sur le support en verre. L'huile s'étale alors sur la plaque en verre ainsi que sur le revêtement HASE-F-RF8/Si. Pour HASE-F-RF8/Si/RF8, l'angle de contact augmente continuellement en fonction de la quantité déposée pour atteindre une surface hautement oléophobe avec un angle de contact de 120°. Le greffage de la chaine fluorocarbonée sur HASE-F-RF8/Si augmente l'angle de contact de 40° et permet de passer d'un revêtement oléophobe (HASE-F-RF8/Si) à un revêtement hautement oléophobe (HASE-F-RF8/Si/RF8), comme cela est illustré aux figures 22a et 22b. De plus, l'adhésion du composé HASE-F-RF8/Si est améliorée.

Pour obtenir une surface superoléophobe, il est nécessaire de combiner des composés oléophobes et une

micro- et nano-structuration de la surface. Probablement, quand le composé fluorocarboné RF8 a été greffé, la structuration de la surface a changé.

Les figures 23a à 23f montrent les analyses par microscopie électronique à balayage (MEB) des composés HASE-F-RF8/Si et HASE-F-RF8/Si/RF8 par les méthodes d'étalement et de spray.

Les figures 23a et 23b correspondent au dépôt de 52 mg de HASE-F-RF8/Si avec une méthode par étalement.

Les figures 23c et 23d correspondent au dépôt de 47 mg de HASE-F-RF8/Si/RF8 avec une méthode par étalement.

Les figures 23e et 23f correspondent au dépôt de 15 mg de HASE-F-RF8/Si/RF8 avec une méthode par spray.

Par la méthode d'étalement, HASE-F-RF8/Si ne possède pas de structuration de surface et les nanoparticules peuvent être observées à la surface du revêtement. Par contre, le composé HASE-F-RF8/Si/RF8 présente une micro- et nano-structuration due à l'organisation en forme d'aiguille et par la présence des nanoparticules mais aussi une oléophobie qui est apportée par les chaines fluorocarbonées. Tous ces résultats sont les mêmes pour le revêtement par spray du composé HASE-F-RF8/Si/RF8.

Comme cela découle des figures 24a et 24b, la méthode de dépôt par spray n'impacte pas la morphologie de la surface ainsi que l'angle de contact qui sont identiques par les deux méthodes: $\theta$ = 106° $\pm$ 5 pour la méthode d'étalement (figure 24a)et $\theta$ = 111° $\pm$ 4 pour la méthode par spray (figure 24b).

La structuration en aiguilles du composé a aussi été mise en évidence par Microscopie à Force Atomique (AFM) pour les deux méthodes, comme cela est illustré aux figures 25a et 25b.

Pour le composé HASE-F-RF8/Si/RF6 la mesure de l'angle de contact en fonction de la quantité de composé étalé ne montre pas une variation aussi importante que pour le composé HASE-F-RF8/Si/RF8. Entre 2 mg et 60 mg de composé déposé par étalement, l'angle de contact mesuré est de 80,6° $\pm$ 3,1 à 88,6° $\pm$ 1,1, respectivement. Le dépôt du composé n'est pas homogène. Ceci est validé par l'image obtenue par Microscopie à Force Atomique (AFM), du dépôt qui présente un trou et de plus aucune structuration particulière n'est observée ce qui prouve la faible variation de l'angle de contact (cf. les figures 26a et 26b). Par spray, l'angle de contact obtenu est de 84,6° $\pm$ 1,9 (2 mg déposé) ce qui est similaire avec la valeur obtenue par la méthode d'étalement.

Pour le composé HASE-F-RF8/Si/RF4 la mesure de l'angle de contact en fonction de la quantité de composé étalée ne montre pas une variation aussi importante que le composé HASE-F-RF8/Si/RF8 mais surtout la valeur de l'angle est plus faible que pour HASE-F-RF8/Si/RF6 et HASE-F-RF8/Si/RF8. Entre 1,2 mg et 49 mg de composé déposé par étalement, l'angle de contact mesuré est de 80,6° $\pm$ 1,8 à 76,6° $\pm$ 4,3, respectivement. Dans ce cas-là, l'angle de contact diminue lorsque la quantité déposée augmente. Il a été remarqué que l'adhésion au substrat n'était pas optimale lorsque la quantité déposée augmente mais aussi que le dépôt possédait des craquelures. Ceci explique cette diminution de l'angle de contact ainsi que l'augmentation de l'écart type. En prenant compte l'augmentation de l'écart type la valeur de l'angle reste vers les 80° quelle que soit la quantité déposée. De même que pour le composé HASE-F-RF8/Si/RF6, l'analyse du dépôt par Microscopie à Force Atomique (AFM) (cf. figures 26a et 26b) ne montre aucune structuration particulière.

La figure 26a représente une image AFM des dépôts par étalement du composé HASE-F-RF8/Si/RF4 pour 49 mg. La figure 26b représente quant à elle une image AFM des dépôts par étalement du composé HASE-F-RF8/Si/RF6 pour 60 mg. Par spray, l'angle de contact obtenu est de 88,1° $\pm$ 2,6 (1 mg déposé) ce qui est légèrement plus élevé que la valeur obtenue par la méthode d'étalement.

## Revendications

1. Composé formé par des micro- ou nanoparticules fonctionnalisées amines associées de façon covalente à des polymères modificateurs de rhéologie, **caractérisé en ce que** :

   - les micro- ou nanoparticules fonctionnalisées sont des micro- ou nanoparticules fonctionnalisées d'oxyde inorganique ou métallique notamment d'oxyde d'aluminium ($Al_2O_3$), d'oxyde de cuivre (CuO), d'oxyde de fer ($Fe_3O_4$ ou $\gamma$-$Fe_2O_3$), d'oxyde de magnésium (MgO), de silice ($SiO_2$), de dioxyde de titane ($TiO_2$ ou d'oxyde de zinc (ZnO), présentant un diamètre nominal compris entre 1 et 1500 nm ;
   - les polymères modificateurs ou adaptateurs de rhéologie sont choisis parmi des polymères non-associatifs ou des polymères associatifs.

2. Composé selon la revendication 1, **caractérisé en ce que** les micro- ou nanoparticules fonctionnalisées amines sont des micro- ou nanoparticules fonctionnalisées amines de silice ($SiO_2$) ou de dioxyde de titane ($TiO_2$).

3. Composé selon l'une des revendications 1 ou 2, **caractérisé en ce que** les micro- ou nanoparticules fonctionnalisées amine présentent un taux de fonctions amines compris entre 0,1 et 10 meq/g de micro- ou nanoparticules.

**4.** Composé selon l'une des revendications 2 ou 3, **caractérisé en ce que** les micro- ou nanoparticules fonctionnalisées amines de silice ($SiO_2$) ou de de dioxyde de titane ($TiO_2$) sont des nanoparticules dont le diamètre nominal est respectivement compris entre 5 et 500 nm et entre 1 et 300 nm.

**5.** Composé selon l'une des revendications précédentes, **caractérisée en ce que** les polymères modificateurs ou adaptateurs de rhéologie sont choisis :

- parmi des polymères non-associatifs ASE-H de formule générale (I) suivante :

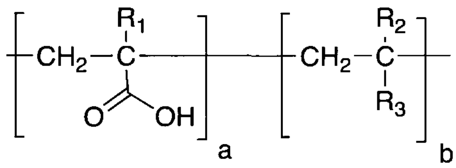

(I)

dans laquelle :

- R1 et R2 représentent un atome d'hydrogène ou un groupement méthyle $-CH_3$ ;
- R3 représente $[Q]_{d1}-(CH_2)_n-H$ dans laquelle n est compris entre 1 et 30, d1 correspond à 0 ou à 1, et Q correspond à -C(O)-O ou -C(O)-NH- ;

ou

R3 représente $[Q]_{d2}-\alpha$, dans laquelle :

• d2 correspond à 0 ou 1 ;
• Q correspond à -C(O)-O ou -C(O)-NH- ; et
• a correspond à '$-C(CH_3)_3$ ; $-CH(CH_3)_2$ ; $-C(CH_3)_2-CH_2-C(CH_3)_3$ ; -CN ; $-CH_2CH_2-N+(CH_3)_2(CH_2CO_2-)$ ; $-CH_2CH_2-NH-C(CH_3)_3$ ; $-CH_2CH_2-N(CH_3)_2$ ; pyrrolidinone ; caprolactame ;

et dans laquelle les indices a et b sont des nombres entiers, identiques ou différents, supérieurs à 1 ; ou

- parmi des polymères non-associatifs ASE-F de formule générale (II) suivante :

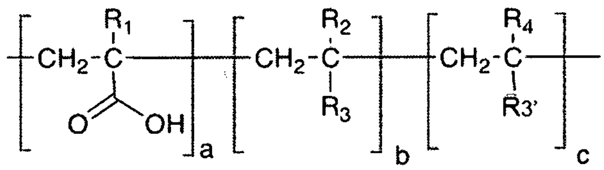

(II)

dans laquelle :

- R1, R2 et R4 représentent un atome d'hydrogène ou un groupement méthyle-$CH_3$ ;
- R3 représente $[Q]_{d1}-(CH_2)_n-H$ dans laquelle n est compris entre 1 et 30, d1 correspond à 0 ou à 1, et Q correspond à -C(O)-O ou -C(O)-NH- ;

ou

R3 représente $[Q]_{d2}-\alpha$, dans laquelle :

• d2 correspond à 0 ou 1 ;
• Q correspond à -C(O)-O ou -C(O)-NH- ; et
• $\alpha$ correspond à $-C(CH_3)_3$ ; $-CH(CH_3)_2$ ; $-C(CH_3)_2-CH_2-C(CH_3)_3$ ; -CN ; $-CH_2CH_2-N+(CH_3)_2(CH_2CO_2-)$ ; $-CH_2CH_2-NH-C(CH_3)_3$ ; $-CH_2CH_2-N(CH_3)_2$ ; pyrrolidinone ; caprolactame ;

- R3' représente $[Q]_{d1}-(CH_2)_n-(CX_2)_pX$ dans laquelle n est compris entre 1 et 30, d1 correspond à 0 ou à 1, Q correspond à -C (0) -0 ou -C(O)-NH- , X est un atome de fluor F et p est compris entre 1 et 12 ; et dans laquelle les indices a et c sont des nombres entiers, identiques ou différents, supérieurs à 1 et b est supérieur ou égal à 0 ;

ou

- parmi les polymères associatifs HASE à macromères à chaine hydrocarbonée (HASE-H-RH ou HASE-F-RH) ou à chaine fluorocarbonée (HASE-F-RF) répondant à la formule générale (III) suivante :

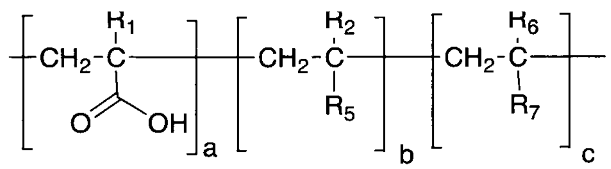

(III)

dans laquelle :

- R1, R2 et R6 représentent un atome d'hydrogène ou un groupement méthyle ;
- R5 représente $[Q]_{d1}-(CH_2)_n-(CX_2)_pX$ dans laquelle n est compris entre 1 et 30, d1 correspond à 0 ou à 1, et Q correspond à -C(O)-O ou -C(O)-NH- ; et :

  • lorsque X est un atome d'hydrogène, alors p est égal à 0 ;
  • lorsque X est un atome de fluor, alors p est compris entre 1 et 12 ;

  ou

  R5 représente $[Q]_{d2}-\alpha$, dans laquelle :

  • d2 correspond à 0 ou 1 ;
  • Q correspond à -C(O)-O ou -C(O)-NH- et
  • $\alpha$ correspond à $-C(CH_3)_3$ ; $-CH(CH_3)_2$ ; $-C(CH_3)_2-CH_2-C(CH_3)_3$ ; -CN ; $-CH_2CH_2-N+(CH_3)_2(CH_2CO_2-)$ ; $-CH_2CH_2-NH-C(CH_3)_3$ ; $-CH_2CH_2-N(CH_3)_2$ ; pyrrolidinone ; caprolactame ;

- R7 représente $-[Q']_{d3}-(OCH_2CH_2)_q-[Q'']_{d4}-(CH_2)_n(CX_2)_pX$ dans laquelle Q' correspond à $-CH_2$, C(O), O-C(O) ou -NH-C(O), n est compris entre 1 et 30, q est compris entre 1 et 150, d3 et d4 correspondent à 0 et/ou à 1, Q" correspond à -O-C(O) ou -NH-C(O) ; et :

  • lorsque X est un atome d'hydrogène, alors p est égal à 0 ;
  • lorsque X est un atome de fluor, alors p est compris entre 1 et 12 ;
  et dans laquelle les indices a et c sont des nombres entiers, identiques ou différents, supérieurs ou égaux à 1, et b est supérieur ou égal à 0.

6. Composé selon la revendication 5, **caractérisé en ce que** les polymères modificateurs ou adaptateurs de rhéologie sont choisis parmi :

- ASE-H ;
- ASE-F ;
- HASE-H-RH4 ;
- HASE-H-RH6 ;
- HASE-H-RH8 ;
- HASE-F-RH4 ;
- HASE-F-RH6 ;
- HASE-F-RH8 ;
- HASE-F-RF4 ;
- HASE-F-RF6 ; ou
- HASE-F-RF8.

7. Composé selon la revendication 5, **caractérisé en ce que** les polymères modificateurs ou adaptateurs de rhéologie sont des polymères associatifs dont le pourcentage molaire de macromonomères ou macromères est compris entre 3 et 50% du polymère.

8. Topique protecteur comprenant un composé selon l'une des revendications 1 à 7, dans un milieu pharmaceutiquement et/ou cosmétiquement acceptable.

9. Topique selon la revendication 8, comprenant en outre un ou plusieurs agents détoxifiants et/ou un ou plusieurs polymères complémentaires.

10. Composé selon l'une des revendications 1 à 7 ou topique protecteur selon l'une des revendications 9 ou 10, à titre de médicament.

11. Composé ou topique protecteur selon la revendication 10, pour son utilisation dans la prévention des irritations cutanées ou des allergies.

12. Composé selon l'une des revendications 1 à 7 ou topique protecteur selon l'une des revendications 8 ou 9, pour son utilisation dans la protection ou la décontamination cutanée due aux agents du risque biologique ou chimique.

13. Composé selon la revendication 12, pour la protection cutanée contre les agents toxiques de la famille des composés organophosphorés (COP) ou contre les agents vésicants.

14. Procédé de synthèse d'un composé selon l'une des revendications 1 à 7 comprenant les étapes de :

   - mélange d'un agent de couplage avec un catalyseur ;
   - ajout du mélange obtenu à une solution de polymères, modificateurs ou adaptateurs de rhéologie choisis parmi les polymères non-associatifs ou les polymères associatifs, dans de l'eau ;
   - agitation du mélange réactionnel ;
   - ajout des micro- ou nanoparticules fonctionnalisées d'oxyde d'aluminium ($Al_2O_3$) d'oxyde de magnésium (MgO), de silice ($SiO_2$), de dioxyde de titane ($TiO_2$), d'oxyde de zinc (ZnO) ou d'oxyde de cuivre (CuO) présentant un diamètre nominal compris entre 5 et 1 500 nm, préalablement dispersées en phase aqueuse au mélange réactionnel ;
   - purification du milieu réactionnel par dialyse ; et
   - récupération du composé formé par une ou plusieurs micro- ou nanoparticules fonctionnalisées amines associées de façon covalente à un ou plusieurs polymères modificateurs de rhéologie.

15. Procédé selon la revendication 14, **caractérisé en ce que** l'agent de couplage est l'hydrochlorure de N-(3-diméthylaminopropyl)-N'-éthylcarbodiimide (EDC) et le catalyseur est le N-hydroxysuccinimide (NHS).

**Patentansprüche**

1. Verbindung, gebildet aus aminofunktionalisierten Mikro- oder Nanopartikeln, welche kovalent an rheologiemodifizierende Polymere gebunden sind, **dadurch gekennzeichnet, dass**:

   - die funktionalisierten Mikro- oder Nanopartikel funktionalisierte anorganische oder metallische Oxid-, insbesondere Aluminiumoxid- ($Al_2O_3$), Kupferoxid- (CuO), Eisenoxid- ($Fe_3O_4$ oder $\gamma$-$Fe_2O_3$), Magnesiumoxid- (MgO), Silizium- ($SiO_2$), Titandioxid- ($TiO_2$) oder Zinkoxid- (ZnO) Mikro- oder Nanopartikel sind, die einen Nenndurchmesser von zwischen 1 und 1500 nm aufweisen;
   - die rheologiemodifizierenden oder -adaptierenden Polymere ausgewählt sind aus nicht-assoziativen Polymeren oder assoziativen Polymeren.

2. Verbindung nach Anspruch 1, **dadurch gekennzeichnet, dass** die aminofunktionalisierten Mikro- oder Nanopartikel aminofunktionalisierte Silizium- ($SiO_2$) oder Titandioxid-($TiO_2$) Mikro- oder Nanopartikel sind.

3. Verbindung nach einem der Ansprüche 1 oder 2, **dadurch gekennzeichnet, dass** die aminofunktionalisierten Mikro- oder Nanopartikel einen Anteil an Aminofunktionen von zwischen 0,1 und 10 meq/g Mikro- oder Nanopartikeln aufweisen.

**4.** Verbindung nach einem der Ansprüche 2 oder 3, **dadurch gekennzeichnet, dass** die aminofunktionalisierten Silizium- ($SiO_2$ oder Titandioxid- ($TiO_2$) Mikro- oder Nanopartikel Nanopartikel sind, deren Nenndurchmesser jeweils zwischen 5 und 500 nm und zwischen 1 und 300 nm beträgt.

**5.** Verbindung nach einem der vorstehenden Ansprüche, **dadurch gekennzeichnet, dass** die rheologiemodifizierenden oder -adaptierenden Polymere ausgewählt sind:

- aus nicht-assoziativen ASE-H-Polymeren der folgenden allgemeinen Formel (I):

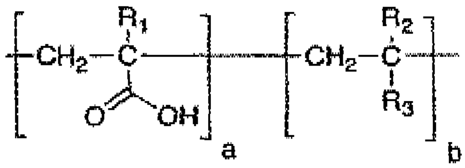

(I)

wobei:

- R1 und R2 für ein Wasserstoffatom oder eine $-CH_3$-Methylgruppe stehen;
- R3 für $[Q]_{d1}-(CH_2)_n-H$ steht, wobei n zwischen 1 und 30 beträgt, d1 0 oder 1 entspricht, und Q -C(O)-O oder - C(O)-NH- entspricht;
oder
R3 für $[Q]_{d2}-\alpha$ steht, wobei:

-- d2 0 oder 1 entspricht;
-- Q -C(O)-O oder -C(O)-NH- entspricht; und
-- $\alpha$ $-C(CH_3)_3$; $-CH(CH_3)_2$; $-C(CH_3)_2-CH_2-C(CH_3)_3$; -CN; - $CH_2CH_2-N+(CH_3)_2(CH_2CO)_2^-$); $-CH_2CH_2-NH-C(CH_3)_3$; $-CH_2CH_2-N(CH_3)_2$; Pyrrolidinon, Caprolactam entspricht;
und wobei die Indizes a und b gleiche oder unterschiedliche Ganzzahlen größer 1 sind;
oder

- aus nicht-assoziativen ASE-F-Polymeren der folgenden allgemeinen Formel (II):

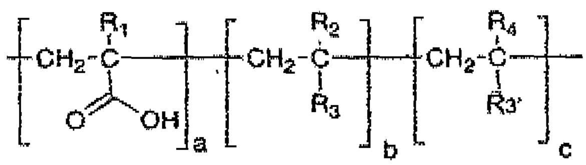

(II)

wobei:

- R1 R2 und R4 für ein Wasserstoffatom oder eine $-CH_3$-Methylgruppe stehen;
- R3 für $[Q]_{d1}-(CH_2)_n-H$ steht, wobei n zwischen 1 und 30 beträgt, d1 0 oder 1 entspricht, und Q -C(O)-O oder - C(O)-NH- entspricht;
oder
R3 für $[Q]_{d2}-\alpha$ steht, wobei:

-- d2 0 oder 1 entspricht;
-- Q -C(O)-O oder -C(O)-NH- entspricht; und
-- $\alpha$ $C(CH_3)_3$; $-CH(CH_3)_2$; $-C(CH_3)_2-CH_2-C(CH_3)_3$; -CN; - $CH_2CH_2-N+$ $(CH_3)_2$ $(CH_2CO_2^-)$; $-CH_2CH_2-NH-C(CH_3)_3$; $-CH_2CH_2-N(CH_3)_2$; Pyrrolidinon, Caprolactam entspricht;

- R3' für $[Q]_{d1}-(CH_2)_n-(CX_2)_pX$ steht, wobei n zwischen 1 und 30 beträgt, d1 0 oder 1 entspricht, Q

-C(O)-O oder - C(O)-NH- entspricht, X ein Fluoratom F ist und p zwischen 1 und 12 beträgt; und wobei die Indizes a und c gleiche oder unterschiedliche Ganzzahlen größer 1 sind und b größer oder gleich 0 ist;
oder
- aus assoziativen HASE-Polymeren mit Makromeren mit Kohlenwasserstoffkette (HASE-H-RH oder HASE-F-RH) oder mit Fluorkohlenstoffkette (HASE-F-RF), die der folgenden allgemeinen Formel (III) entsprechen:

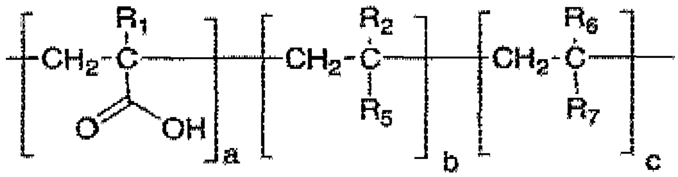

(III)

wobei:

- R1, R2 und R6 für ein Wasserstoffatom oder eine Methylgruppe stehen;
- R5 für $[Q]_{d1}$-$(CH_2)_n$-$(CX_2)_pX$ steht, wobei n zwischen 1 und 30 beträgt, d1 0 oder 1 entspricht, und Q -C(O)-O oder - C(O)-NH- entspricht; und:

  -- wenn x ein Wassersstoffatom ist, p gleich 0 ist;
  -- wenn X ein Fluoratom ist, p zwischen 1 und 12 beträgt; oder
  R5 für $[Q]_{d2}$-α steht, wobei:

    -- d2 0 oder 1 entspricht;
    -- Q -C(O)-O oder -C(O)-NH- entspricht; und
    -- α $-C(CH_3)_3$; $-CH(CH_3)_2$ $-C(CH_3)_2$-$CH_2$-$C(CH_3)_3$; -CN; $CH_2CH_2$-N+$(CH_3)_2(CH_2CO_2^-)$; $-CH_2CH_2NH$-$C(CH_3)_3$; $-CH_2CH_2$-$N(CH_3)_2$; Pyrrolidinon, Caprolactam entspricht;

- R7 für -$[Q']_{d3}$-$(OCH_2CH_2)_q$-$[Q'']_{d4}$-$(CH_2)_n(CX_2)_pX$ steht, wobei Q' $-CH_2$, C(O), O-C(O) oder -NH-C(O) entspricht, n zwischen 1 und 30 beträgt, q zwischen 1 und 150 beträgt, d3 und d4 0 und/oder 1 entsprechen, Q'' -O-C(O) oder -NH-C(O) entspricht; und:

  -- wenn X ein Wassersstoffatom ist, p gleich 0 ist;
  -- wenn X ein Fluoratom ist, p zwischen 1 und 12 beträgt;
  und wobei die Indizes a und c gleiche oder unterschiedliche Ganzzahlen größer oder gleich 1 sind und b größer oder gleich 0 ist.

6. Verbindung nach Anspruch 5, **dadurch gekennzeichnet, dass** die rheologiemodifizierenden oder -adaptierenden Polymere ausgewählt sind aus:

- ASE-H;
- ASE-F;
- HASE-H-RH4;
- HASE-H-RH6;
- HASE-H-RH8;
- HASE-F-RH4;
- HASE-F-RH6;
- HASE-F-RH8;
- HASE-F-RF4;
- HASE-F-RF6; oder
- HASE-F-RF8.

7. Verbindung nach Anspruch 5, **dadurch gekennzeichnet, dass** die rheologiemodifizierenden oder -adaptierenden

Polymere assoziative Polymere sind, deren Molprozentsatz an Makromonomeren oder Makromeren zwischen 3 und 50 % des Polymers beträgt.

**8.** Schützendes Topikum, welches eine Verbindung nach einem der Ansprüche 1 bis 7 in einem pharmazeutisch und/oder kosmetisch akzeptablen Medium umfasst.

**9.** Topikum nach Anspruch 8, welches des Weiteren ein oder mehrere Entgiftungsmittel und/oder ein oder mehrere ergänzende Polymere umfasst.

**10.** Verbindung nach einem der Ansprüche 1 bis 7 oder schützendes Topikum nach einem der Ansprüche 9 oder 10, als Arzneimittel.

**11.** Verbindung oder schützendes Topikum nach Anspruch 10, zu seiner Verwendung in der Vorbeugung von Hautreizungen oder Allergien.

**12.** Verbindung nach einem der Ansprüche 1 bis 7 oder schützendes Topikum nach einem der Ansprüche 8 oder 9, zu seiner Verwendung im Schutz oder der Cekontaminierung der Haut infolge biologischer oder chemischer Gefahrstoffe.

**13.** Verbindung nach Anspruch 12, zum Schutz der Haut vor Giftstoffen der Familie der Organophosphorverbindungen (OPC) oder vor Vesikantien.

**14.** Verfahren zur Herstellung einer Verbindung nach einem der Ansprüche 1 bis 7, welches die Schritte umfasst des:

- Vermischens eines Kopplungsmittels mit einem Katalysator;
- Hinzufügens des erhaltenen Gemisches zu einer Lösung aus rheologiemodifizierenden oder -adaptierenden Polymeren, ausgewählt aus nicht-assoziativen Polymeren oder assoziativen Polymeren, in Wasser;
- Rührens des Reaktionsgemisches;
- Hinzufügens der zuvor in wässriger Phase dispergierten funktionalisierten Aluminiumoxid- ($Al_2O_3$), Magnesiumoxid-(MgO), Silizium- ($SiO_2$), Titandioxid- ($TiO_2$), Zinkoxid-(ZnO) oder Kupferoxid- (CuO) Mikro- oder Nanopartikel, die einen Nenndurchmesser von zwischen 5 und 1500 nm aufweisen, zu dem Reaktionsgemisch,
- Reinigens des Reaktionsmediums mittels Dialyse; und
- Rückgewinnens der Verbindung, gebildet aus einem oder mehreren aminofunktionalisierten Mikro- oder Nanopartikeln, welche kovalent an ein oder mehrere rheologiemodifizierende Polymere gebunden sind.

**15.** Verfahren nach Anspruch 14, **dadurch gekennzeichnet, dass** das Kopplungsmittel N-(3-Dimethylaminopropyl)-N'-ethylcarbodiimid-Hydrochlorid (EDC) ist und der Katalysator N-Hydroxysuccinimid (NHS) ist.

## Claims

**1.** Compound formed by amine functionalised micro- or nanoparticles covalently associated with rheology-modifying polymers, **characterised in that**:

- the functionalised micro- or nanoparticles are functionalised micro- or nanoparticles of metal or inorganic oxide in particular aluminium oxide ($Al_2O_3$), copper oxide (CuO), iron oxide ($Fe_3O_4$ or $\gamma$-$Fe_2O_3$), magnesium oxide (MgO), silica ($SiO_2$), titanium dioxide ($TiO_2$) or zinc oxide (ZnO), having a nominal diameter of between 1 and 1500 nm;
- the rheology-modifying or -adapting polymers are chosen from non-associative polymers or associative polymers.

**2.** Compound according to claim 1, **characterised in that** the amine functionalised micro- or nanoparticles are amine functionalised micro- or nanoparticles of silica ($SiO_2$) or of titanium dioxide ($TiO_2$).

**3.** Compound according to one of claims 1 or 2, **characterised in that** the amine functionalised micro- or nanoparticles having an amine function rate between 0.1 and 10 meq/g of micro- or nanoparticles.

**4.** Compound according to one of claims 2 or 3, **characterised in that** the amine functionalised micro- or nanoparticles

of silica ($SiO_2$) or of titanium dioxide ($TiO_2$) are nanoparticles of which the nominal diameter is respectively between 5 and 500 nm and between 1 and 300 nm.

**5.** Compound according to one of the preceding claims, **characterised in that** the rheology-modifying or -adapting polymers are chosen:

- from non-associative polymers ASE-H having the following general formula (I):

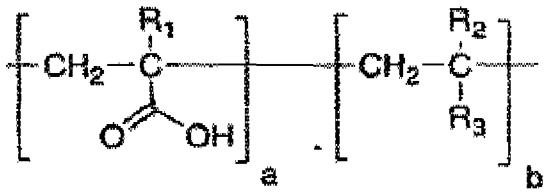

(I)

wherein:

- R1 and R2 represent one atom of hydrogen or a methyl group $-CH_3$;
- R3 represents $[Q]_{d1}-(CH_2)_n-H$ wherein n is between 1 and 30, d1 corresponds to 0 or to 1, and Q corresponds to -C(O)-O or C(O)-NH-;

or

R3 represents $[Q]_{d2}-\alpha$, wherein:

-- d2 corresponds to 0 or 1;
-- Q corresponds to -C(O)-O or -C(O) -NH-; and
$\alpha$ corresponds to $-C(CH_3)_3$; $-CH(CH_3)_2$; $-C(CH_3)_2-CH_2-C(CH_3)_3$; -CN; $-CH_2CH_2-N+(CH_3)_2(CH_2CO_2^-)$; $-CH_2CH_2-NH-C(CH_3)_3$; $-CH_2CH_2-N\ (CH_3)_2$; pyrrolidinone; Caprolactam;
and wherein the indexes a and b are integers, identical or different, greater than 1;
or

- from non-associative polymers ASE-F having the following general formula (II):

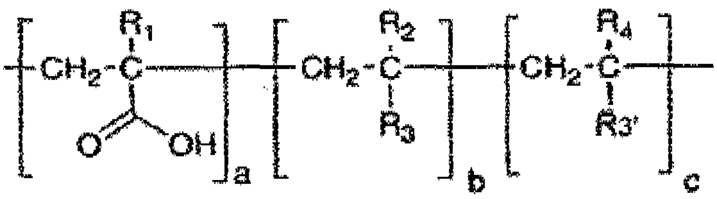

(II)

wherein:

- R1, R2 and R4 represent one atom of hydrogen or a methyl group - CH3;
- R3 represents $[Q]_{d1}-(CH_2)_n-H$ wherein n is between 1 and 30, d1 corresponds to 0 or to 1, and Q corresponds to -C(O)-O or -C(O)-NH-;

or

R3 represents $[Q]_{d2}-\alpha$, wherein:

-- d2 corresponds to 0 or 1;
-- Q corresponds to -C(O)-O or -C(O)-NH-; and
$\alpha$ corresponds to $-C(CH_3)_3$; $-CH(CH_3)_2$; $-C(CH_3)_2-CH_2-C(CH_3)_3$; -CN; $-CH_2CH_2-N+(CH_3)_2(CH_2CO_2^-)$; $-CH_2CH_2-NH-C(CH_3)_3$; $-CH_2CH_2-N\ (CH_3)_2$; pyrrolidinone; caprolactam;

- R3' represents $[Q]_{d1}$-$(CH_2)_n$-$(CX2)_p$X wherein n is between 1 and 30, d1 corresponds to 0 or to 1, Q corresponds to -C(O)-O or -C(O)-NH-, X is an atom of fluorine F and p is between 1 and 12;
and wherein the indexes a and c are integers, identical or different, greater than 1 and b is greater than or equal to 0;
or
- from associative polymers HASE with macromers with a hydrocarbon chain (HASE-H-RH or HASE-F-RH) or with a fluorocarbon chain (HASE-F-RF) having the following general formula (III):

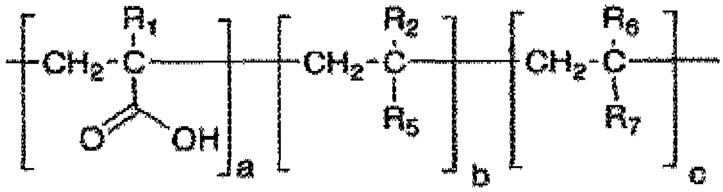

(III)

wherein:

- R1, R2 and R6 represent an atom of hydrogen or a methyl group;
- R5 represents $[Q]_{d1}$-$(CH_2)_n$-$(CX_2)_p$X wherein n is between 1 and 30, d1 corresponds to 0 or to 1, and Q corresponds to -C(O)-O or -C(O)-NH-; and:

  -- when X is an atom of hydrogen, then p is equal to 0;
  -- when X is an atom of fluorine, then p is between 1 and 12;
  or
  R5 represents $[Q]_{d2}$-$\alpha$, wherein:

    -- d2 corresponds to 0 or 1;
    -- Q corresponds to -C(O)-O or -C(O)-NH-; and
    $\alpha$ corresponds to -$C(CH_3)_3$; -$CH(CH_3)_2$; -$C(CH_3)_2$-$CH_2$-$C(CH_3)_3$; -CN; -$CH_2CH_2$-N+$(CH_3)_2(CH_2CO_2^-)$; -$CH_2CH_3$NH-$C(CH_3)_3$; -$CH_2CH_2$-N( $CH_3)_2$; pyrrolidinone; caprolactam;

- R7 represents - $[Q']_{d3}$-$(OCH_2CH_2)_q$-$[Q'']_{d4}$-$(=CH2)_n(CX2)_p$X wherein Q' corresponds to -$CH_2$, C(O), O-C(O) or -NH-C(O), n is between 1 and 30, q is between 1 and 150, d3 and d4 correspond to 0 and/or 1, Q'' corresponds to -O-C(O) or -NH-C(O); and:

  -- when X is an atom of hydrogen, then p is equal to 0;
  -- when X is an atom of fluorine, then p is between 1 and 12;
  and wherein the indexes a and c are integers, identical or different, greater than or equal to 1, and b is greater than or equal to 0.

**6.** Compound according to claim 5, **characterised in that** the rheology-modifying or -adapting polymers are chosen from:

- ASE-H;
- ASE-F;
- HASE-H-RH4;
- HASE-H-RH6;
- HASE-H-RH8;
- HASE-F-RH4;
- HASE-F-RH6;
- HASE-F-RH8;
- HASE-F-RF4;
- HASE-F-RF6; or
- HASE-F-RF8.

7. Compound according to claim 5, **characterised in that** the rheology-modifying or -adapting polymers are associative polymers of which the mole percentage of macromonomers or macromers is between 3 and 50% of the polymer.

8. Protective topical comprising compound according to one of claims 1 to 7, in a pharmaceutically and/or cosmetically acceptable medium.

9. Topical according to claim 8, further comprising one or several detoxifying agents and/or one or several complementary polymers.

10. Compound according to one of claims 1 to 7 or protective topical according to one of claims 9 or 10, as a drug.

11. Compound or protective topical according to claim 10, for the user thereof in the prevention of skin irritations or allergies.

12. Compound according to one of claims 1 to 7 or protective topical according to one of claims 8 or 9, for the use thereof in the protection or décontamination of skin due to agents of the biological or chemical risk.

13. Compound according to claim 12, for protection of skin against toxic agents of the family of organophosphorus compounds (OP) or against blister agents.

14. Method for synthesising a compound according to one of claims 1 to 7 comprising the steps of:

- mixing a coupling agent with a catalyst;
- adding the mixture obtained to a solution of rheology-modifying or -adapting polymers chosen from non-associative polymers or associative polymers, in water;
- stirring of the reaction mixture;
- adding of functionalised micro- or nanoparticles of aluminium oxide ($Al_2O_3$), magnesium oxide (MgO), silica ($SiO_2$), titanium dioxide ($TiO_2$), zinc oxide (ZnO) or of copper oxide (CuO) having a nominal diameter of between 5 and 1500 nm, dispersed beforehand in aqueous phase in the reaction mixture;
- purifying the reaction medium by dialysis; and
- recovering the compound formed by one or several amine functionalised micro- or nanoparticles covalently associated with one or several rheology-modifying polymers.

15. Method according to claim 14, **characterised in that** the coupling agent is N-(3-dimethylaminopropyl)-N'-ethylcarbodiimide hydrochloride (EDC) and the catalyst is N-hydroxysuccinimide (NHS).

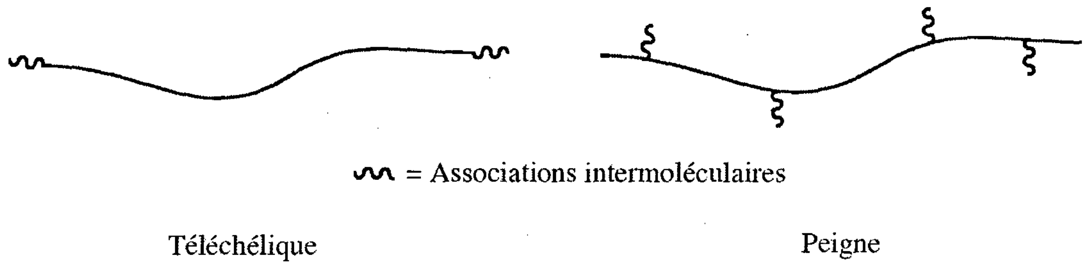


# Figure 1

$$Si(OEt)_4 + H_2O \longrightarrow (OEt)_3Si(OH) + EtOH \quad \text{(hydrolyse)}$$

Acide silicique

$$(OEt)_3Si(OH) + H_2O \longrightarrow SiO_2 + 3EtOH \quad \text{(condensation)}$$

Bilan :

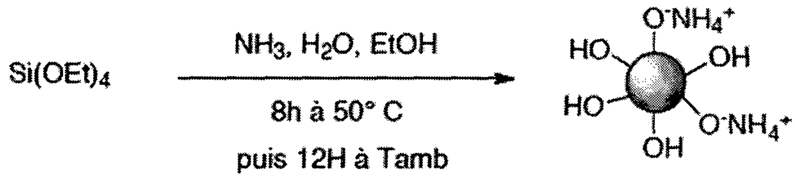


# Figure 2a

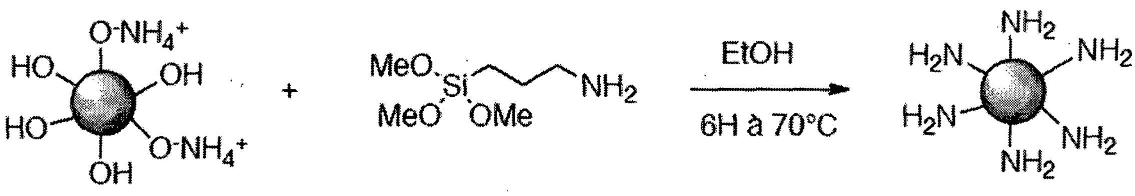


# Figure 2b

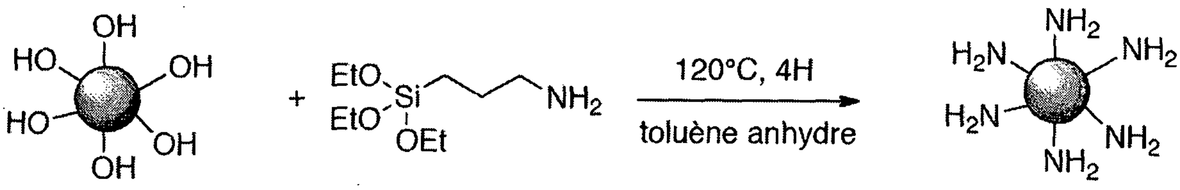


Figure 3

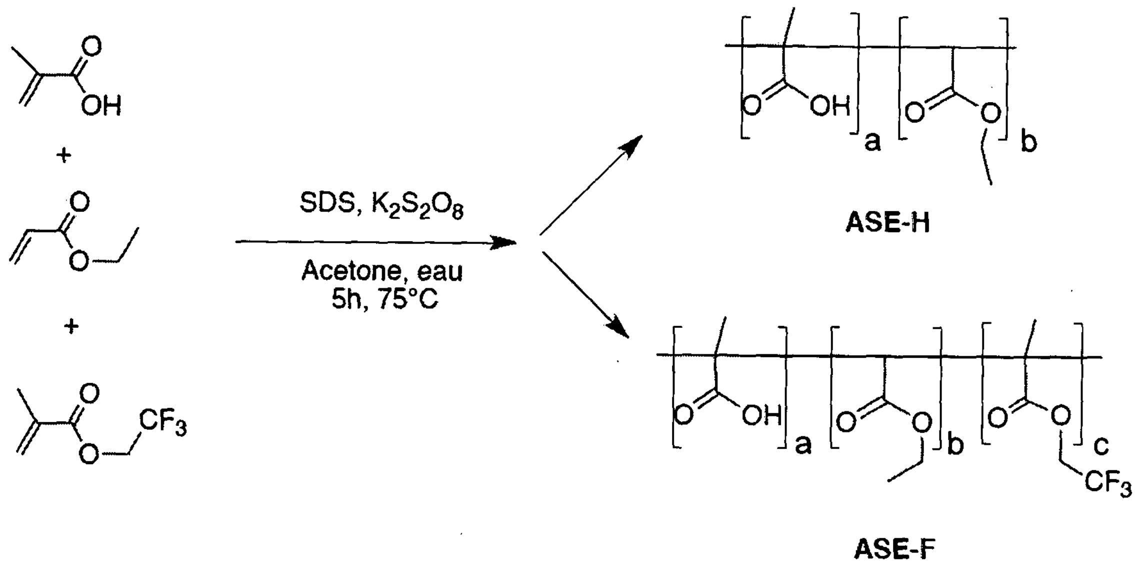


Figure 4

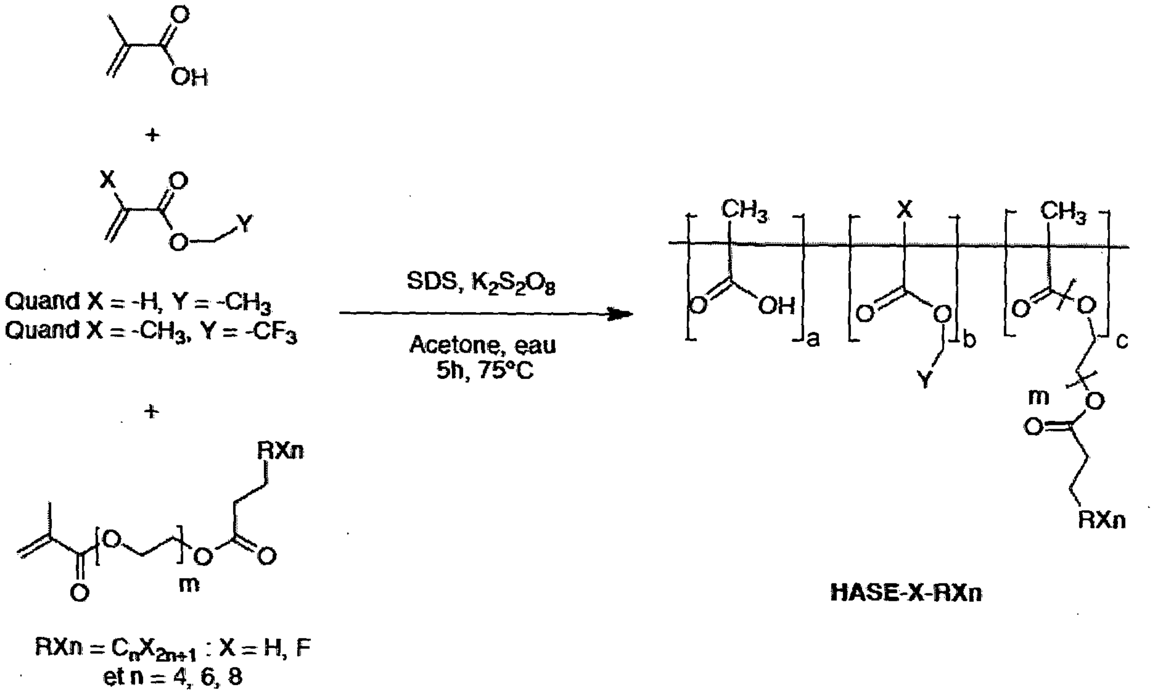


**HASE-X-RXn**

Figure 5

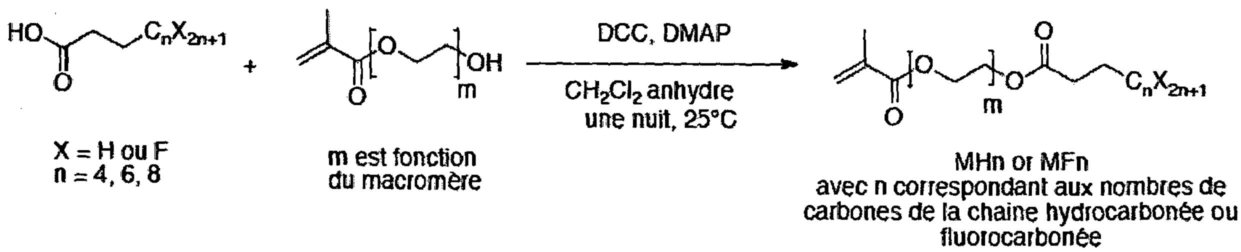


Figure 6

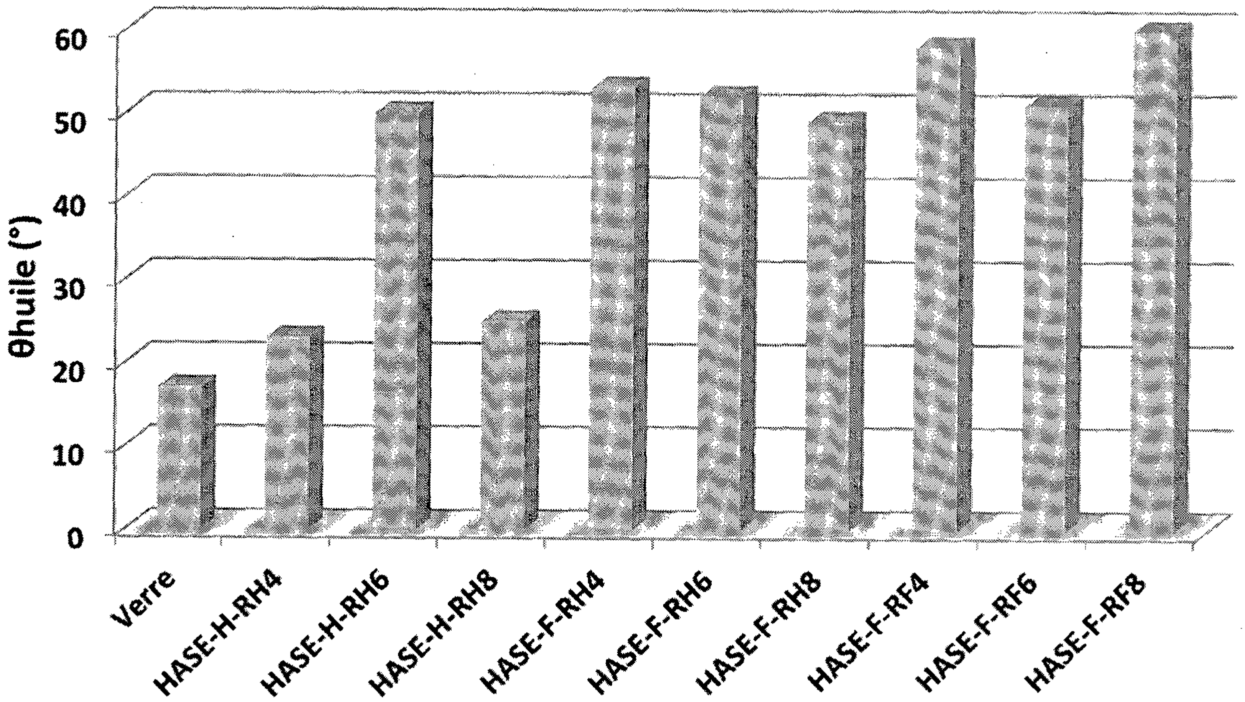

θhuile (°)
60
50
40
30
20
10
0
Verre
HASE-H-RH4
HASE-H-RH6
HASE-H-RH8
HASE-F-RH4
HASE-F-RH6
HASE-F-RH8
HASE-F-RF4
HASE-F-RF6
HASE-F-RF8


Figure 7

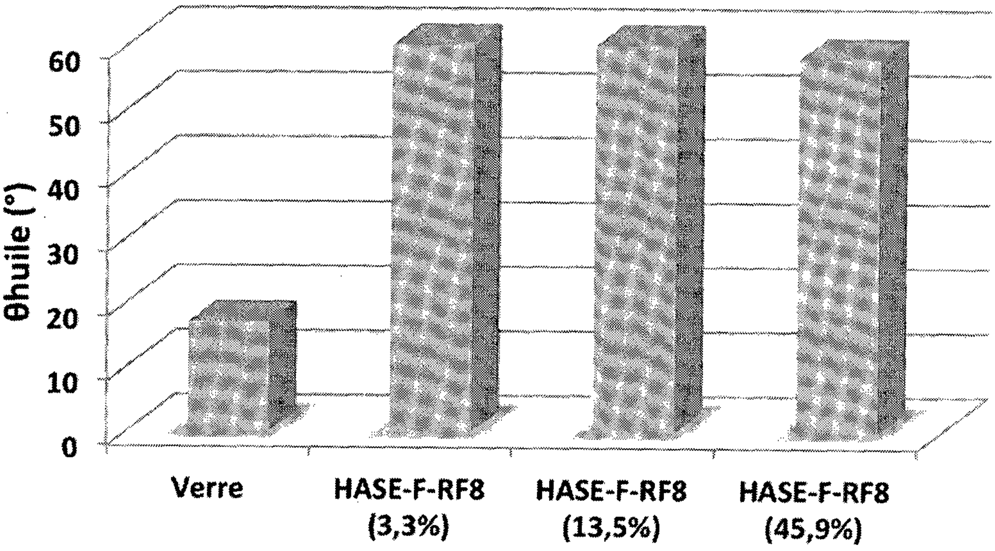


Figure 8

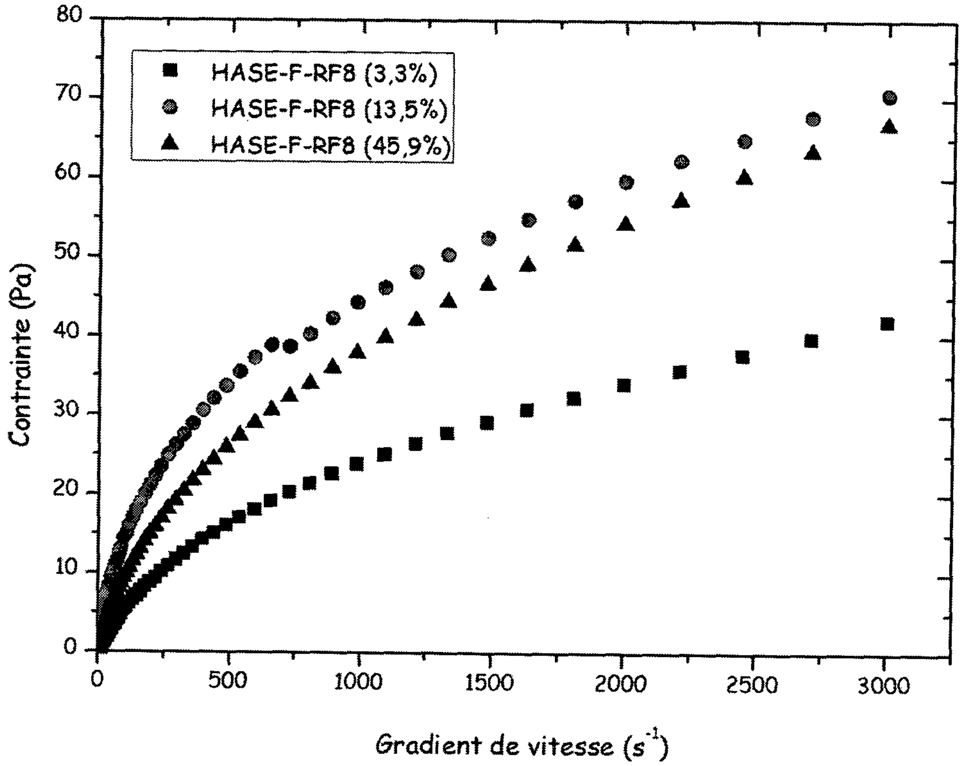


Figure 9

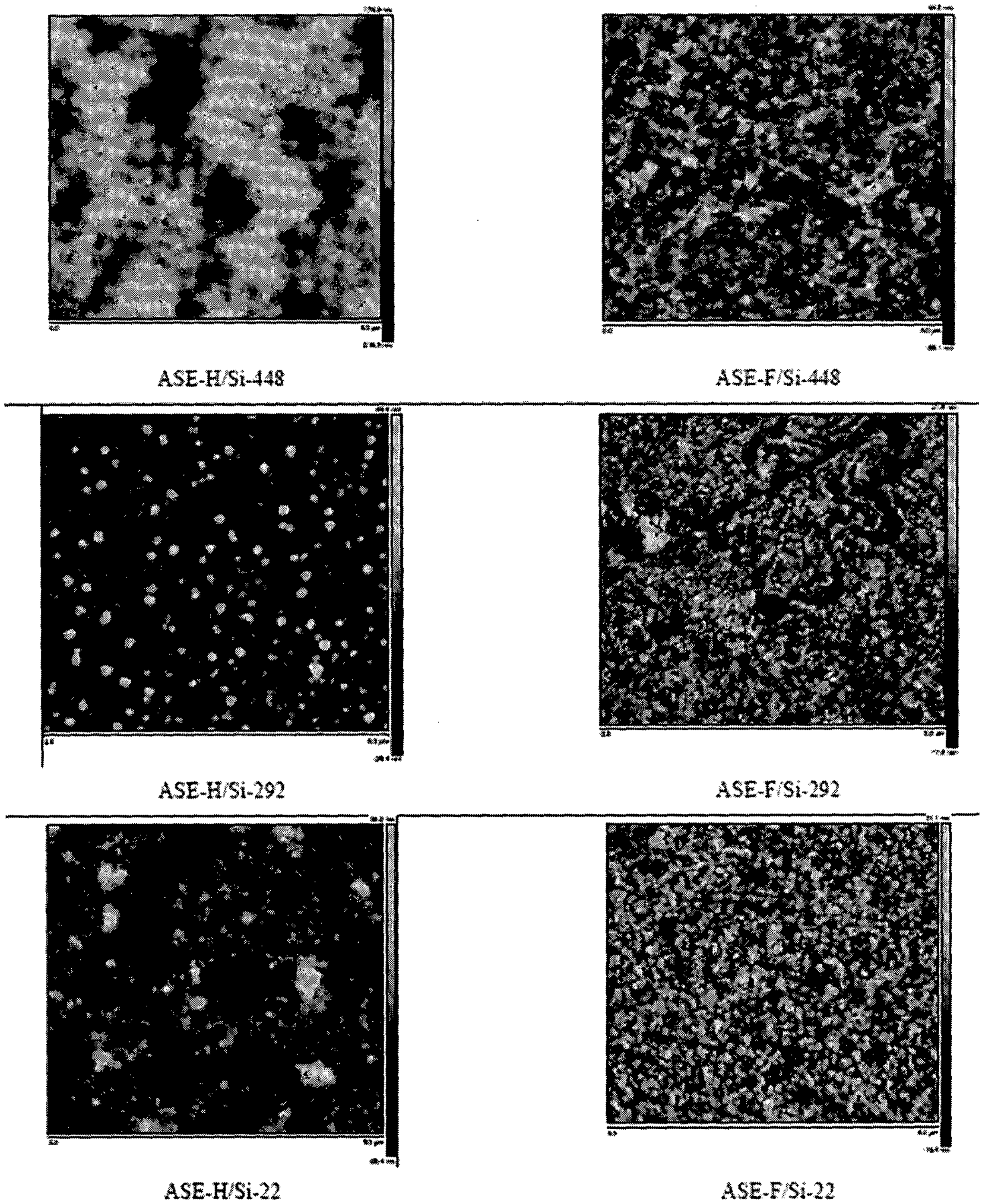
ASE-H/Si-448
ASE-F/Si-448
ASE-H/Si-292
ASE-F/Si-292
ASE-H/Si-22
ASE-F/Si-22

Figure 10

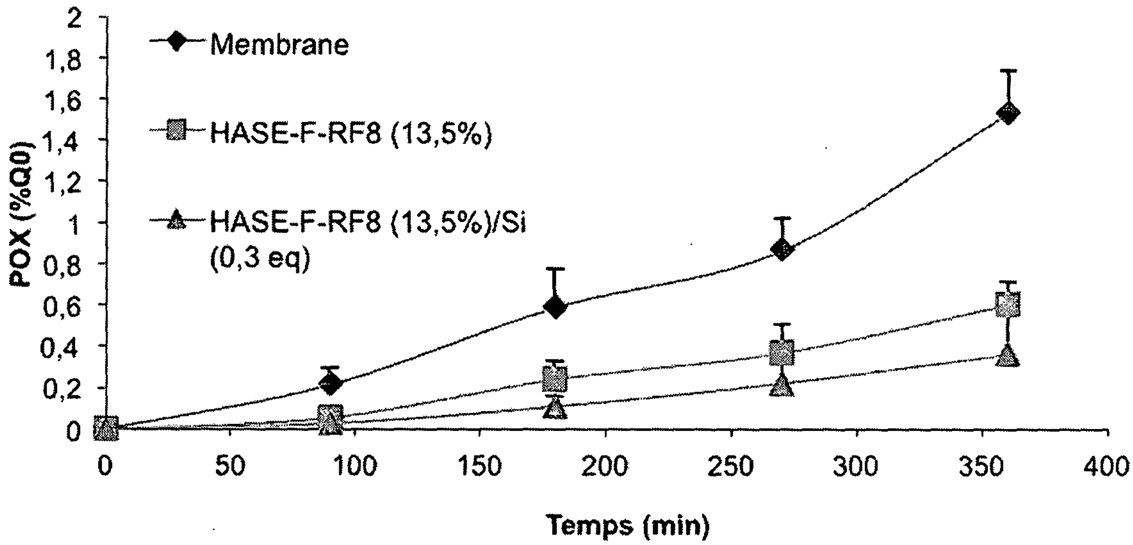

2
1,8
1,6
1,4
1,2
1
0,8
0,6
0,4
0,2
0
POX (%Q0)
Membrane
HASE-F-RF8 (13,5%)
HASE-F-RF8 (13,5%)/Si
(0,3 eq)
0
50
100
150
200
250
300
350
400
Temps (min)


Figure 11

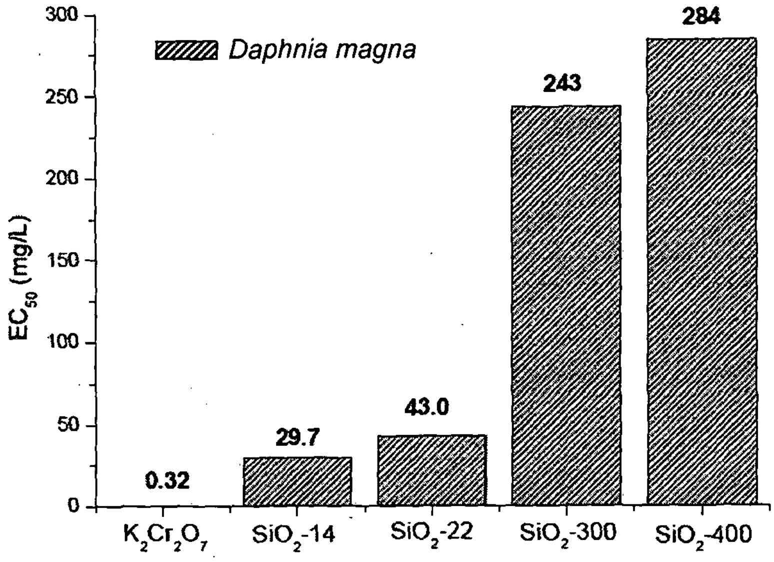


Figure 12

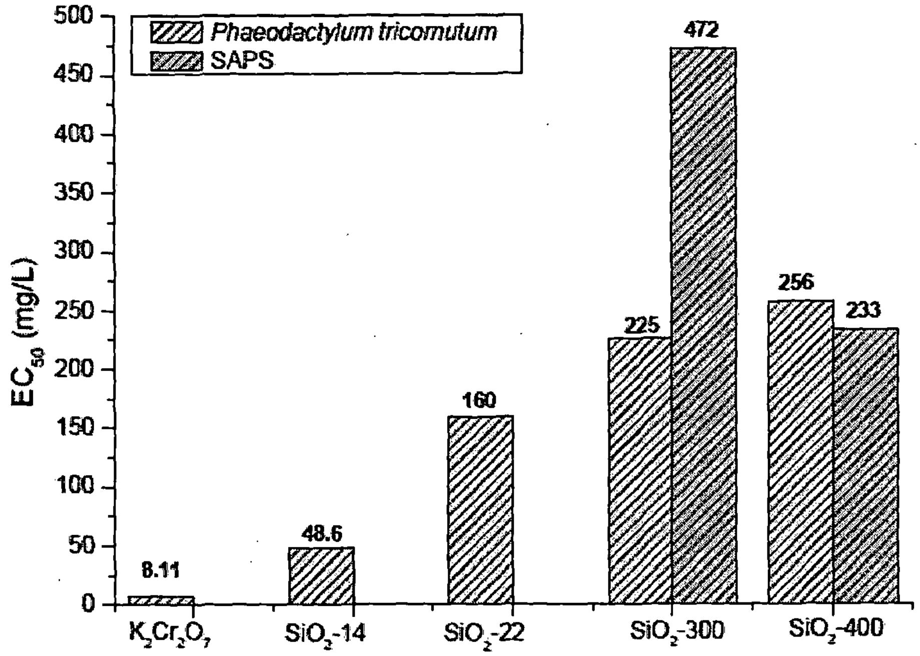


Figure 13

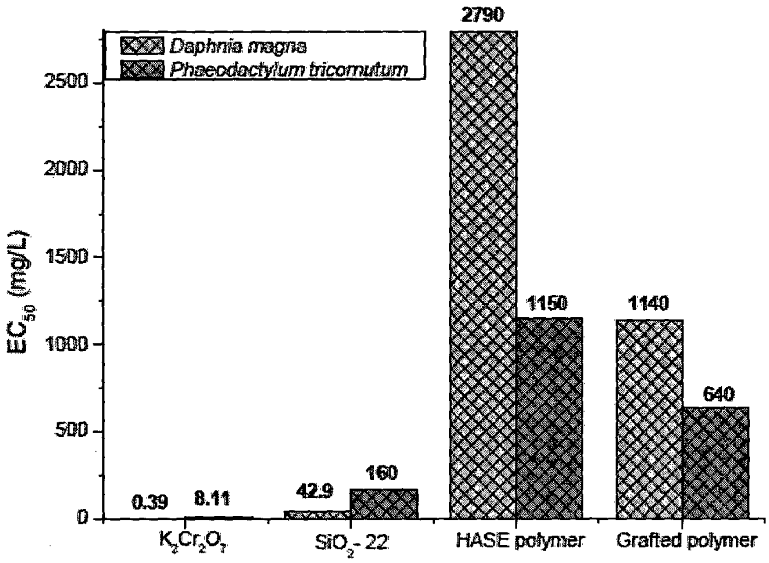


Figure 14

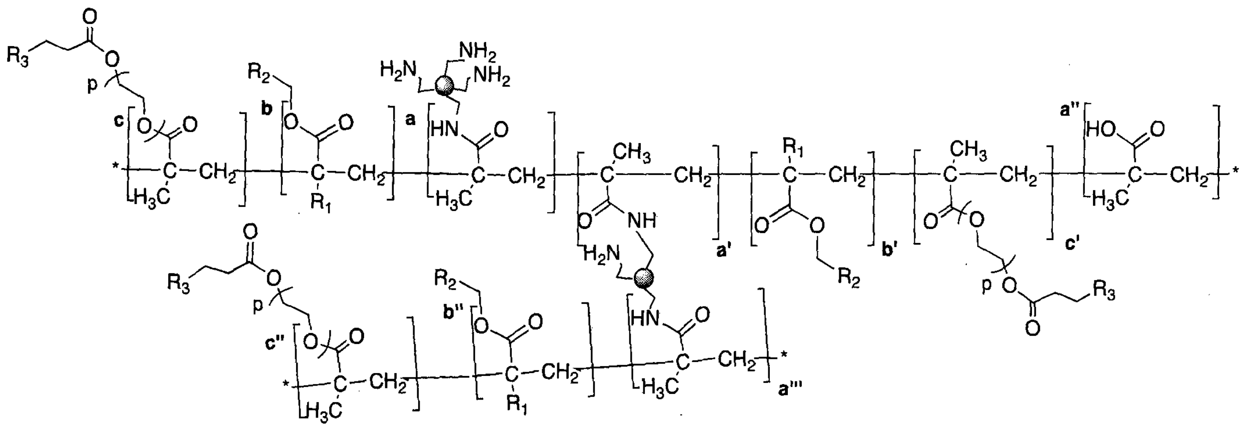

Figure 15

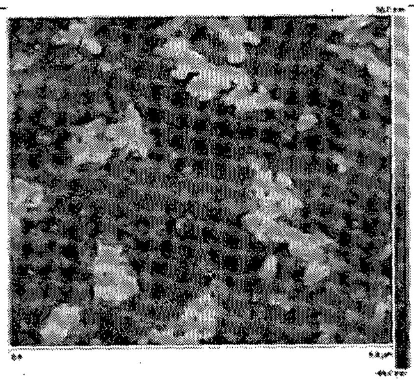

Dépôt à 1 équivalent de silice

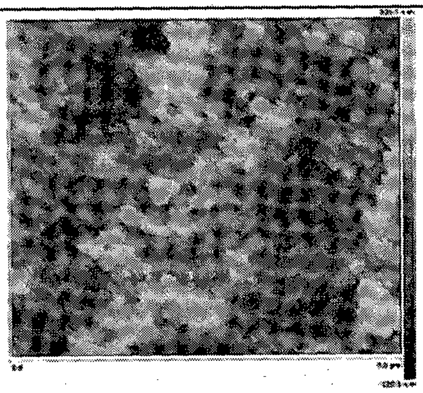

Dépôt à 0,3 équivalent de silice

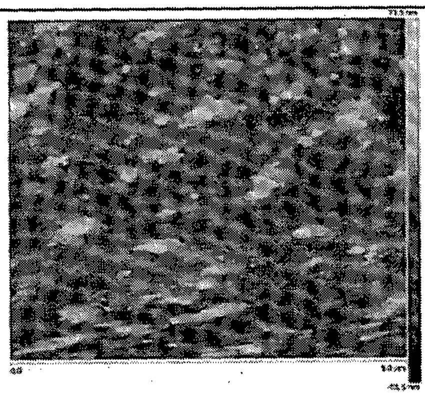

Dépôt à 0,1 équivalent de silice

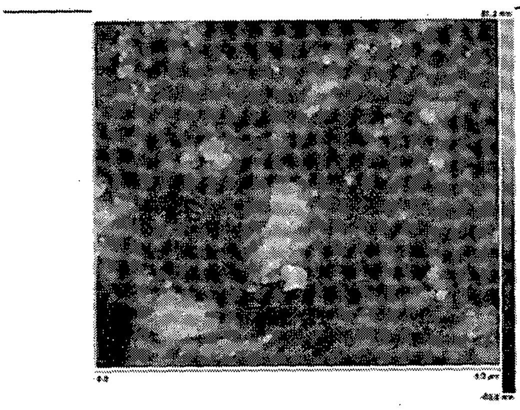

Dépôt à 0,05 équivalent de silice

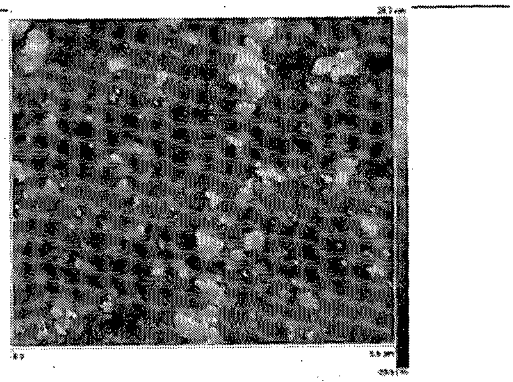

Dépôt à 0,01 équivalent de silice

Figure 16

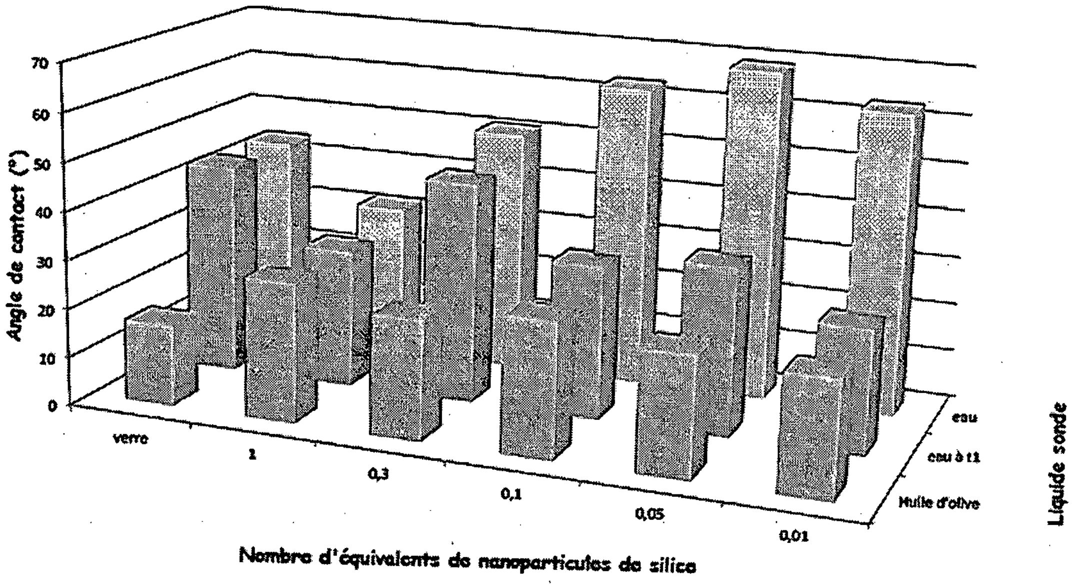


Figure 17

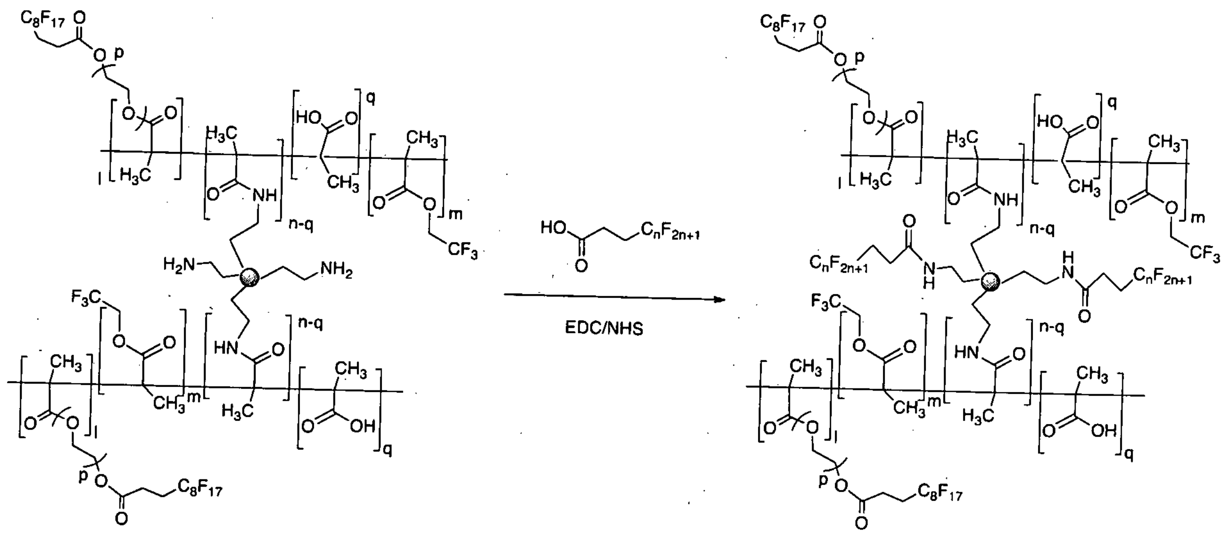


Figure 18

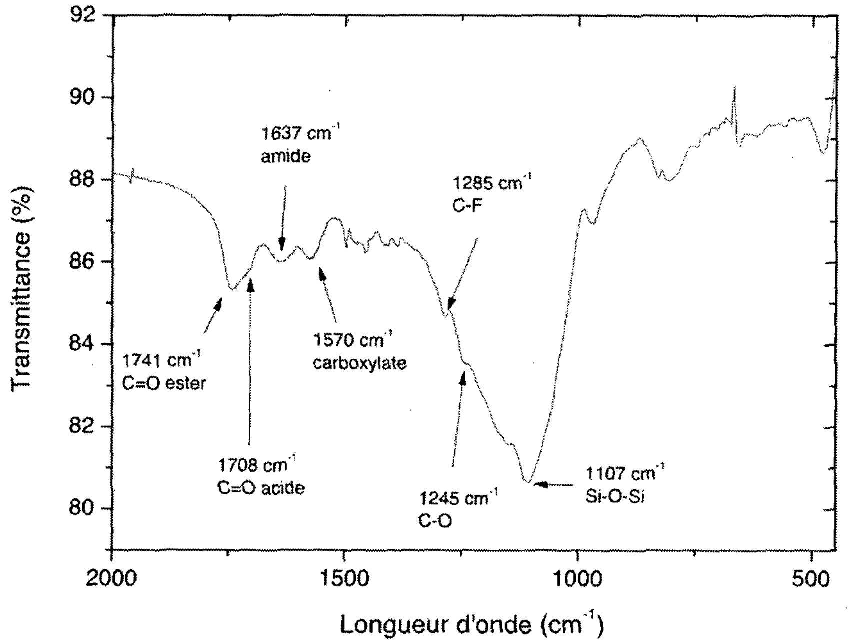


Figure 19

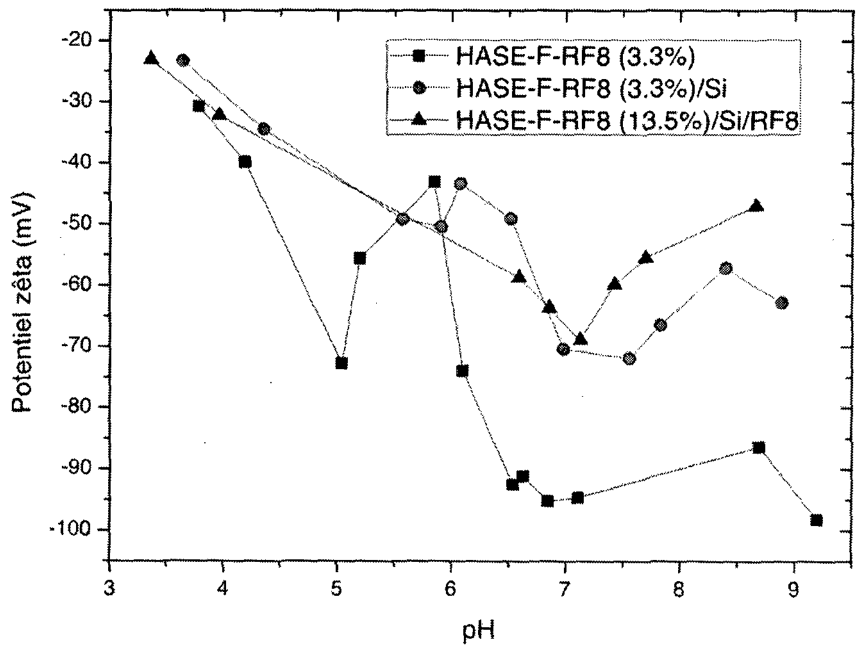


Figure 20

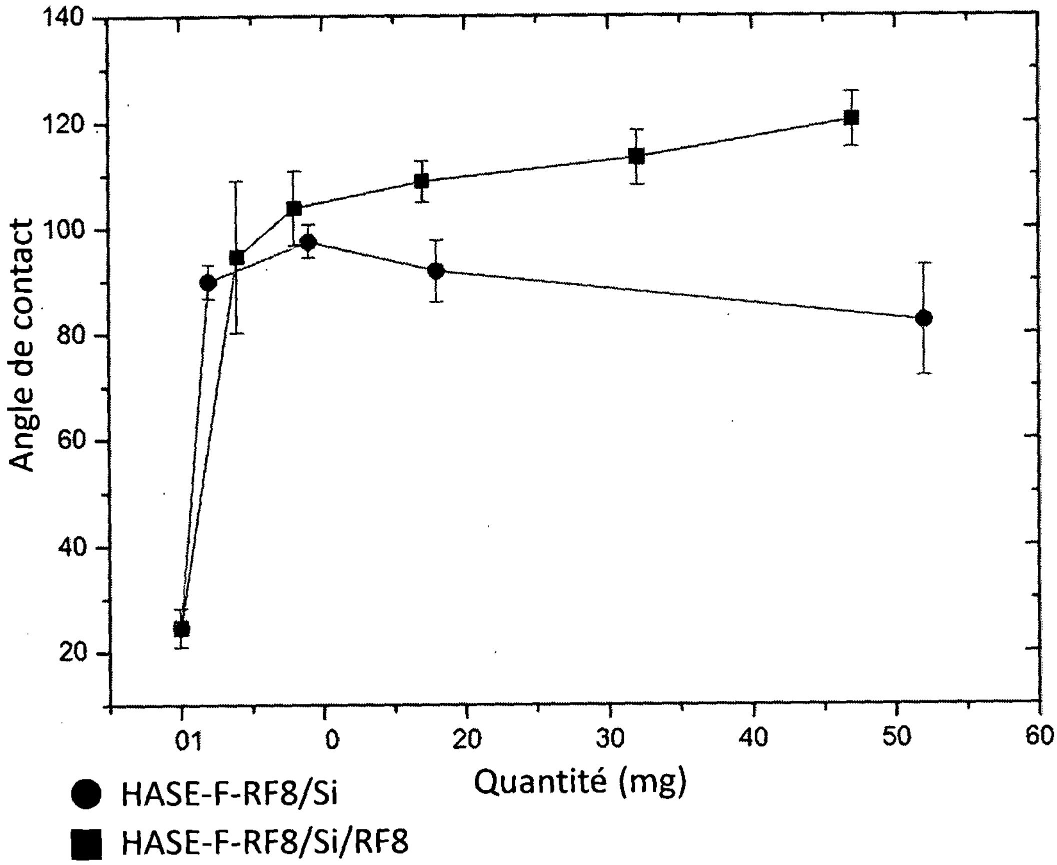


Figure 21

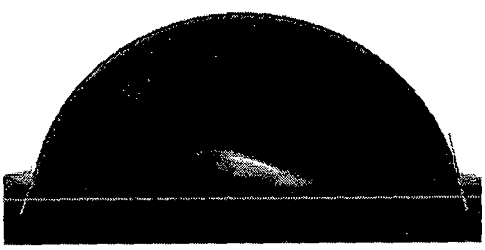

Figure 22a

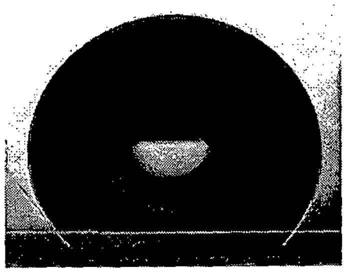

Figure 22b

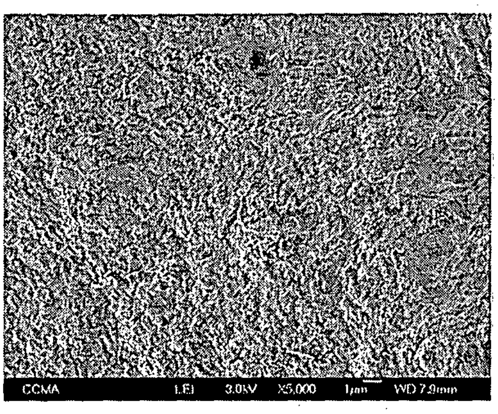

Figure 23a

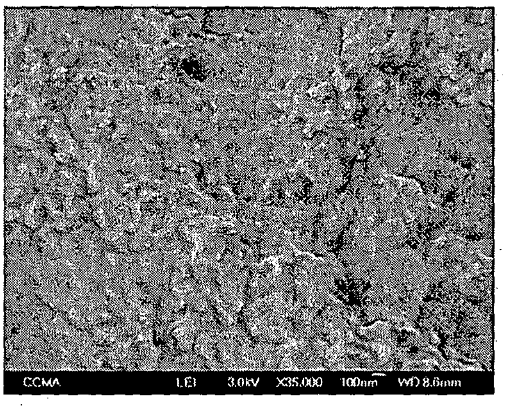

Figure 23b

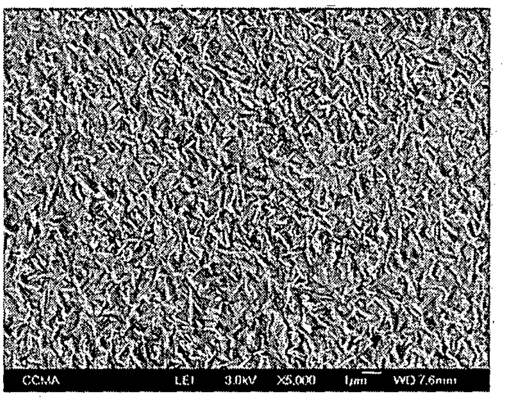

Figure 23c

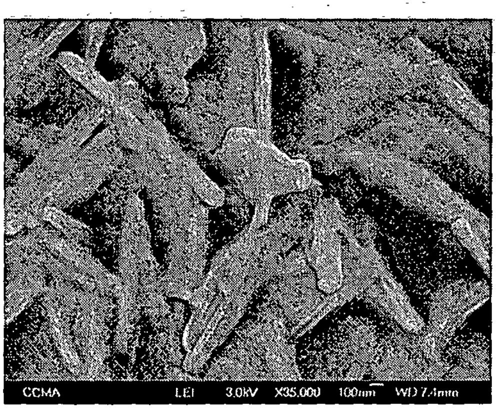

Figure 23d

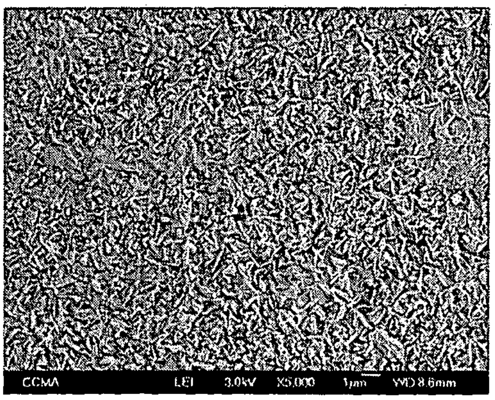

Figure 23e

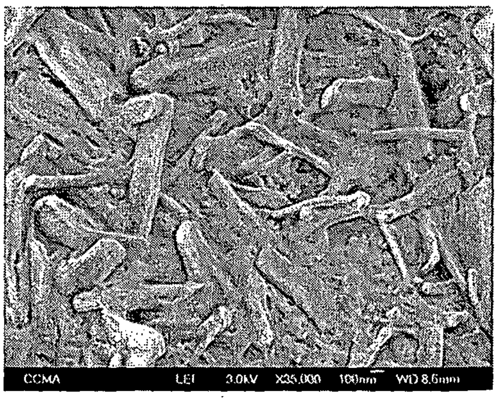

Figure 23f

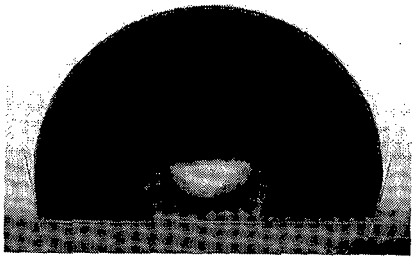

Figure 24a

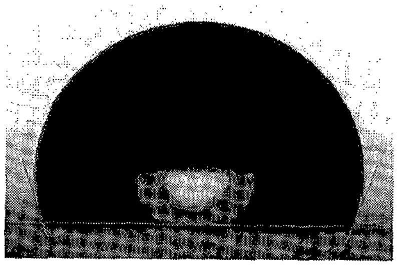

Figure 24b

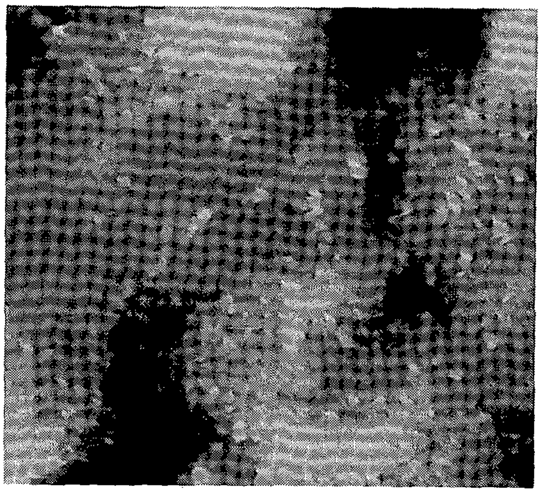

Figure 25a

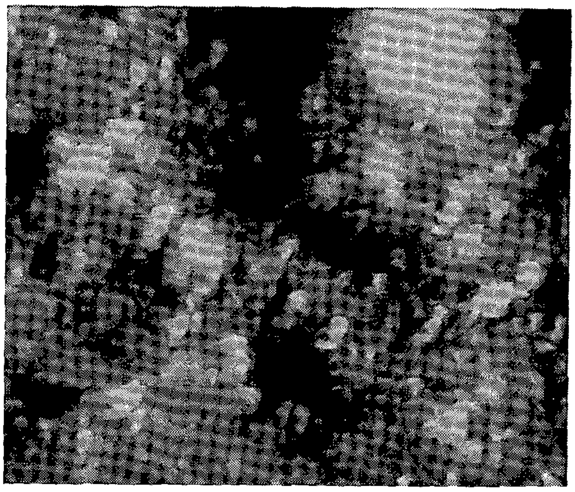

Figure 25b

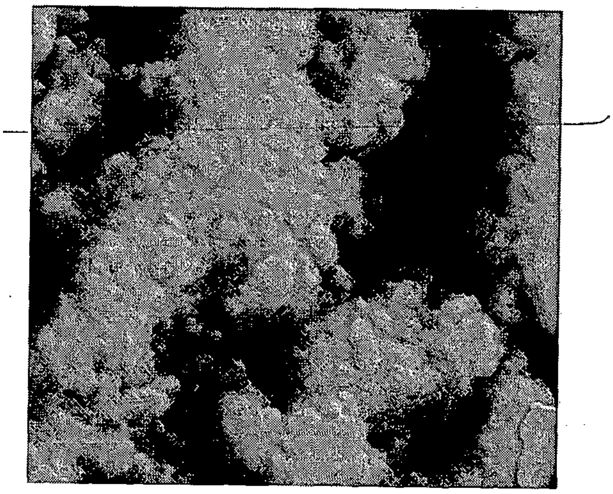

Figure 26a

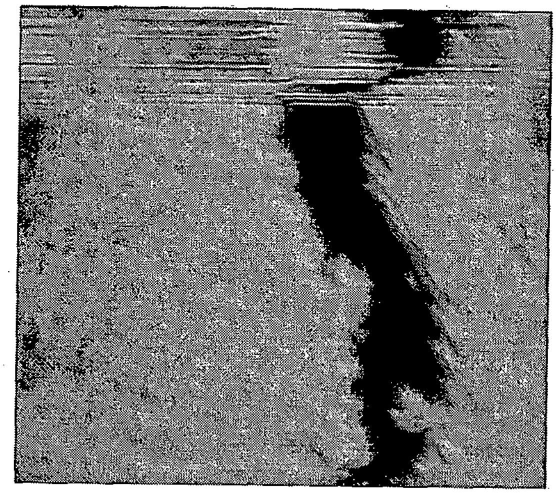

Figure 26b

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5607979 A
- GB 2314020 A


### Littérature non-brevet citée dans la description


- **ZHANG J. ; SMITH E.W. ; SURBER C.** Galenical principles in skin protection. *Curr. Probl. Dermatol.,* 2007, vol. 34, 11-18
- **KOPER O. ; LUCAS E. ; KLABUNDE K.J.** Development of reactive topical skin protectants against sulfur mustard and nerve agents. *J. Appl. Toxicol.,* 1999, vol. 19, 59-70
- **SAXENA A. ; SRIVASTAVA A.K. ; SINGH B. ; GOYAL A.** Removal of sulphur mustard, sarin and simulants on impregnated silica nanoparticles. *J. Hazard. Mater.,* 2012, 211-212, 226-232